# FASCICULE DE BREVET EUROPEEN

(11) **EP 3 131 930 B1**
(45) Date de publication et mention de la délivrance du brevet: **06.02.2019**
(21) Numéro de dépôt: 15715289.3
(22) Date de dépôt: 14.04.2015
(51) Int. Cl.: C07K 16/40

(54) **ANTICORPS RECONNAISSANT LE DOMAINE CENTRAL DE L'ADN POLYMERASE POL THETA ET LEURS UTILISATIONS POUR LE DIAGNOSTIC DU CANCER**
ANTIKÖRPER GEGEN DIE ZENTRALE DOMÄNE VON DNA-POLYMERASE POL-THETA SOWIE VERWENDUNG FÜR DIE DIAGNOSE VON KREBS
ANTIBODIES RECOGNIZING THE CENTRAL DOMAIN OF DNA POLYMERASE POL THETA, AND USE THEREOF FOR DIAGNOSING CANCER

(30) Priorité: 14.04.2014 FR 1453333
(43) Date de publication de la demande: 22.02.2017
(73) Titulaire: Centre National de la Recherche Scientifique (C.N.R.S.), 75016 Paris (FR); Institut National de la Santé et de la Recherche Médicale (INSERM), 75013 Paris (FR); Université Paul Sabatier Toulouse III, 31400 Toulouse (FR)
(72) Inventeur: CAZAUX, Christophe, F-31830 Plaisance du Touch (FR); VIDAL-FERNANDEZ, Anne, F-31170 Tournefeuille (FR); HOFFMANN, Jean-Sébastien, F-31500 Toulouse (FR)
(74) Mandataire: Inserm Transfert
(86) Numéro de dépôt international: PCT/EP2015/058098
(87) Numéro de publication internationale: WO 2015/158729

(56) Documents cités:
- EP-A1- 2 322 658
- WO-A1-2013/102680
- M SEKI ET AL: "POLQ (Pol theta), a DNA polymerase and DNA-dependent ATPase in human cells.", NUCLEIC ACIDS RES, 8 novembre 2006 (2006-11-08), pages 6117-6126, XP055158381,
- F. LEMEE ET AL: "DNA polymerase up-regulation is associated with poor survival in breast cancer, perturbs DNA replication, and promotes genetic instability", PROCEEDINGS OF THE NATIONAL ACADEMY OF SCIENCES, vol. 107, no. 30, 27 juillet 2010 (2010-07-27), pages 13390-13395, XP055008221, ISSN: 0027-8424, DOI: 10.1073/pnas.0910759107
- C ALLERA-MOREAU ET AL: "DNA replication stress response involving PLK1, CDC6, POLQ, RAD51 and CLASPIN upregulation prognoses the outcome of early/mid-stage non-small cell lung cancer patients", ONCOGENESIS, vol. 1, no. 10, 1 octobre 2012 (2012-10-01), page e30, XP055157804, DOI: 10.1038/oncsis.2012.29
- HIGGINS G S ET AL: "332 POLQ (DNA polyermase theta) as a novel therapeutic target: preclinical and clinical data", EUROPEAN JOURNAL OF CANCER. SUPPLEMENT, PERGAMON, OXFORD, GB, vol. 8, no. 7, 1 novembre 2010 (2010-11-01), page 106, XP027498022, ISSN: 1359-6349, DOI: 10.1016/S1359-6349(10)72039-5 [extrait le 2010-11-01]
- HOGG M ET AL: "Lesion Bypass Activity of DNA Polymerase theta (POLQ) Is an Intrinsic Property of the Pol Domain and Depends on Unique Sequence Inserts", JOURNAL OF MOLECULAR BIOLOGY, ACADEMIC PRESS, UNITED KINGDOM, vol. 405, no. 3, 21 janvier 2011 (2011-01-21), pages 642-652, XP027583570, ISSN: 0022-2836, DOI: 10.1016/J.JMB.2010.10.041 [extrait le 2010-11-02]

## Description

La présente description concerne le domaine du cancer y compris le diagnostic et le pronostic du cancer ainsi que la sélection de traitement chimio thérapeutique. Plus particulièrement, la présente description concerne de nouveaux anticorps capables de se lier au domaine central de l'ADN Polymérase Thêta ou Pol θ (codée par le gène *POLQ*), les séquences peptidiques correspondantes ainsi que les séquences nucléotidiques codant pour ces anticorps. La description concerne l'utilisation de ces anticorps, ainsi que les méthodes correspondantes, pour la détection, le diagnostic et le pronostic de désordres pathologiques hyper prolifératifs associés à l'expression, particulièrement l'expression aberrante, de l'ADN polymérase Thêta. La description traite aussi de produits, compositions et/ou kits comprenant au moins un anticorps selon la description pour le diagnostic, le pronostic ou la surveillance et le suivi de la thérapie de cancers, en particulier les cancers solides, plus particulièrement encore les cancers du sein, du poumon ou du côlon.

Le cancer est une pathologie dans laquelle un groupe de cellules se développent de manière incontrôlée, entraînant une invasion et une destruction des tissus adjacents en créant aussi parfois des métastases via une dispersion dans l'organisme par la lymphe ou le sang. Ces trois paramètres définissant un cancer se différencient de ceux caractérisant une tumeur bénigne, qui n'est pas invasive et ne génère pas de métastases.

Il existe de nombreuses méthodes pour le traitement du cancer, que ce soit par chirurgie, radiothérapie, chimiothérapie ou une combinaison de celles-ci.

La sélection d'un traitement approprié est essentielle pour le patient. Il est ainsi primordial de savoir à quel moment initier un protocole de traitement lourd et agressif afin de prévenir l'évolution d'un cancer agressif. D'un autre côté, lorsque la tumeur ne le nécessite pas, un traitement lourd et agressif représente forcément un désavantage thérapeutique pour le patient. En effet, de tels traitements induisent diverses toxicités et effets secondaires qui ont un impact significatif sur la qualité de vie du patient et sont en outre la plupart du temps onéreux. Ces traitements ne devraient donc être initiés que lorsqu'ils sont indispensables et/ou utiles.

A ce jour, la sélection du traitement des tumeurs solides est basée sur le stade tumoral utilisant le test TNM de l'American Joint Committee on Cancer. Le stade TMN attribut un nombre (I à IV) basé sur la combinaison de trois caractéristiques : T identifie la taille de la tumeur, N le nombre de nodules lymphatiques et M l'existence et la localisation de métastases. Selon les localisations tumorales, la combinaison des trois repères TNM permet d'établir un stade (de I à IV). Plus le stade est élevé plus le cancer est avancé et présage d'une issue défavorable.

Bien que reconnu internationalement, cette classification ne permet pas l'identification des cancers dans leurs tous premiers stades de la progression tumorale. En outre, le stade TNM ne donne qu'une information partielle quant à l'agressivité de la tumeur et n'est que d'une utilité limitée pour le pronostic.

Différentes protéines et marqueurs génétiques ont été décrits afin d'affiner le pronostic. En particulier l'analyse de l'expression génique a permis l'identification de signatures génétiques. Cependant les informations rassemblées dans diverses études sont souvent confuses (voir e.g. Lenz, Gastrointest Cancer Res, 1(4 Suppl 2): S29-32, 2007; Walther et al., Nat Rev Cancer, 9(7): 489-99, 2009). La robustesse de signatures multigéniques est questionnable car elles concernent les gènes du cycle cellulaire et n'ajoute que peu d'information par rapport au stade TNM.

Il existe ainsi un besoin pour de meilleurs tests de pronostic de cancer, non seulement pour améliorer la survie des patients mais aussi leur qualité de vie et afin de réserver les protocoles lourds, agressifs et couteux uniquement lorsque cela est nécessaire et bénéfique pour le patient. Il existe un besoin pour un test monogénique qui peut être utilisé de manière répétable, aisée et fidèle pour le pronostic de divers types de cancers.

Lors de la prolifération cellulaire normale, la réplication de l'ADN est assurée par 3 ADN polymérases (Polα, Polδ et Polε). Cependant 13 autres ADN polymérases ont été identifiées dans les cellules humaines. Il s'agit de polymérases spécialisées et dont les fonctions sont peu connues. Il semble aussi que ces polymérases sont hautement mutagènes et que leur activité est étroitement contrôlée.

L'ADN polymérase Pol thêta (codée par le gène POLQ) est une de ces ADN polymérases et contient un domaine hélicase du côté N terminal et un domaine polymérase du côté C terminal. Bien que sa fonction soit peu connue, elle semble être impliquée dans le maintien de la stabilité du génome et dans la réparation de l'ADN (Seki et al., EMBO J, 23: 4484-4494, 2004; Masuda et al., Proc. Natl. Acad. Sci. U.S.A., 102: 13986-13991, 2005, Yoshimura et al., Mol. Cell, 24 : 115-125, 2006), mais aussi dans l'initiation de la réplication de l'ADN. L'expression de l'ARNm POL Q a été analysée dans diverses tumeurs (Kawamura et al., Int J Cancer, 109: 9-16, 2004; Pillaire et al., Oncogene, 29(6):876-887, 2010; Lemée , Bergoglio et al., Proc Natl Acad Sci U S A., 107(30): 13390-5, 2010; Higgins et al., Oncotarget, 1(3): 175-184, 2010). Kawamura et ses collaborateurs (Kawamura et al., Int J Cancer, 109: 9-16, 2004) ont comparé le niveau d'expression de l'ARNm POLQ dans divers tissus tumoraux et ont trouvé que celui-ci est à peine détectable dans les tissus non tumoraux mais surexprimée dans les tissus tumoraux pulmonaires, stomacaux et du colon. Les auteurs ont aussi indiqué que la surexpression de l'ARNm POL Q est corrélée à un pronostic vital défavorable.

Il a été démontré que la dérégulation dans les tumeurs du sein et du poumon de gènes impliqués dans le maintien de la stabilité du génome, dont l'ADN polymérase *POL Q,* était associée au pronostic vital des patients concernés, indépendamment des marqueurs cliniques conventionnels (Lemée, F., Bergoglio, V. et al. (2010) DNA polymerase θ up-regulation is associated with poor survival in breast cancer, perturbs DNA replication, and promotes genetic instability Proc Natl Acad Sci U S A. 107(30): 13390-13395*,* Allera-Moreau, C., et al. (2012) DNA replication stress response involving PLK1, CDC6, POLQ, RAD51 and CLASPIN upregulation prognoses the outcome of early/mid-stage non-small cell lung cancer patients Oncogenesis. 1(10): e30).

Il a aussi été démontré récemment que l'expression de ce biomarqueur, *POL Q,* perturbe le déroulement normal de la duplication de l'ADN, instaurant un « stress réplicatif » (Lemée, F., Bergoglio, V., et al, 2010). Par ailleurs, le stress réplicatif est un évènement précurseur et «moteur» de la cancérogénèse dès ses stades les plus précoces *(*Pillaire, M. J., et al. (2007) Up-regulation of error-prone DNA polymerases beta and kappa slows down fork progression without activating the replication checkpoint Cell Cycle 6: 471-7 - Rey, L., et al. (2009) Human DNA polymerase eta is required for common fragile site stability during unperturbed DNA replication Mol Cell Biol 29: 3344-3354 - Bartkova, J., et al. (2005) DNA damage response as a candidate anti-cancer barrier in early human tumorigenesis Nature 434: 864-70 - Gorgoulis, V. G., et al. (2005) Activation of the DNA damage checkpoint and genomic instability in human precancerous lesions. Nature 434: 907-913 - Bartkova, J., et al. (2006) Oncogene-induced senescence is part of the tumorigenesis barrier imposed by DNA damage checkpoints. Nature 444: 633-637). En ce sens, ce biomarqueur anticiperait la prolifération cellulaire (avant de se diviser, la cellule doit dupliquer son génome) et présente donc un avantage original par rapport aux marqueurs conventionnels cliniques ou en développement, qui mesurent tous la prolifération ou la dédifférenciation cellulaire et donc ne mettent en évidence que la conséquence et non la cause.

Il a par ailleurs été décrit la préparation d'anticorps polyclonaux de lapin reconnaissant le domaine central de l'ADN polymérase Pol Thêta ainsi que leur utilisation pour la détection de la protéine par immunoblotting (Seki, Mineaki, Federica Marini, and Richard D. Wood. "POLQ (Pol θ), a DNA polymerase and DNA-dependent ATPase in human cells." Nucleic acids research 31.21 (2003): 6117-6126).

Les cliniciens sont en forte attente d'outils de détection de ces biomarqueurs, capables de prédire à la fois l'agressivité de la tumeur solide néo-diagnostiquée et la réponse aux traitements thérapeutiques conventionnels.

A l'heure actuelle il n'existe pas d'outils de détection de biomarqueurs, utilisables en routine dans un environnement clinique. Dans le domaine du cancer du sein il existe deux tests génétiques ; Oncotype® et MammaPrint®. Dans les deux cas il s'agit de marqueurs de la prolifération cellulaire et non de la stabilité du génome. Ils n'apportent donc que peu de valeur ajoutée par rapport à la classification clinico-pathologique usuelle (dite TNM). En outre, il s'agit de signatures multi-géniques très difficiles à «populariser» (nécessité d'appareillage de PCR en temps réel multiplex, quantification difficile, pas d'analyse anatomo-pathologique extemporanée possible) et qui concernent donc seulement les structures hospitalières de pointe. En effet, ces techniques de diagnostic et/ou pronostic ne sont pas utilisables ou transférables dans les structures thérapeutiques de tous les hôpitaux ou dans les laboratoires d'anatomopathologie, en particulier ceux «de ville». En ce qui concerne les tests de diagnostic basés sur l'utilisation d'anticorps, ceux-ci sont basés sur des techniques de western-blot impliquant l'extraction des protéines de l'échantillon, leur dénaturation, leur séparation par électrophorèse et transfert sur membrane avant révélation de la protéine d'intérêt à l'aide d'anticorps commerciaux. Ces anticorps commerciaux ne sont pas fonctionnels en immunohistochimie sur des coupes de tissus à tester extemporanément car ils sont incapables de reconnaître la protéine dans sa conformation native et dans son environnement tissulaire/cellulaire.

La grande majorité des marqueurs actuels, moléculaires ou cliniques, n'indiquent en réalité que l'état de «prolifération» et de «différenciation» de la tumeur, c'est-à-dire sa capacité à se diviser rapidement. Ils ont, en effet, été généralement découverts suite à des génotypages (puces Affymetrix) qui ont logiquement sélectionnés les évènements génétiques «passengers» - c'est-à-dire les conséquences - et non «drivers» - c'est-à-dire les causes - de la tumeur déjà développée.

Aussi, malgré les progrès liés au dépistage et aux techniques chirurgicales, la mortalité associée aux cancers du sein et du poumon reste très élevée ; le cancer du poumon étant le plus meurtrier des cancers chez les hommes et le cancer du sein le plus meurtrier chez les femmes.

Les médecins oncologues, même lorsque la tumeur a été détectée de façon précoce, ne possèdent pas actuellement les marqueurs cliniques, et les outils nécessaires à l'établissement d'un pronostic vital fiable et à la prédiction de la réponse chimio-thérapeutique. En l'absence de tels marqueurs, il leur est souvent difficile d'adapter les traitements individualisés adéquats et de moduler la «charge» thérapeutique.

Les praticiens appellent ainsi de leurs voeux l'arrivée sur le marché d'outils d'identification et de suivi de marqueurs d'un nouveau type facilement utilisable en routine, qui leur permettraient ainsi soit d'éviter à leurs patients des traitements lourds et mutagènes et/ou d'adapter la chimiothérapie en fonction de la réponse individuelle attendue.

C'est ainsi là un but de la présente description que de fournir un outil de détection de biomarqueur d'un nouveau type, facile et rapide à mettre en oeuvre.

La présente description a ainsi pour objectif de fournir au moins un produit qui peut être utilisé en tant qu'outil de diagnostic et/ou pronostic ou de surveillance des désordres pathologiques hyperprolifératifs, par exemple le cancer, particulièrement ceux caractérisés par une expression de l'ADN polymérase Pol Thêta ou provoqués par une expression aberrante de l'ADN polymérase Pol Thêta.

Plus particulièrement la description a pour but de fournir des outils utilisables en routine et particulièrement en immunohistochimie sur des prélèvements de tissus de patient sain, de patient susceptible d'être atteint d'un cancer ou de patient atteint de cancer.

Ainsi la présente description fournit au moins un anticorps, particulièrement au moins un anticorps monoclonal, capable de se lier au domaine central de l'ADN polymérase Pol Thêta.

Aussi la présente invention concerne un anticorps monoclonal caractérisé en ce qu'il est sélectionné dans le groupe comprenant les anticorps suivants :
i. anticorps capable de se lier à la séquence amino acide ayant la SEQ 1 du domaine central de l'ADN polymérase Pol Thêta,
ii. anticorps capable de se lier à la séquence amino acide ayant la SEQ 2 du domaine central de l'ADN polymérase Pol Thêta,
iii. anticorps capable de se lier à la séquence amino acide ayant la SEQ 4 du domaine central de l'ADN polymérase Pol Thêta.

Plus particulièrement, l'invention est définie par les revendications.

Les anticorps selon la description présentent la capacité à pouvoir être utilisés à des fins de diagnostic et/ou de pronostic. Dans une forme préférée, le au moins un anticorps selon la présente description, particulièrement monoclonal, est capable de se lier au domaine central de l'ADN polymérase Pol Thêta et permet la détection de cette l'ADN polymérase en immunohistochimie ou immunofluorescence, préférentiellement immunohistochimie.

De manière surprenante et *a contrario* des anticorps du commerce, les anticorps selon la présente description ont la propriété remarquable de pouvoir se lier au domaine central de l'ADN polymérase Pol Thêta sous sa forme entière en western blot et non seulement une forme tronquée ou une fraction de celle-ci. La capacité de liaison des anticorps selon la présente description se manifeste en particulier sur des cellules ou des coupes histologiques de tissus obtenus à partir d'un patient. Cette capacité de liaison peut ainsi être révélée et évaluée par des techniques d'immunohistochimie ou d'immunofluorescence. Comme indiqué plus avant les anticorps commerciaux ou ceux de l'art antérieur (en particulier les anticorps polyconaux décrits par Seki et al (Nucleic Acid Research, 2003, 6117-6126) ne sont pas fonctionnels en immunohistochimie sur des coupes de tissus à tester extemporanément car ils sont incapables de reconnaître et donc de localiser la protéine dans sa conformation native et/ou dans son environnement tissulaire/cellulaire.

Dans une forme de réalisation, les anticorps selon la description sont des anticorps monoclonaux.

Dans un de ses aspects, la description concerne des anticorps isolés, un dérivé ou fragment fonctionnel de ceux-ci ayant la capacité de liaison antigénique, et qui se lient au domaine central de l'ADN polymérase Pol Thêta. Ainsi la description concerne au moins un anticorps selon la description pour son utilisation pour le diagnostic *in vitro* ou *ex vivo* d'un désordre associé à l'expression de l'ADN polymérase Pol Thêta.

Dans un mode de réalisation de la présente description, le désordre associé à l'expression de l'ADN polymérase Pol Thêta est le cancer.

Dans un mode de réalisation de la présente description l'anticorps monoclonal selon la description est ainsi caractérisé par sa capacité de se lier au domaine central de l'ADN polymèrase Thêta au sein de cellules ou coupes histologique de tissus.

Dans un mode de réalisation particulier de la présente description, les anticorps monoclonaux selon la description, sont caractérisés par leur capacité à se lier au domaine central de l'ADN polymérase Pol Thêta et permettent la détection de l'ADN polymérase Pol Thêta en immunohistochimie ou immunofluorescence, préférentiellement en immunohistochimie.

Dans un autre mode de réalisation particulier de la présente description, les anticorps monoclonaux selon la description, capables de se lier au domaine central de l'ADN polymérase Pol Thêta, permettent la détection de l'ADN polymérase Pol Thêta en western blot.

Dans un mode de réalisation particulier de la présente description, les anticorps monoclonaux selon la description, capables de se lier au domaine central de l'ADN polymérase Pol Thêta, permettent la détection de l'ADN polymérase Pol Thêta en immunoprécipitation.

La présente description concerne aussi des anticorps, leurs dérivés ou fragments fonctionnels, obtenus par recombinaison génétique ou synthèse chimique.

Dans un mode de réalisation particulier, les anticorps selon la présente description sont des anticorps monoclonaux. Un anticorps monoclonal correspond à une population d'anticorps identiques dirigés contre le même épitope d'un antigène. Ces anticorps sont produits en culture par un clone de lymphocytes B selon la technique des hybridomes ou *via* une cellule hôte transfectée par des acides nucléiques codant pour l'anticorps en question. Après avoir immunisé une souris, par exemple, contre un antigène donné, on prélève dans sa rate des lymphocytes B. Ceux-ci sont fusionnés avec des plasmocytes tumoraux immortalisés. Après sélection, les hybridomes ainsi obtenus sont une source permanente et stable d'un seul type d'anticorps monoclonal. La spécificité des anticorps monoclonaux est supérieure à celle des sérums polyclonaux. Un anticorps monoclonal est généralement caractérisé par des chaînes lourdes d'une et seulement une classe et sous-classe et des chaînes légères d'un seul type.

Les antigènes utilisés pour obtenir les anticorps selon la description comprennent des séquences d'amino-acides ou peptidiques du domaine central de la protéine ADN polymérase Pol Thêta. Ils sont avantageusement choisis au sein de sous-domaines tridimensionnels de la protéine présentant des critères d'exposition externe et donc d'accessibilité, mais aussi selon un équilibre hydrophobicité/hydrophilicité desdits sous-domaines du domaine central, selon l'encombrement stérique et la polarité des différents groupes mais aussi selon les interactions électrostatiques et hydrogène. Cette évaluation peut être réalisée *via* une modélisation de la structure tridimensionnelle et du repliement de la protéine grâce à des outils prédictifs logiciels connus de l'homme du métier à partir de la séquence peptidique de la protéine.

Ainsi les séquences d'amino-acides ou peptidiques correspondant à celles indiquées dans le Tableau 1 ont été déterminées et les peptides correspondants utilisés pour réaliser l'immunisation de souris aux fins de production des anticorps selon la description. Ces séquences peptidiques correspondent aux épitopes antigéniques reconnus par les anticorps selon la présente description et sont situés au début ou à la fin du domaine central de la protéine ADN Polymérase Pol Thêta.

**Tableau 1 : Peptides utilisés pour immunisation et séquences amino-acides correspondants reconnus par les anticorps selon la description**

| Peptides | Séquences amino-acides | SEQ ID N° |
|---|---|---|
| Peptide 1 | CKHSPNIVQDLNKSREHTSS | 1 |
| Peptide 2 | CSIFRARKRASLDINKEKPG | 2 |
| Peptide 3 | CLQEDLIKKSNVNENQDTH | 3 |
| Peptide 4 | HDETSSLLPRKESNIVDDNGC | 4 |

Les anticorps obtenus sont les anticorps 1B1, 18E1, 10B2 et 15G9 qui reconnaissent, respectivement, les séquences d'amino-acides ou peptidiques 2, 4, 1, et 4. Dans une certaine mesure, l'anticorps 10B2 reconnait aussi la séquence d'amino-acides 2 mais plus faiblement.

Une immunoglobuline de type G (IgG) appartient à une classe de molécules anticorps. Un anticorps IgG est typiquement constitué de deux chaînes lourdes identiques et de deux chaînes légères identiques. Chaque chaîne lourde et légère comprend un domaine constant et un domaine variable. Les 2 chaînes lourdes (H pour *heavy*) et les 2 chaînes légères (L pour *light*) qui sont reliées entre elles par un nombre variable de ponts disulfures assurant une flexibilité de la molécule. Ces chaînes forment une structure en Y (chaque chaîne légère constitue pour moitié un bras du Y). Chaque chaîne légère est constituée d'un domaine constant et d'un domaine variable; les chaînes lourdes sont composées d'un fragment variable et de 3 ou 4 fragments constants. Pour un anticorps donné, les deux chaînes lourdes sont identiques, de même pour les deux chaînes légères.

Chaque domaine variable contient 3 segments dénommés Région Déterminant la Complémentarité ou CDD ou encore région hyper-variable. On désignera dans la présente description CDR ou CDRs lorsqu'on se réfère à un seul CDR ou à plus d'un CDR, respectivement.

Les CDRs sont responsables de la liaison spécifique à l'épitope de l'antigène. Il existe 3 CDRs au niveau de chaque chaîne lourde et de chaque chaîne légère. Ils sont classiquement identifiés CDR1, CDR2 et CDR3, numérotés séquentiellement à partir de l'extrémité N terminale.

Les portions plus conservées des domaines variables sont appelées « framework régions ».

Selon la présente description, les CDRs des anticorps sont définis selon la nomenclature IMGT (Système d'information international en immunogénétique). Le système de numérotation IMGT a été défini pour comparer les domaines variables indépendamment de l'espèce. Selon le système IMGT, les acides aminés conservés ont toujours la même position, par exemple la cystéine 23, le tryptophane 41, un acide aminé hydrophobe 89, la cystéine 104, la phénylalanine ou le tryptophane 118. Le système de numérotation IMGT fournit une délimitation standardisée des régions framework et des CDRs. Ainsi le CDR1 correspond à la fraction des résidus 27 à 38, le CDR2 des résidus 56 à 65 et le CDR3 des résidus 105 à 117 (Lefranc et al ; Nucl. Acids Res. (1999) 27 (1): 209-212).

Dans un mode de réalisation, l'anticorps monoclonal selon la description capable de lier au domaine central de l'ADN polymérase Pol Thêta est sélectionné dans le groupe comprenant les anticorps suivants :
a. anticorps capable de se lier à la séquence amino-acide ayant la SEQ 1 du domaine central de l'ADN polymérase Pol Thêta,
b. anticorps capable de se lier à la séquence amino-acide ayant la SEQ 2 du domaine central de l'ADN polymérase Pol Thêta ,
c. anticorps capable de se lier à la séquence amino-acide ayant la SEQ 4 du domaine central de l'ADN polymérase Pol Thêta .
Dans un mode de réalisation, l'anticorps monoclonal selon la description capable de lier au domaine central de l'ADN polymérase Pol Thêta est sélectionné dans le groupe comprenant les anticorps suivants :
a. anticorps capable de se lier à la séquence amino-acide ayant la SEQ 1 du domaine central de l'ADN polymérase Pol Thêta caractérisé en ce que ledit anticorps comprend les 6 CDRs comprenant les séquences amino-acides respectives suivantes : SEQ ID 21, SEQ ID 22, SEQ ID 23, SEQ ID 24, SEQ ID 25, SEQ ID 26.
b. anticorps capable de se lier à la séquence amino-acide ayant la SEQ 2 du domaine central de l'ADN polymérase Pol Thêta caractérisé en ce que ledit anticorps comprend les 6 CDRs comprenant les séquences amino-acides respectives suivantes : SEQ ID 5, SEQ ID 6, SEQ ID 7, SEQ ID 8, SEQ ID 9, SEQ ID 10.
c. anticorps capable de se lier à la séquence amino-acide ayant la SEQ 4 du domaine central de l'ADN polymérase Pol Thêta caractérisé en ce que ledit anticorps comprend les 6 CDRs comprenant les séquences amino-acides respectives suivantes : SEQ ID 13, SEQ ID 14, SEQ ID 15, SEQ ID 16, SEQ ID 17, SEQ ID 18.
d. anticorps capable de se lier à la séquence amino-acide ayant la SEQ 4 du domaine central de l'ADN polymérase Pol Thêta caractérisé en ce que ledit anticorps comprend les 6 CDRs comprenant les séquences amino-acides respectives suivantes : SEQ ID 29, SEQ ID 30, SEQ ID 31, SEQ ID 32, SEQ ID33, SEQ ID 34.
De manière préférentielle, l'anticorps capable de se lier à la séquence d'amino-acides ayant la SEQ 1 du domaine central de l'ADN polymérase Pol Thêta comprend :
i. Une chaîne lourde comprenant les 3 CDRs : CDR3H1, CDR3H2 et CDR3H3 ayant les séquences respectives suivantes SEQ ID 21, SEQ ID 22 et SEQ ID 23,
ii. Une chaîne légère comprenant les 3 CDRs : CDR3L1, CDR3L2 et CDR3L3 ayant les séquences respectives suivantes SEQ ID 24, SEQ ID 25 et SEQ ID 26.

De manière aussi préférée, l'anticorps capable de se lier à la séquence d'amino-acides ayant la SEQ 2 du domaine central l'ADN polymérase Pol Thêta comprend :
i. Une chaîne lourde comprenant les 3 CDRs : CDR1H1, CDR1H2 et CDR1H3 ayant les séquences respectives suivantes SEQ ID 5, SEQ ID 6 et SEQ ID 7,
ii. Une chaîne légère comprenant les 3 CDRs : CDR1L1, CDR1L2 et CDR1L3 ayant les séquences respectives suivantes SEQ ID 8., SEQ ID 9 et SEQ ID 10.
De manière encore plus préférée, l'anticorps capable de se lier à la séquence d'amino-acides ayant la SEQ 4 du domaine central l'ADN polymérase POL Q comprend :
i. une chaîne lourde comprenant les 3 CDRs : CDR2H1, CDR2H2 et CDR2H3 ayant les séquences respectives suivantes SEQ ID 13, SEQ ID 14 et SEQ ID 15,
ii. une chaîne légère comprenant les 3 CDRs : CDR2L1, CDR2L2 et CDR2L3 ayant les séquences respectives suivantes SEQ ID 16, SEQ ID 17 et SEQ ID 18.

De manière préférée, l'anticorps capable de se lier à la séquence d'amino-acides ayant la SEQ 4 du domaine central l'ADN polymérase Pol Thêta comprend :
i. une chaîne lourde comprenant les 3 CDRs : CDR4H1, CDR4H2 et CDR4H3 ayant les séquences respectives suivantes SEQ ID 29, SEQ ID 30 et SEQ ID 31,
ii. une chaîne légère comprenant les 3 CDRs : CDR4L1, CDR4L2 et CDR4L3 ayant les séquences respectives suivantes SEQ ID 32, SEQ ID 33 et SEQ ID 34.
Dans une variante encore préférée, l'anticorps selon la présente description est choisi parmi :
a. L'anticorps capable de se lier à la fraction peptidique ayant la SEQ 1 du domaine central de l'ADN polymérase Pol Thêta comprenant :
   i. une chaîne lourde comprenant les 3 CDRs : CDR3H1, CDR3H2 et CDR3H3 ayant les séquences respectives suivantes SEQ ID 21, SEQ ID 22 et SEQ ID 23,
   ii. un domaine variable de chaîne légère comprenant la SEQ ID 28,
b. L'anticorps capable de se lier à la fraction peptidique ayant la SEQ 2 du domaine central de l'ADN polymérase Pol Thêta comprenant :
   i. une chaîne lourde comprenant les 3 CDRs : CDR1H1, CDR1H2 et CDR1H3 ayant les séquences respectives suivantes SEQ ID 5, SEQ ID 6 et SEQ ID 7,
   ii. un domaine variable de chaîne légère comprenant la SEQ ID 12,
c. L'anticorps capable de se lier à la fraction peptidique ayant la SEQ 4 du domaine central de l'ADN polymérase POL Q comprenant :
   i. une chaîne lourde comprenant les 3 CDRs : CDR2H1, CDR2H2 et CDR2H3 ayant les séquences respectives suivantes SEQ ID 13, SEQ ID 14 et SEQ ID 15,
   ii. un domaine variable de chaîne légère comprenant la SEQ ID 20,
d. L'anticorps capable de se lier à la fraction peptidique ayant la SEQ 4 du domaine central de l'ADN polymérase Pol Thêta comprenant :
   i. une chaîne lourde comprenant les 3 CDRs : CDR4H1, CDR4H2 et CDR4H3 ayant les séquences respectives suivantes SEQ ID 29 SEQ ID 30 et SEQ ID 31,
   ii. un domaine variable de chaîne légère comprenant la SEQ ID 36.
Dans un mode de réalisation particulièrement préféré, l'anticorps selon la description est choisi parmi :
a. l'anticorps capable de se lier à la fraction peptidique ayant la SEQ 1 du domaine central de l'ADN polymérase Pol Thêta comprenant :
   i. une chaîne légère comprenant les 3 CDRs : CDR3L1, CDR3L2 et CDR3L3 ayant les séquences respectives suivantes SEQ ID 24, SEQ ID 25 et SEQ ID 26,
   ii. un domaine variable de chaîne lourde comprenant la SEQ ID 27,
b. l'anticorps capable de se lier à la fraction peptidique ayant la SEQ 2 du domaine central l'ADN polymérase Pol Thêta comprenant :
   i. une chaîne légère comprenant les 3 CDRs : CDR1L1, CDR1L2 et CDR1L3 ayant les séquences respectives suivantes SEQ ID 8, SEQ ID 9 et SEQ ID 10,
   ii. un domaine variable de chaîne lourde comprenant la SEQ ID 11,
c. l'anticorps capable de se lier à la fraction peptidique ayant la SEQ 4 du domaine central de l'ADN polymérase POL Théta comprenant :
   i. une chaîne légère comprenant les 3 CDRs : CDR2L1, CDR2L2 et CDR2L3 ayant les séquences respectives suivantes SEQ ID 16, SEQ ID 17 et SEQ ID 18,
   ii. un domaine variable de chaîne lourde comprenant la SEQ ID 19,
d. l'anticorps capable de se lier à la fraction peptidique ayant la SEQ 4 du domaine central de l'ADN polymérase Pol Thêta comprenant :
   i. une chaîne légère comprenant les 3 CDRs : CDR4L1, CDR4L2 et CDR4L3 ayant les séquences respectives suivantes SEQ ID 32, SEQ ID 33. et SEQ ID 34,
   ii. un domaine variable de chaîne lourde comprenant la SEQ ID 35.
Dans un mode de réalisation encore plus préféré, l'anticorps selon la description est choisi parmi :
a. l'anticorps capable de se lier à la fraction peptidique ayant la SEQ 1 du domaine central de l'ADN polymérase Pol Thêta comprenant :
   i. un domaine variable de la chaîne lourde comprenant la SEQ ID 27,
   ii. un domaine variable de chaîne légère comprenant la SEQ ID 28,
b. l'anticorps capable de se lier à la fraction peptidique ayant la SEQ 2 du domaine central l'ADN polymérase Pol Thêta comprenant :
   i. un domaine variable de la chaîne lourde comprenant la SEQ ID 11,
   ii. un domaine variable de la chaîne légère comprenant la SEQ ID 12,
c. l'anticorps capable de se lier à la fraction peptidique ayant la SEQ 4 du domaine central l'ADN polymérase Pol Thêta comprenant :
   iii. un domaine variable de la chaîne lourde comprenant la SEQ ID 19,
   iv. un domaine variable de la chaîne légère comprenant la SEQ ID20,
d. l'anticorps capable de se lier à la fraction peptidique ayant la SEQ 4 du domaine central l'ADN polymérase Pol Thêta comprenant :
   i. un domaine variable de la chaîne lourde comprenant la SEQ ID 35,
   ii. un domaine variable de la chaîne légère comprenant la SEQ ID 36.

Dans une forme préférée de la description, l'anticorps est l'anticorps 1B1 comprenant :
i. une chaîne lourde comprenant la séquence SEQ ID 39,
ii. une chaîne légère comprenant la séquence SEQ ID 40.

Dans une forme préférée de la description, l'anticorps est l'anticorps 18E1 comprenant :
i. une chaîne lourde comprenant SEQ ID41,
ii. une chaîne légère comprenant SEQ ID 42.

Dans une forme préférée de la description, l'anticorps est l'anticorps 10B2 comprenant :
i. une chaîne lourde comprenant SEQ ID 37,
ii. une chaîne légère comprenant SEQ ID 38.

Dans une forme préférée de la description, l'anticorps est l'anticorps 15G9 comprenant :
i. une chaîne lourde comprenant SEQ ID 43,
ii. une chaîne légère comprenant SEQ ID 44.

Les anticorps selon la présente description présentent la propriété remarquable de pouvoir se lier au domaine central de l'ADN polymérase Pol Thêta , sous forme entière non tronquée, ou bien d'isoformes de la protéine, en particulier dans son environnement tissulaire endogène, plus particulièrement encore au sein de coupes histologique de tissus issus de prélèvement sur un sujet (pièce opératoire ou biopsie) chez lequel on souhaite diagnostiquer un cancer, pronostiquer l'évolution d'un cancer et/ou orienter le traitement par chimiothérapie. Cette propriété de liaison au domaine central de l'ADN Polymérase Pol Thêta peut être observée en western blot, immunohistochimie, en immunofluorescence ainsi qu'en immunoprécipitation, particulièrement en immunohistochimie.

Les anticorps selon la présente description présentent ainsi la propriété particulièrement avantageuse et inattendue de pouvoir se lier au domaine central de l'ADN polymérase Pol Thêta, non seulement en western blot, c'est-à-dire sur la protéine dénaturée et transférée sur une membrane, mais aussi et surtout dans l'environnement cellulaire et/ou cellulaire endogène de la protéine qui se trouve ainsi sous sa conformation entière, proche de la conformation native, non tronquée et permettre ainsi sa détection et sa localisation tissulaire/cellulaire par des techniques d'immunohistochimie, par exemple immunofluorescence, au sein de coupes histologiques de tissus sains ou cancéreux.

Cette propriété permet ainsi la localisation histologique de la protéine ADN polymérase Pol Thêta au sein d'échantillons tissulaires ou cellulaires. Cela n'est pas possible avec les anticorps de l'art antérieur qui ne fonctionnent que selon la technologie de western blot impliquant lyse cellulaire et dénaturation protéique. En outre cette technique de western blot ne permet pas la localisation cellulaire ou tissulaire de la protéine puisque l'échantillon est broyé.

Les exemples de la présente description illustrent ces propriétés des anticorps selon la description. C'est donc une différence majeure entre les anticorps de la présente description et ceux de l'art antérieur que de permettre la détection, la localisation histologique et la quantification, au sein de coupes et/ou d'échantillons cellulaires et tissulaires, de la protéine ADN polymérase Pol Thêta.

Une telle propriété illustre bien la solution au problème que se propose de résoudre la présente description qui comme indiqué plus avant vise la fourniture d'outils pouvant être utilisés en tant qu'outil de diagnostic et/ou pronostic ou de surveillance des désordres pathologiques hyperprolifératifs, par exemple le cancer, particulièrement ceux caractérisés par une expression de l'ADN polymérase Pol Thêta ou provoqués par une expression aberrante de l'ADN polymérase Pol Thêta et plus particulièrement des outils utilisables en routine et préférentiellement en immunohistochimie sur des prélèvements de tissus de patient sain, de patient susceptible d'être atteint d'un cancer ou de patient atteint de cancer.

Dans un mode de réalisation de la présente description l'anticorps monoclonal selon la description est ainsi caractérisé par sa capacité de se lier au domaine central de l'ADN polymérase Thêta au sein de cellules ou coupes histologique de tissus.

Dans un mode de réalisation particulier de la présente description, les anticorps monoclonaux selon la description, sont caractérisés par leur capacité à se lier au domaine central de l'ADN polymérase Pol Thêta et permettent la détection de l'ADN polymérase Pol Thêta en immunohistochimie ou immunofluorescence, préférentiellement en immunohistochimie.

En histologie humaine, le prélèvement de tissus que l'on souhaite évaluer peut se faire sur pièces opératoires ou par biopsie.

La fixation de ces prélèvements a pour but de conserver les structures. En effet, le prélèvement des tissus provoque leur mort : ce qui peut entraîner autodigestion du tissu ou putréfaction des tissus par contamination. L'intérêt de la fixation réside dans l'immobilisation des constituants tissulaires/cellulaires, prévient l'autolyse cellulaire, la putréfaction bactérienne post-mortem et permet les techniques histologiques ainsi que les colorations ultérieures.

Les techniques de fixation ne sont pas critiques et sont bien connues de l'homme du métier. Par exemple, un fixateur bien connu en microscopie optique est le formol.

Durée et quantité de fixateur dépendent de la quantité de tissus à fixer. Ces paramètres ne sont pas critiques dans le cadre de la présente description et sont bien connus de l'homme du métier.

Pour que la lumière puisse passer à travers le tissu à examiner, celui-ci doit être très fin et solide. Cela peut être obtenu *via* les techniques d'inclusion. L'inclusion en paraffine consiste à infiltrer et à enrober les tissus à examiner avec de la paraffine. L'inclusion est précédée de deux étapes. Il faut tout d'abord procéder à la déshydratation par passage des tissus dans des bains d'alcool de degré croissant (70°, 80°, 90°, 95°, 99°, 100°). L'intérêt de la déshydratation est d'éliminer le fixateur. L'alcool (éthanol) est ensuite remplacé par un solvant miscible à la paraffine. Il peut s'agir soit de xylène, soit de toluène par exemple. Ces substances éliminent l'éthanol. Au fur et à mesure de leur infiltration par le solvant, les tissus ont tendance à s'éclaircir. Une fois totalement imprégné, le tissu est placé dans de la paraffine fondue (portée à 56/58°C) ; la chaleur provoque l'évaporation du solvant (et sa dissolution dans la paraffine) : les espaces ainsi libérés sont remplis par la paraffine. Les tissus enrobés de paraffine sont placés dans de petits moules, à température ambiante, ce qui provoque le durcissement de la paraffine et donc la rigidification des fragments tissulaires prélevés. On procède alors au démoulage : on obtient des fragments tissulaires inclus dans un bloc de paraffine.

Pour isoler des coupes à partir du bloc de paraffine, on utilise pour cela un microtome, qui fait avancer le bloc sur un rasoir : le bloc avance d'environ 2 à 3 µm à chaque fois. L'ensemble des tranches vont former un ruban dans lequel on retrouve des coupes sériées de prélèvement tissulaire. Les coupes sont ensuite collées par chauffage sur des lames de verre.

Les tissus de l'organisme ne sont pas spontanément colorés, ce qui rend les observations difficiles. Cependant, pour que l'on puisse utiliser une coloration, la paraffine doit être éliminée. On procède donc au déparaffinage, qui consiste à passer les lames dans des bains de toluène ou de xylène afin de dissoudre la paraffine. On effectue ensuite une réhydratation : l'alcool se mélange avec l'eau et le toluène, on passe les lames dans des bains d'alcool de degré décroissant.

L'utilisation de tissus congelés ou de tissus fixés et inclus en paraffine est envisagée dans le cadre de la présente description.

Dans le cadre de tissus congelés, il conviendra de pratiquer des coupes au cryostat ; dans ce cas, la morphologie sera moins bien conservée que sur des coupes fixées et incluses dans la paraffine. Un intérêt est de pouvoir préserver des épitopes qui pourraient avoir été détruits par des procédés de fixation et d'inclusion. Néanmoins, l'utilisation de l'immunohistochimie sur coupes congelées tend à diminuer, d'une part en raison de la fabrication d'anticorps reconnaissant des épitopes résistants à la fixation, d'autre part en raison de l'encombrement et de la logistique nécessaires à cette méthode de conservation.

De manière avantageuse mais non obligatoire, des tissus fixés inclus en paraffine seront utilisés dans le cadre de la présente description. Les avantages sont l'utilisation a posteriori quand le tissu a été fixé et la possibilité d'études rétrospectives (sur blocs ou sur lames) ainsi qu'une morphologie bien conservée.

Les techniques d'immunomarquage sont assimilées à des techniques de coloration ; on peut utiliser des anticorps couplés à des substances que l'on peut révéler en microscopie (enzymes, substances fluorescentes, ...).

L'immunohistochimie (ou « IHC ») est le nom d'une méthode de localisation de protéines dans les cellules au sein d'une coupe de tissu au moyen d'anticorps capables de révéler lesdites protéines.

L'immunohistochimie exploite la capacité d'un anticorps à se lier spécifiquement à un antigène dans les tissus biologiques. Les anticorps peuvent être d'origine polyclonale ou monoclonale. L'immunohistochimie (ou immunocytochimie) consiste à détecter dans les tissus ou les cellules, le site de la liaison d'un anticorps spécifique avec la protéine contre laquelle il est dirigé.

En immunohistochimie, on utilise la spécificité de l'interaction anticorps-antigène pour identifier de manière sélective des protéines spécifiques se trouvant dans des cellules ou des sections de tissu. Cette technique peut être utilisée sur des cellules fracturées ou sur des sections de tissus congelées ou fixées.

L'intérêt de cette technique est de repérer des antigènes (protéines le plus souvent) d'intérêt au niveau cellulaire ou extracellulaire à l'aide d'anticorps spécifiques.

La principale difficulté de l'IHC est de disposer d'un bon anticorps spécifique et pouvant atteindre son épitope sur la coupe. Or, c'est une propriété des anticorps selon la présente description que de pouvoir atteindre et se lier au domaine central de l'ADN Polymérase Pol Thêta au sein de coupes de tissus et être révélés en immunohistochimie.

La visualisation de l'interaction entre un anticorps selon la description et le domaine central de l'ADN Polymérase Pol Thêta peut se réaliser par divers moyens. Dans les cas les plus courants, l'anticorps secondaire reconnaissant l'anticorps primaire de la description est conjugué à une enzyme, par exemple la peroxydase qui a la capacité de catalyser une réaction colorée.

Alternativement, l'interaction peut être détectée par immunofluorescence. "Immunofluorescence", telle qu'utilisée dans la présente demande se réfère à une réaction antigène-anticorps dans laquelle l'anticorps secondaire est marqué avec une molécule fluorescente (fluorochrome) et le complexe antigène-anticorps formé est visualisé à l'aide d'un microscope à fluorescence.

Dans le cadre de la présente description, l'immunofluorescence comprend l'immunofluorescence directe et l'immunofluorescence indirecte. Dans le cas d'immunofluorescence directe, un anticorps marqué directement par un fluorophore est mis en oeuvre pour détecter l'ADN polymérase Pol Thêta. En immunofluorescence indirecte, un anticorps primaire spécifique de l'ADN polymérase Pol Thêta est reconnu par un anticorps secondaire lié à un fluorochrome.

Les fluorochromes sont des composés qui absorbent à une certaine longueur d'onde et émettent à une autre longueur d'onde visible par fluorescence. Des fluorochromes communs sont la rhodamine, la lissamine par exemple. Lorsque de la fluorescéine (FITC) est excitée par une lumière bleue (longueur d'onde 488nm), elle émet une lumière verte (520nm). La phycoérythrine (PE) émet une lumière orange (570nm). Les fluorochromes couramment utilisés en immunofluorescence sont l'isothiocyanate de fluorescéine (vert) et l'isothiocyanate de tetraméthyl rhodamine (rouge) par exemple.

L'immunofluorescence directe sur coupe en congélation est une méthode très spécifique qui évite toutes les réactions non spécifiques. C'est une méthode qui nécessite d'utiliser des anticorps couplés à un chromogène fluorescent (Fluorescéine, Rhodamine, Rouge Texas...).

C'est ainsi un objet de la présente description que de fournir des anticorps tels que décrits plus avant qui sont marqués eux-mêmes ou bien l'anticorps secondaire qui les reconnait, particulièrement par couplage avec un chromogène fluorescent.

Dans la coloration immunohistochimique directe, un anticorps étiqueté se lie directement à son antigène. Bien qu'elle n'utilise qu'un seul anticorps et qu'elle soit simple et rapide à mettre en oeuvre, la coloration immunohistochimique peut souffrir d'un certain manque de sensibilité. Pour la révélation de l'immunohistochimie indirecte, les enzymes utilisées peuvent être la peroxydase ou la phosphatase alcaline par exemple et les chromogènes sont le plus souvent la diaminobenzidine (réaction de couleur brune) ou l'aminoéthylcarbazole (3-amino, 9-éthyl carbazole, AEC, réaction de couleur rouge).

L'intensité du signal obtenu après marquage d'une réaction antigène-anticorps dépend du nombre de molécules colorées visibles.

Il est fréquent d'utiliser des méthodes indirectes, où un anticorps secondaire marqué reconnait l'anticorps primaire lié à l'antigène. Plusieurs anticorps secondaires peuvent être en mesure de lier différents sites antigéniques sur l'anticorps primaire, amplifiant ainsi le signal à détecter. La réaction anticorps-antigène peut être visualisée en utilisant un agent réactif tertiaire tel qu'une enzyme rapporteur (comme la peroxydase qui catalyse une réaction de formation de couleur), un métal colloïdal, une étiquette radio ou un fluorophore (comme le rouge Texas ou le FITC). Dans le cas d'un système à enzyme rapporteur, l'ajout d'un substrat adéquat (chromogène) tel que l'AEC ou la DAB a pour résultat la production d'un produit coloré qui colore la cellule avec la peroxydase comme enzyme rapporteur.

Plusieurs mécanismes d'amplification sont possibles, parmi lesquelles les méthodes à trois couches, d'autant que l'anticorps intermédiaire divalent peut aussi servir à attacher un complexe réactif : peroxydase-antiperoxydase, avidine-biotine peroxydase (ou phosphatase alcaline) et streptavidine-biotine-peroxydase par exemple. On peut multiplier le nombre de molécules réactives en les attachant à un polymère ou bien l'amplification peut être générée par la création d'un complexe qui multiplie les molécules d'enzyme attachées de façon non covalente à l'anticorps (tyramide). L'amplification peut être apportée par l'augmentation du temps d'incubation et aussi par le prétraitement des coupes déparaffinées par la chaleur ou des enzymes.

A titre d'exemple non limitatif d'amplification, on peut citer la méthode immunoenzymatique de type streptavidine-biotine-peroxydase : l'anticorps primaire se fixe à l'antigène, ensuite l'anticorps secondaire se fixe à l'anticorps primaire et porte une molécule de biotine qui fixe un complexe streptavidine-peroxydase. Une autre méthode immunoenzymatique avec amplification du signal par un polymère existe : l'anticorps primaire se fixe à l'antigène, l'anticorps secondaire se fixe à l'anticorps primaire et est couplé à un polymère inerte qui porte plusieurs molécules d'anticorps secondaires et de peroxydase.

Un autre mode de détection peut être le Duolink® (Olink Bioscience) qui est une technologie qui permet la détection, la visualisation et la quantification des interactions entre les protéines, mais aussi des protéines seules, ou des modifications des protéines dans des échantillons de tissus ou des cellules préparées pour la microscopie. La cible est détectée au moyen d'un (ou deux) anticorps primaire(s) en fonction de l'application. Dans le cas où deux anticorps primaires sont utilisés, ils ont été produits dans des espèces différentes.

Cette technique est basée sur le PLA (Proximity Ligation Assay). in situ, qui est une technologie de ligation de proximité. Elle utilise deux anticorps secondaires couplés chacun à un oligonucléotide et formant les sondes PLA. Le signal n'est généré que lorsque les deux sondes PLA sont fixées à proximité l'une de l'autre, permettant ainsi la ligation et l'amplification à partir des oligonucléotides. Ces sondes PLA reconnaissent soit un seul anticorps primaire soit deux, selon l'expérience, ces anticorps primaires étant eux-mêmes fixés à l'échantillon. Le signal obtenu est une tache fluorescente ou colorée (pour la détection sur coupe de tissus en champ clair) qui apparait en microscopie à l'endroit où la ligation s'est produite. Ces signaux PLA peuvent être quantifiés (comptés) et assignés à une localisation subcellulaire spécifique grâce à l'analyse des images obtenues en microscopie.

Ces exemples ne sont bien sûr pas limitatifs et de nombreuses techniques ou réactifs commerciaux sont disponibles pour révéler des anticorps.

Dans un mode de réalisation, la présente description concerne un ou plusieurs anticorps monoclonaux murins.

La présente description concerne aussi des dérivés ou fragments fonctionnels des anticorps selon la description, ayant les mêmes propriétés de liaison antigénique que les anticorps selon la présente description.

Par l'expression « dérivés ou fragments fonctionnels ayant des propriétés de liaison antigénique », on entend en particulier des structures protéiques ayant une capacité de liaison spécifique ou identique aux anticorps selon la description et comprenant au moins un, au moins 2, au moins 3, au moins 4, au moins 5, particulièrement 6 des CDRs d'un anticorps selon la présente description, de manière à préserver la capacité de liaison au domaine central l'ADN polymérase Pol Thêta ou aux épitopes. Par l'expression fragment, on entend une partie de l'anticorps et par l'expression dérivés on entend toute structure comprenant l'anticorps sur lequel est greffé un autre anticorps ou une autre chaîne peptidique par exemple de type échafaudage.

En particulier, les composés dérivés selon la présente description peuvent consister en des échafaudages protéiques de type immunoglobuline par exemple. L'homme du métier connait diverses techniques de construction d'échafaudage peptidique ainsi que les moyens de greffage de CDRs des anticorps selon la présente description afin de préserver les propriétés de reconnaissance et de liaison aux épitopes du domaine central de l'ADN polymérase Pol Thêta tels que décrits dans la présente description.

Un autre aspect de la présente description concerne des fragments fonctionnels des anticorps selon la présente description.

Les fragments fonctionnels des anticorps selon la présente description peuvent ainsi être des fragments Fv, Fab, (Fab')2, Fab', scFv, scFv-Fc ou des ditanticorps ou tout fragment dont la demi-vie a été accrue tels que des fragments modifiés par addition de PEG.

Par soucis de clarté, le Tableau 2 résume les séquences correspondant aux anticorps selon la description.

**Tableau 2 : Séquences SEQ ID N° des séquences protéiques des composantes des anticorps selon la description**

| Anticorps | Chaine lourde | Chaine légère | Séquence protéique | SEQ ID |
|---|---|---|---|---|
| 1B1 | CDR1H1 | | G Y T F T N Y W | 5 |
| | CDR1H2 | | I D P S D R Y T | 6 |
| | CDR1H3 | | T M Y S F A Y | 7 |
| | | CDR1L1 | Q S L L D S D G K T Y | 8 |
| | | CDR1L2 | L V S | 9 |
| | | CDR1L3 | V Q G A H L P Q T | 10 |
| | Domaine variable chaine lourde | | | 11 |
| | | Domaine variable chaine légère | | 12 |
| 18E1 | CDR2H1 | | G D T F T D Y P | 13 |
| | CDR2H2 | | I N A E T A E P | 14 |
| | CDR2H3 | | A S S Y G Y | 15 |
| | | CDR2L1 | Q S L L Y S D G K T Y | 16 |
| | | CDR2L2 | L V S | 17 |
| | | CDR2L3 | V Q G S H F P H T | 18 |
| | Domaine variable chaine lourde | | | 19 |
| | | Domaine variable chaine légère | | 20 |
| 10B2 | CDR3H1 | | G Y T F T D Y Y | 21 |
| | CDR3H2 | | I N P N N G G S | 22 |
| | CDR3H3 | | A R G D Y S G T S F V M F A H | 23 |
| | | CDR3L1 | K S L L H S N G N T Y | 24 |
| | | CDR3L2 | Y M S | 25 |
| | | CDR3L3 | M Q S L E Y P V T | 26 |
| | Domaine variable chaine lourde | | | 27 |
| | | Domaine variable chaine légère | | 28 |
| 15G9 | CDR4H1 | | G Y T F T D Y A | 29 |
| | CDR4H2 | | I R T Y S G D A | 30 |
| | CDR4H3 | | A T G F N Y | 31 |
| | | CDR4L1 | Q S L L H S N G K T Y | 32 |
| | | CDR4L2 | L V S | 33 |
| | | CDR4L3 | L Q A T H F P H T | 34 |
| | Domaine variable chaine lourde | | | 35 |
| | | Domaine variable chaine légère | | 36 |
| 10B2 | Chaîne lourde | | | 37 |
| | | | | |
| | Chaîne légère | | | 38 |
| 1B1 | Chaîne lourde | | | 39 |
| | Chaîne | | | 40 |
| | légère | | | |
| 18E1 | Chaîne lourde | | | 41 |
| | Chaîne légère | | | 42 |
| | | | | |
| 15G9 | Chaîne lourde | | | 43 |
| | Chaîne légère | | | 44 |

La présente description concerne aussi un acide nucléique sélectionné parmi :
a. ADN ou ARN codant pour un anticorps, ou un fragment fonctionnel, selon la présente description ou ADN codant pour une chaîne lourde ou une chaîne légère d'un anticorps selon la présente description,
b. Séquence d'ADN comprenant SEQ ID 45, SEQ ID 46, SEQ ID 47, SEQ ID 48, SEQ 49, SEQ ID 50, SEQ ID 51, SEQ ID 52, SEQ ID 53, SEQ ID 54, SEQ ID 55, SEQ ID 56, SEQ ID 57, SEQ ID 58, SEQ ID 59, SEQ ID 60, SEQ ID 61, SEQ ID 62, SEQ ID 63, SEQ ID 64, SEQ ID 65, SEQ ID 66, SEQ ID 67, SEQ ID 68, SEQ ID 69, SEQ ID 70, SEQ ID 71, SEQ ID 72, SEQ ID 73, SEQ ID 74, SEQ ID 75, SEQ ID 76.
c. Séquence d'ARN transcrit à partir d'une séquence d'ADN ci-dessus.
La présente description concerne aussi un vecteur comprenant une séquence acide nucléique selon la description. La description concerne aussi notamment un vecteur comprenant un ADN codant pour une chaîne lourde d'un anticorps selon la présente description et un ADN codant pour une chaîne légère d'un anticorps selon la présente description.

Dans un mode de réalisation, la présente description vise une cellule hôte contenant un vecteur comprenant une séquence d'acide nucléique selon la description.

Les vecteurs selon la présente description contiennent des éléments permettant l'expression des séquences nucléiques dans la cellule hôte.

Le terme vecteur utilisé ici se réfère à une séquence d'acide nucléique capable de transporter une autre séquence d'acide nucléique auquel il a été attaché/lié. Par exemple, il peut s'agir d'un plasmide ou de vecteurs viraux.

**Tableau 3 : Séquences SEQ ID N° des séquences nucléotidiques des composantes des anticorps selon la description**

| Anticorps | Chaine lourde | Chaine légère | Séquence nucléotidique | SEQ ID N° |
|---|---|---|---|---|
| 1B1 | CDR1 H1 | | GGCTACACCTTCACCAACTACTGG | 45 |
| | CDR1H2 | | ATTGATCCTTCTGATAGATATACT | 46 |
| | CDR1 H3 | | ACAATGTATTCGTTTGCTTAC | 47 |
| | | CDR1 L1 | | 48 |
| | | CDR1 L2 | CTGGTGTCT | 49 |
| | | CDR1 L3 | GTGCAAGGTGCACATCTCCCTCAAACG | 50 |
| | Domaine variable chaine lourde | | | 51 |
| | | Domaine variable chaine légère | | 52 |
| 18E1 | CDR1 H1 | | GGTGACACCTTCACAGACTATCCA | 53 |
| | CDR1 H2 | | ATAAACGCTGAGACTGCTGAGCCA | 54 |
| | CDR1H3 | | GCTAGTTCCTATGGTTAC | 55 |
| | | CDR1 L1 | | 56 |
| | | CDR1 L2 | CTGGTGTCT | 57 |
| | | CDR1L3 | GTGCAAGGTTCACATTTCCCTCATACG | 58 |
| | Domaine variable chaine lourde | | | 59 |
| | | | | |
| | | Domaine variable chaine légère | | 60 |
| 15G9 | CDR1 H1 | | GGCTACACATTCACTGATTATGCT | 61 |
| | CDR1 H2 | | ATTCGTACTTACTCTGGTGATGCT | 62 |
| | CDR1 H3 | | GCAACCGGGTTTAACTAC | 63 |
| | | CDR1 L1 | | 64 |
| | | CDR1 L2 | CTGGTGTCT | 65 |
| | | CDR1 L3 | TTGCAAGCTACACATTTTCCTCATACG | 66 |
| | Domaine variable chaine lourde | | | 67 |
| | | Domaine variable chaine légère | | 68 |
| 10B2 | CDR1 H1 | | GGATACACGTTCACTGATTACTAC | 69 |
| | CDR1 H2 | | ATTAATCCTAATAATGGTGGTAGT | 70 |
| | CDR1 H3 | | | 71 |
| | | CDR1 L1 | | 72 |
| | | CDR1 L2 | TATATGTCC | 73 |
| | | CDR1 L3 | ATGCAAAGTCTAGAATATCCTGTCACG | 74 |
| | Domaine variable chaine lourde | | | 75 |
| | | Domaine variable chaine légère | | 76 |
| | | | | |

La présente description concerne aussi une cellule hôte transformée/transfectée/transduite par ou comprenant un vecteur selon la description. La cellule hôte selon la description peut être une cellule procaryote ou eucaryote, par exemple une bactérie, une levure, une cellule animale, en particulier une cellule de mammifère.

Un autre aspect de la présente description comprend une méthode de production d'un anticorps selon la description, ou un fragment fonctionnel ayant des propriétés de liaison antigéniques, caractérisée en ce que cette méthode comprend les étapes suivantes :
- faire croitre une cellule hôte selon la description dans un milieu approprié et dans des conditions appropriées,
- récupérer l'anticorps ou un fragment fonctionnel ayant des propriétés de liaison antigéniques.

La récupération pourra se faire par tout moyen de purification classique connu de l'homme du métier tel que la chromatographie, par exemple.

Les anticorps selon la présente description présentent la propriété particulière de pouvoir se lier au domaine central de l'ADN polymérase Pol Thêta sous forme entière, et non seulement une forme tronquée et ce en western blot ou dans son environnement cellulaire, plus particulièrement au sein d'une coupe histologique de tissus et révélé en immunohistochimie en particulier. Dans un mode de réalisation de la présente description l'anticorps monoclonal selon la description est ainsi caractérisé par sa capacité de se lier au domaine central de l'ADN polymérase Thêta au sein de cellules ou coupes histologique de tissus.

Dans un mode de réalisation particulier de la présente description, les anticorps monoclonaux selon la description, sont caractérisés par leur capacité à se lier au domaine central de l'ADN polymérase Pol Thêta et permettent la détection de l'ADN polymérase Pol Thêta en immunohistochimie ou immunofluorescence, préférentiellement en immunohistochimie.

La présente description concerne des anticorps tels que décrits supra pour leur utilisation *in vitro* ou *ex vivo* à des fins de diagnostic et/ou de pronostic d'un désordre pathologique hyperprolifératif associé à l'expression de l'ADN polymérase Pol Thêta. Plus particulièrement l'expression de l'ADN polymérase Pol Thêta est une expression aberrante. Plus particulièrement encore, l'expression aberrante de l'ADN Polymérase Pol Thêta est une surexpression.

Dans un mode de réalisation particulier, le désordre pathologique hyperprolifératif est un cancer.

La présente description concerne une méthode pour déterminer *in vitro* ou *ex vivo* la présence d'une tumeur sur-exprimant l'ADN polymérase Pol Thêta chez un sujet comprenant les étapes suivantes :
a. Mettre en contact un échantillon biologique obtenu du sujet avec un anticorps selon la présente description,
b. Détecter la liaison de l'anticorps avec l'ADN polymérase Pol Thêta au sein de l'échantillon.

L'expression «diagnostic» s'entend dans le contexte de la présente description comme définissant un procédé d'identification ou de détection de la présence d'un désordre pathologique hyperprolifératif, en particulier un cancer, associé à ou provoqué par l'expression, en particulier l'expression aberrante, plus particulièrement encore la surexpression, de l'ADN polymérase Pol Thêta.

La notion de diagnostic englobe la notion de surveillance de la progression de la maladie ainsi que l'identification de cellules, de tissus ou d'échantillons indicateurs d'un désordre associé à ou provoqué par l'expression, en particulier l'expression aberrante, plus particulièrement encore la surexpression, de l'ADN polymérase Pol Thêta.

La notion de pronostic s'entend dans le contexte de la présente description, comme la probabilité ou la prédiction ou la prédisposition au développement d'une maladie de type désordre pathologique hyperprolifératif, en particulier un cancer. Cette notion de pronostic inclut aussi le pronostic sur l'issue d'une telle maladie.

En particulier, si un échantillon obtenu à partir d'un sujet est négatif sur la base d'une révélation avec un anticorps selon la présente description, alors le pronostic pour ce sujet est meilleur que si l'échantillon est positif.

Les échantillons obtenus à partir d'un sujet peuvent être évalués quant au niveau d'expression de l'ADN polymérase Pol Thêta selon une échelle telle que décrit ci-après.

Tel qu'indiqué ci avant, les anticorps selon la présente description peuvent être sous la forme d'immunoconjugué ou d'anticorps marqué afin d'obtenir un signal détectable et/ou quantifiable.

Lorsqu'ils sont utilisés - marqués ou conjugués - avec tout autre réactif détectable approprié, les anticorps selon la présente description sont particulièrement adaptés pour des applications de diagnostic ou pronostic *in vitro* ou *ex vivo* mettant en oeuvre des techniques d'immunohistochimie ou d'immunofluorescence.

Dans le contexte de la présente description, l'expression « désordre pathologique hyperprolifératif associé à l'expression de l'ADN polymérase Pol Thêta » se réfère à une maladie pour laquelle la présence de hauts niveaux, ou niveaux aberrants, d'expression de l'ADN polymérase Pol Thêta, chez un sujet souffrant d'une telle maladie, a été mise en évidence.

De tels désordres peuvent être ainsi mis en évidence par une augmentation du niveau d'expression de l'ADN polymérase Pol Thêta par l'utilisation d'anticorps selon la présente description.

C'est ainsi un objet de la présente description que de fournir une méthode pour déterminer *in vitro* ou *ex vivo* le niveau d'expression de l'ADN polymérase Pol Thêta chez un sujet comprenant les étapes suivantes :
a. mettre en contact un échantillon biologique obtenu du sujet avec un anticorps selon la présente description,
b. quantifier le niveau d'expression de l'ADN Polymérase Pol Thêta au sein de l'échantillon.

Dans le contexte de la présente description, le niveau d'expression de l'ADN Polymérase Pol Thêta est évalué *via* la liaison des anticorps avec la protéine. Plus la protéine est présente, plus il y aura de liaisons détectables et plus l'intensité du signal sera importante.

C'est encore un objet de la présente description que de fournir une méthode pour évaluer *in vitro* ou *ex vivo* la gravité ou l'agressivité d'une tumeur ou d'un cancer chez un sujet comprenant les étapes suivantes :
a. mettre en contact un échantillon biologique de tumeur obtenu du sujet avec un anticorps selon la présente description,
b. quantifier le niveau d'expression de l'ADN polymérase Pol Thêta au sein de l'échantillon,
c. évaluer la tumeur ou le cancer par comparaison du niveau d'expression de l'ADN polymérase Pol Thêta à une échelle.

Selon la présente description, l'expression «agressivité» d'un cancer ou d'une tumeur s'entend comme étant la propension de ce cancer ou de cette tumeur à envahir les tissus voisins et de générer des métastases ainsi que la rapidité avec laquelle ces invasions se manifestent.

L'agressivité est bien sûr liée au taux de survie et la méthode selon la description peut aussi servir au pronostic de survie du patient.

Dans un tel cas de figure, un diagnostic d'agressivité signifie un mauvais pronostic de survie et l'absence d'agressivité signifie un bon pronostic de survie.

Un diagnostic d'agressivité ou d'absence d'agressivité s'évalue *via* une échelle en comparant le niveau d'expression de l'ADN Polymérase Pol Thêta au sein de l'échantillon par rapport à des témoins positifs ou négatifs.

En particulier, selon la présente description, la détection, au sein de l'échantillon est réalisée par immunohistochimie.

C'est un objet de la présente description que de fournir une méthode telle que décrite ci-avant dans laquelle la détection de la liaison de l'anticorps ou la quantification du niveau d'expression de l'ADN Polymérase Pol Thêta au sein de l'échantillon est réalisée par immunohistochimie

Dans une autre forme de réalisation, la description concerne une méthode de détection de cellules cancéreuses présentant une instabilité génétique. L'expression dérégulée de l'ADN polymérase Pol Thêta mène à une augmentation des dommages à l'ADN et une instabilité chromosomique, ce qui déclenche l'activation constitutive de « [gamma]H2AX-ATM-CHK2 DNA damage checkpoint ».

L'expression "instabilité génétique" d'un cancer signifie la propension des cellules tumorales du cancer à souffrir de dommages de l'ADN.

Par l'expression "dommages à l'ADN" il est entendu une ou des modifications de l'ADN affectant le fonctionnement normal de l'ADN en causant la création de liaisons covalentes ou en modifiant des liaisons covalentes et entrainant un ou des changements dans la conformation normale en double hélice de l'ADN. De tels changements entrainent à leur tour des distorsions de structure interférant avec la réplication et la transcription. Généralement, lorsque une cellule subit un dommage à l'ADN, le cycle cellulaire est stoppé ce qui peut mener à l'activation d'un processus de réparation de l'ADN (en cas de dommage mineur) ou à l'apoptose (en cas de dommage majeur).

Les dommages à l'ADN peuvent arriver de manière spontanée ou être induits par des facteurs environnementaux tels que les radiations (rayons X, UV, rayons gamma ou radiothérapie), les toxines (par exemple les toxines des plantes ou les toxines de synthèse) mais aussi par les drogues telles que les chimiothérapies anticancéreuses.

Ainsi, la présente description fournit une méthode pour le diagnostic de l'instabilité d'un cancer chez un sujet à partir d'un échantillon cancéreux d'un tel sujet comprenant :
a. mettre en contact un anticorps selon la description avec un échantillon cancéreux obtenu du sujet
b. quantifier le niveau d'expression de l'ADN polymérase Pol Thêta dans l'échantillon testé
c. quantifier le niveau d'expression de l'ADN polymérase Pol Thêta dans un échantillon sain du même sujet
d. mesurer le ratio entre le niveau d'expression obtenu à l'étape b et le niveau obtenu à l'étape c,
dans laquelle le ratio fourni une information sur l'instabilité génétique du cancer.

Dans un mode de réalisation particulier, un ratio supérieur à 1 indique un risque, ou un risque accru, d'instabilité génétique du cancer. A contrario, un ratio inférieur ou égal à 1 indique un risque faible ou une absence de risque d'instabilité génétique du cancer.

Dans un mode particulier, le niveau d'expression de l'ADN polymérase Pol Thêta est évalué en immunohistochimie selon l'échelle ALLRED.

Dans un mode de réalisation particulier, un ratio supérieur à 1,5 indique un risque, ou un risque accru, d'instabilité génétique du cancer. A contrario, un ratio inférieur ou égal à 1,5 indique un risque faible ou une absence de risque d'instabilité génétique du cancer.

Dans un autre mode de réalisation particulier, un ratio supérieur à 2 indique un risque, ou un risque accru, d'instabilité génétique du cancer. A contrario, un ratio inférieur ou égal à 2 indique un risque faible ou une absence de risque d'instabilité génétique du cancer.

L'expression « échantillon sain » s'entend ici, dans le contexte de la présente description, comme d'un échantillon non cancéreux.

De manière préférée, la quantification du niveau de liaison de l'anticorps à l'ADN Polymérase Pol Thêta et/ou du niveau d'expression de l'ADN Polymérase Pol Thêta est réalisée *via* l'échelle appropriée, telle que par exemple l'échelle ALLRED.

Dans une forme de réalisation particulière de la description, l'échelle appropriée, par exemple l'échelle ALLRED, est basée sur deux paramètres qui sont l'intensité de la coloration et le pourcentage de cellules positives.

De manière encore plus préférentielle de réalisation, l'échelle appropriée, telle que l'échelle ALLRED, est une échelle de 0 à 8 dans laquelle « sans réactivité » est évaluée au niveau 0, et une forte réactivité dans une proportion de « 67 à 100% réactif » est évaluée au niveau 8

Une fois la quantification du niveau de liaison de l'anticorps à l'ADN polymérase Pol Thêta, reflétant la quantité d'ADN polymérase Pol Thêta présente dans l'échantillon et donc le niveau d'expression de l'ADN Polymérase Pol Thêta dans l'échantillon, le résultat peut être comparé à celui obtenu à partir d'un échantillon témoin, normal, obtenu, préparé et traité de la même manière mais à partir d'un sujet qui ne présente pas de désordre pathologique hyperprolifératif tel que cancer. Dans un mode de réalisation particulier, l'échantillon témoin - préparé et traité de la même manière - est obtenu à partir du même sujet et provient d'un tissu non cancéreux de ce même sujet. Un tel échantillon témoin est considéré comme un témoin négatif et son score dans une échelle ALLRED serait de zéro ou proche de zéro.

Si le niveau mesuré est significativement plus élevé dans l'échantillon testé par rapport à l'échantillon témoin, il peut être conclu que le sujet a un mauvais pronostic de survie ou que l'échantillon cancéreux est génétiquement instable.

Un niveau significativement plus élevé correspond par exemple au niveau 3 et au-delà de l'échelle ALLRED, particulièrement au niveau 5 et au-delà de l'échelle ALLRED.

En ce qui concerne le développement de thérapie anticancéreuse ciblée, le diagnostic à l'aide de méthodes immunohistochimiques selon la description donne des informations cruciales sur le niveau d'expression de l'ADN Polymérase Pol Thêta et permet la sélection de patient, ou de déterminer l'éligibilité de patient, pour un traitement de chimiothérapie, en particulier un traitement de chimiothérapie adjuvant, ou un traitement de radiothérapie.

En effet, la capacité pronostique et prédictive de l'évolution d'un cancer selon les méthodes de la présente description qui est associée à la détermination de l'agressivité dudit cancer est cruciale pour la sélection du traitement adéquat sachant que des traitements lourds et couteux avec des effets secondaires sévères, en complément des traitements chirurgicaux, ne devraient être utilisés que lorsqu'ils sont nécessaires et seulement lorsqu'ils sont nécessaires.

La présente description concerne ainsi une méthode pour sélectionner un traitement adéquat d'un cancer chez un patient ayant subi un traitement chirurgical, comprenant :
- diagnostiquer l'agressivité ou la non agressivité d'un cancer chez un patient en utilisant la méthode selon la description et
- ajouter un traitement de chimiothérapie ou radiothérapie au traitement chirurgical si ledit cancer est diagnostiqué comme agressif.

L'évaluation de l'agressivité ou de la gravité d'un cancer selon une méthode telle que décrite plus avant peut être réalisée par comparaison du niveau quantifié d'expression de l'ADN polymérase Pol Thêta à une échelle. Particulièrement il peut s'agir par exemple de l'échelle ALLRED.

Dans une forme encore simplifiée, cette évaluation peut aussi être catégorisée en négative ou positive par un examen visuel des échantillons d'immunohistochimie par un spécialiste en anatomopathologie.

Une approche plus objective et quantitative de l'évaluation du niveau d'expression de l'ADN polymérase Pol Thêta peut être réalisée *via* la mesure de deux paramètres qui sont la proportion de cellules colorées et l'intensité de ladite coloration (c'est-à-dire positives) au sein de l'échantillon testé.

Dans un mode de réalisation particulier d'une méthode selon la description, la quantification du niveau d'expression de l'ADN polymérase Pol Thêta est réalisée selon l'échelle ALLRED. Cette échelle est basée sur la combinaison de deux paramètres : l'intensité de la coloration et le pourcentage de cellules colorées (Harvey et al ; J Clin. Oncol. 1999 ; 17 ; 1474-1481).

Le tableau 4 illustre l'utilisation de résultats d'immunohistochimie selon l'échelle ALLRED.

**Tableau 4 : Evaluation des scores de résultats d'immunohistochimie selon échelle ALLRED.**

| Intensité de la réaction | Score 1 | Proportion de cellules réactives | Score 2 |
|---|---|---|---|
| Aucune réactivité | 0 | Aucune | 0 |
| Réactivité faible | 1 | 0-1% | 1 |
| Réactivité modérée | 2 | 1-10% | 2 |
| Forte réactivité | 3 | 11-33% | 3 |
| | | 34-67% | 4 |
| | | 67-100% | 5 |
| | | | |
| SCORE TOTAL (Score 1 + Score 2) | Interprétation | | |
| 0-2 | Négatif | | |
| 3-8 | Positif | | |

Ainsi dans une méthode selon la présente description, un score total compris entre 0 et 2 pourra être compris comme négatif en ce qui concerne l'agressivité du cancer par exemple et un score compris entre 3 et 8 pourra être considéré comme positif en ce qui concerne l'agressivité du cancer.

C'est un autre objet de la présente description que de fournir une méthode d'évaluation du statut d'une tumeur chez un sujet, *in vitro* ou *ex vivo,* comprenant les étapes :
- mettre en contact un échantillon de tumeur obtenu du sujet avec un anticorps selon la présente description, en immunohistochimie,
- mesurer le score de la tumeur sur l'échelle ALLRED entre 0 et 8
- déterminer que la tumeur a un statut POLQ(-) (ou Pol Thêta -) si le score est compris entre 0 et 2,
- déterminer que la tumeur a un statut POLQ(+) (ou Pol Thêta +) si le score est compris entre 3 et 8.

Dans un aspect particulier, on détermine que la tumeur a un statut POLQ(+) si le score est de 3.

Dans un aspect particulier, on détermine que la tumeur a un statut POLQ(+) si le score est de 4.

Dans un aspect particulier, on détermine que la tumeur a un statut POLQ(+) si le score est de 5.

Dans un aspect particulier, on détermine que la tumeur a un statut POLQ(+) si le score est de 6.

Dans un aspect particulier, on détermine que la tumeur a un statut POLQ(+) si le score est de 7.

Dans un aspect particulier, on détermine que la tumeur a un statut POLQ(+) si le score est de 8.

L'expression POLQ(+) (ou Pol Thêta +) signifie que l'échantillon évalué présente une expression aberrante de l'ADN polymérase Pol Thêta, plus particulièrement une surexpression de l'ADN polymérase Pol Thêta.

L'expression POLQ(-) (ou Pol Thêta -) signifie que l'échantillon évalué ne présente pas une expression aberrante de l'ADN polymérase Pol Thêta, plus particulièrement ne présente pas une surexpression de l'ADN polymérase Pol Thêta.

Le test ALLRED présenté ici représente une méthode de quantification parmi d'autres et l'homme du métier ne sera pas limité pour l'évaluation de l'expression de l'ADN polymérase Pol Thêta dans l'échantillon testé et toute autre méthode visant à conclure quant à l'expression aberrante, en particulier la surexpression, de cette protéine pourra être envisagée.

La présente description fournit aussi une méthode d'évaluation du risque de développement ou de progression d'un cancer chez un patient, cette méthode comprenant la mesure du niveau d'expression de l'ADN polymérase Pol Thêta dans un échantillon biologique obtenu du patient.
Ainsi la description concerne une méthode d'évaluation du risque de développement ou de progression d'un cancer chez un sujet, comprenant les étapes :
a. mettre en contact un anticorps selon la description avec un échantillon cancéreux obtenu du sujet
b. quantifier le niveau d'expression de l'ADN polymérase Pol Thêta dans l'échantillon testé
c. quantifier le niveau d'expression de l'ADN polymérase Pol Thêta dans un échantillon équivalent du même sujet à un moment plus tardif
d. mesurer le ratio entre le niveau d'expression obtenu à l'étape b et le niveau obtenu à l'étape c,
dans laquelle le ratio fourni une information sur le risque de développement ou de progression du cancer.

Dans un mode de réalisation particulier, un ratio inférieur à 1 indique un risque, ou un risque accru, de développement ou de progression d'un cancer. A contrario, un ratio supérieur ou égal à 1 indique un risque faible ou une absence de risque de développement ou de progression d'un cancer.

Dans un mode de réalisation plus particulier, un ratio inférieur à 1,5 indique un risque, ou un risque accru, de développement ou de progression d'un cancer. A contrario, un ratio supérieur ou égal à 1,5 indique un risque faible ou une absence de risque de développement ou de progression d'un cancer.

Dans un mode de réalisation plus particulier, un ratio inférieur à 2 indique un risque, ou un risque accru de développement ou de progression d'un cancer. A contrario, un ratio supérieur ou égal à 2 indique un risque faible ou une absence de risque de développement ou de progression d'un cancer.

Dans un mode particulier, le niveau d'expression de l'ADN polymérase Pol Thêta est évalué en immunohistochimie selon l'échelle ALLRED.

Dans le contexte de la description, par l'expression «échantillon cancéreux », il est fait référence ici à un échantillon de tissu tumoral. L'échantillon cancéreux, ou l'échantillon de cancer, peut être un échantillon de cancer solide provenant d'une biopsie ou d'une thérapie chirurgicale de résection.

L'échantillon de cancer solide est un échantillon contenant des cellules cancéreuses et peut être préparé selon des techniques usuelles histologiques connues de l'homme du métier afin de préparer cet échantillon pour son évaluation en immunohistochimie ou en immunofluorescence, par exemple.

Dans le cadre de la présente description, le terme cancer signifie une condition physiologique caractérisée par une hyperprolifération cellulaire, plus particulièrement une hyperprolifération pathologique.

Les termes "cancer" ou "cancéreux" correspondent ici à tous les stades de la maladie. Avantageusement, le cancer est un cancer à un stade précoce ou moyennement avancé. Par les expressions « précoce » ou « moyennement avancé », on entend ici un cancer dont le stade est compris entre le stade IA et le stade IIIA. Dans un mode de réalisation préféré de la présente description, le cancer est un cancer à un stade précoce.

Plus particulièrement, un cancer selon la présente description peut être choisi parmi le cancer du poumon, le cancer du poumon à petites cellules, le cancer du poumon non-à petites cellules, l'adénocarcinome du poumon, le cancer gastrointestinal, le cancer du pancréas, le cancer de la prostate, le cancer de la vessie, le cancer du foie, le cancer des ovaires, le cancer colorectal, le cancer du sein, le cancer du rein, le cancer du côlon, le cancer du cerveau, le cancer de la tête et du cou, le cancer de l'endomètre, le cancer des glandes salivaires, le cancer de la thyroïde, l'hépatocarcinome, les mélanomes, les mélanomes nodulaires, les lymphomes, les lymphomes non-Hodgkiniens (NHL), les leucémies, les leucémies lymphoblastiques, les leucémies myeloblastiques, les désordres myeloprolifératifs et les désordres lymphoprolifératifs post transplantation.

De manière particulière, le cancer est sélectionné parmi le cancer du sein, le cancer du côlon, le cancer du poumon, le cancer du poumon à petites cellules et le cancer du poumon non-à petites cellules.

De manière préférée, le cancer est le cancer du sein.

De manière préférée, le cancer est le cancer du côlon.

De manière préférée, le cancer est le cancer du poumon.

Dans les méthodes selon la description, le niveau d'expression de l'ADN polymérase Pol Thêta peut avantageusement être comparé ou mesuré en relation avec les niveaux d'expression dans un échantillon de référence ou échantillon témoin qui correspond à un échantillon non pathologique, c'est-à-dire non cancéreux. Cet échantillon témoin ou de référence peut provenir du même patient et dans ce cas ledit échantillon témoin est non cancéreux. Cet échantillon peut aussi provenir d'un autre patient qui n'est pas atteint par un cancer.

Le niveau d'expression dans l'échantillon témoin est évalué selon la même technique et la même méthodologie que l'échantillon cancéreux.

Les hybridomes capables de produire les anticorps selon la description ont été déposés à la CNCM, Institut Pasteur, Paris, France, sous les numéros et jour suivants :
L'hybridome 1B1-3D4-2C5 capable de produire l'anticorps 1B1 a été déposé sous le numéro CNCM I-4824, le 3 décembre 2013.

L'hybridome 18E1-3E12 capable de produire l'anticorps 18E1 a été déposé sous le numéro CNCM I-4827, le 3 décembre 2013.

L'hybridome 10B2-3B10-3D1 capable de produire l'anticorps 10B2 a été déposé sous le numéro CNCM I-4825, le 3 décembre 2013.

L'hybridome 15G9-3D5 capable de produire l'anticorps 15G9 a été déposé sous le numéro CNCM I-4826, le 3 décembre 2013.

L'anticorps monoclonal, désigné ici par 1B1, ou ses dérivés ou fragments fonctionnels, est caractérisé en ce qu'il est sécrété par l'hybridome 1B1-3D4-2C5, CNCM I-4824.

L'anticorps monoclonal, désigné ici par 18E1, ou ses dérivés ou fragments fonctionnels, est caractérisé en ce qu'il est sécrété par l'hybridome 18E1-3E12, CNCM I-4827.

L'anticorps monoclonal, désigné ici par 10B2, ou ses dérivés ou fragments fonctionnels, est caractérisé en ce qu'il est sécrété par l'hybridome 10B2-3B10-3D1, CNCM I-4825.

L'anticorps monoclonal, désigné ici par 15G9, ou ses dérivés ou fragments fonctionnels, est caractérisé en ce qu'il est sécrété par l'hybridome 15G9-3D5, CNCM I-4826.

Un autre objet de la présente description comprend un kit, c'est-à-dire un ensemble de réactifs emballés en quantité prédéterminée avec instructions d'usage afin de réaliser des essais de diagnostic et/ou de pronostic selon les méthodes de la présente description.

Un kit selon la description peut comprendre au moins un anticorps selon la présente description pour la détection et/ou la quantification de l'expression de l'ADN polymérase Pol Thêta *in vitro* ou *ex vivo.* De manière préférentielle, le kit est destiné à un usage en immunohistochimie ou immunofluorescence.

Dans un mode particulier de réalisation de la description, un anticorps selon la description est marqué, par exemple par une enzyme et le kit peut comprendre les substrats et cofacteurs nécessaires.

Selon un autre aspect de réalisation de la description, les anticorps selon la présente description, ou leurs fragments fonctionnels tels que décrits supra, sont marqués avec une partie détectable de manière à être préparés et commercialisés sous forme de kit pour l'identification et/ou pour le diagnostic de tissus ou de cellules selon une des méthodes selon la présente description ici décrite.

Des exemples non limitatifs de tels marquages comprennent les fluorophores tels que l'isothiocyanate de fluorescéine; des chromophores, des radionucléotides, biotine ou enzymes.

Les anticorps selon la description ainsi marqués peuvent être utilisés pour les tests de localisation histologique de l'ADN Polymérase Pol Thêta sur des échantillons tissulaires, particulièrement en immunohistochimie ou immunofluorescence.

Dans une forme particulière de la description, le kit selon la description comprend un réactif pour détecter la liaison de l'anticorps à l'ADN polymérase Pol Thêta.

Dans un autre mode de réalisation de la description, le kit comprend un réactif pour quantifier le niveau de liaison de l'anticorps selon la description avec l'ADN polymérase Pol Thêta.

Dans une autre forme de réalisation de la description, le kit comprend en outre un anticorps monoclonal ou polyclonal reconnaissant les anticorps murins. De manière avantageuse, cet anticorps monoclonal ou polyclonal est marqué.

Les exemples suivants servent à illustrer les différents aspects de la description.

### Exemple 1 : Peptides antigéniques & production des anticorps selon la description

Les trois peptides suivants ont été utilisés pour réaliser l'immunisation de souris OF1 afin de produire les anticorps selon la description

| Peptides | Séquence amino acide | SEQ ID N° |
|---|---|---|
| Peptide 1 | CKHSPNIVQDLNKSREHTSS | 1 |
| Peptide 2 | CSIFRARKRASLDINKEKPG | 2 |
| Peptide 3 | CLQEDLIKKSNVNENQDTH | 3 |
| Peptide 4 | HDETSSLLPRKESNIVDDNGC | 4 |

Les peptides sont couplés à la KLH (aux fins d'immunisation) et à la BSA (pour criblage des hybridomes). 3 souris sont immunisées avec le mélange KLH-peptides 1 + 2 et 3 souris sont immunisées avec le mélange KLH-peptide 4. Les sérums sont prélevés et testés pour la reconnaissance des peptides libres par Elisa et de l'ADN Polymérase Pol Thêta sous sa forme pleine taille par western blot.

Les peptides 1 et 4 comprennent un C en position 1 et en position terminale, respectivement. Ce résidu cystéine n'est pas présent dans la séquence de POLQ et cet acide aminé a été ajouté pour faciliter l'immunisation. Les anticorps reconnaissent bien cette séquence mais cependant dans la séquence de POLQ il n'y a pas ce résidu cystéine.

Les meilleures souris sont retenues pour la fusion de leurs lymphocytes avec des cellules du myélome Sp2/O-Ag14.

Les clones sont criblés, sélectionnés par test ELISA sur les peptides couplés à la BSA puis testés pour leur capacité à reconnaitre l'ADN Polymérase Thêta en western blot et immunofluorescence sur cellules de mammifère. Les hybridomes sélectionnés après sous clonage et stabilisation 1B1-3D4-2C5 ; 18E1-3E12 ; 10B2-3B10-3D1 et 15G9-3D5 ont été utilisés pour la production et purification des anticorps 1B1, 18E1, 10B2 et 15G9, respectivement.

### Exemple 2 : Immunodétection par western blot de l'ADN polymérase Thêta ectopique sur des fibroblastes pulmonaires surexprimant stablement l'ADN polymérase Thêta.

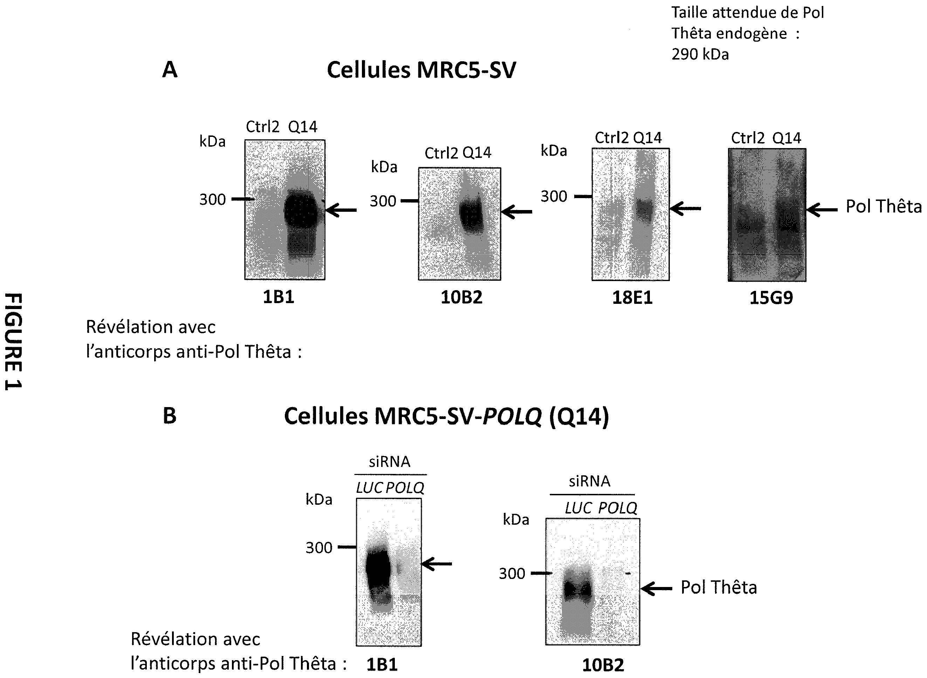

### Légende de la figure 1

Figure 1 : (A) Révélation par western blot de l'ADN polymérase Thêta ectopique sous sa forme pleine taille grâce à l'utilisation des anticorps anti-Pol Thêta 1B1, 10B2, 18E1 et 15G9, dans des fibroblastes de poumon MRC5-SV surexprimant (Q14) ou non (Ctrl2) Pol Thêta de façon stable.
(B) La spécificité du signal généré par les anticorps anti-Pol Thêta 1B1 et 10B2 a été confirmée dans les fibroblastes de poumons surexprimant stablement Pol Thêta (MRC5-SV-*POLQ* : Q14), 24h après transfection par un siRNA contrôle dirigé contre la luciférase (siRNA *LUC*) ou un siRNA dirigé spécifiquement contre *POLQ* (siRNA *POLQ*).

### Résultats

L'utilisation des anticorps anti-Pol Thêta 1B1, 10B2, 18E1 et 15G9 provenant des surnageants des hybridomes permettent la détection de l'ADN polymérase Thêta ectopique sous sa forme pleine taille dans les cellules MRC5-SV-*POLQ* (Q14) surexprimant la protéine Pol Thêta de façon stable, en comparaison avec les cellules qui ne la surexpriment pas (cellules Ctrl2 transfectées par le vecteur vide) (Figure 1-A). La spécificité des anticorps 1B1 et 10B2 pour Pol Thêta a été confirmée dans les échantillons Q14 grâce à l'utilisation de siRNA dirigés spécifiquement contre *POLQ,* aboutissant à l'extinction de la protéine et donc du signal généré par ces anticorps (Figure 1-B). Un résultat identique est obtenu lorsque les anticorps anti-Pol Thêta purifiés sont utilisés en western blot à la dilution de 0.1µg/ml.

### Matériel & Méthodes

La lignée cellulaire MRC5-SV a été stablement transfectée soit par un vecteur vide (pcDNA3.1, Ctrl2) soit par un vecteur codant pour l'ADN polymérase Thêta (pcDNA3.1-*POLQ*, Q14). Les cellules Ctrl2 et Q14, après trypsinisation et lavages au PBS, ont été incubées dans la glace durant 30 min dans le tampon de lyse (Tris-HCI: 50mM, pH 7.5, NaCl: 250mM, EDTA: 1mM, Triton X100: 0.1%, inhibiteurs de protéases). Après centrifugation à 13000 rpm pendant 20 min, le surnageant est prélevé, dosé et conservé dans du Leammli. 40 µg de protéines ont été déposés sur gel gradient Tris Acétate 3-8% (Nupage Novex Invitrogen) ou sur gel 6% acrylamide. Après migration à 150V pendant 1h15, les protéines ont été transférées sur une membrane de PVDF dans du tampon froid contenant du CAPS (Sigma) 10mM à pH 11, de l'éthanol 10% et du SDS 0.01%, pendant 2h à 200mA sous agitation. Les sites non spécifiques de la membrane ont été ensuite saturés pendant 1h dans du tampon PBS-Tween 0.1% (PBS-T 0.1%), 5% lait écrémé. La membrane est ensuite incubée durant une nuit à 4°C dans du PBS-T 0.1% avec les différents anticorps : anticorps anti-Pol Thêta 1B1, 10B2, 18E1 ou 15G9 (surnageants des hybridomes dilués au 10^{ème}). Après 3 lavages de 10 min au PBS-T 0.1%, les membranes ont été incubées avec l'anticorps secondaire anti-souris couplé HRP au 10 000ème (Jackson Immunoresearch), dans du PBS-T 0.1% pendant 1h à température ambiante. Apres 3 nouveaux lavages de 10 min au PBS-T 0.1%, la révélation est effectuée avec de l'ECL-plus (Pierce).

Afin de s'assurer de la spécificité du signal généré par les anticorps anti-Pol Thêta 1B1 et 10B2, les cellules surexprimant de façon stable la protéine Pol Thêta ectopique (Q14) ont été transfectées par des siRNA ciblant soit la luciférase (siRNA *LUC*, Sigma) soit spécifiquement l'ADN polymérase Thêta (siRNA *POLQ*, on Target plus smart pool Dharmacon L-015180-01) à 60nM avec la lipofectamine 2000 (Invitrogen) suivant les instructions du fournisseur. 24h après la transfection, les cellules ont été récupérées par trypsinisation, lavées dans du PBS puis lysées dans le tampon de lyse. Les extraits cellulaires ont été traités comme précédemment décrit.

### Exemple 3 : immunodétection par western blot de l'ADN polymérase Thêta endogène dans des cellules tumorales colorectales RKO.

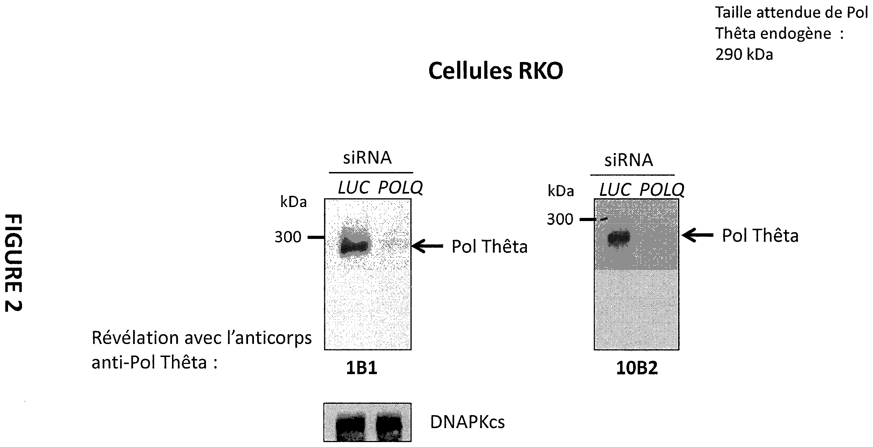

### Légende de la figure 2

Figure 2 : Révélation par western blot de l'ADN polymérase Thêta endogène sous sa forme pleine taille (290 kDa) grâce à l'utilisation des anticorps anti-Pol Thêta 1B1 et 10B2 dans des cellules de cancer colorectal (RKO), 24h après transfection par un siRNA contrôle dirigé contre la luciférase (siRNA *LUC*) ou un siRNA dirigé spécifiquement contre *POLQ* (siRNA POLQ*).*

### Résultats

L'utilisation des surnageants d'hybridomes contenant l'anticorps anti-Pol Thêta 1B1 ou 10B2 permet la détection de l'ADN polymérase Thêta endogène sous sa forme pleine taille (290kDa) dans les cellules RKO. Le signal généré par les anticorps anti-Pol Thêta 1B1 ou 10B2 est éteint après extinction de la protéine Pol Thêta suite à la transfection par le siRNA *POLQ,* démontrant ainsi la spécificité des anticorps utilisés pour la détection de l'ADN Pol Thêta (Figure 2). Le western blot DNA-PKcs est utilisé comme témoin de charge protéique. Un résultat identique est obtenu lorsque les anticorps anti-Pol Thêta purifiés sont utilisés en western blot à la dilution de 0.1µg/ml.

### Matériel & Méthodes

La lignée cellulaire RKO a été transfectée par des siRNA ciblant soit la luciférase (siRNA *LUC,* Sigma-Aldrich), soit l'ADN polymérase Thêta (siRNA *POLQ,* on Target plus smart pool Dharmacon L-015180-01) à 60nM avec la lipofectamine 2000 (Invitrogen) suivant les instructions du fournisseur. 24h après transfection, les cellules ont été récupérées par trypsinisation, lavées dans du PBS puis incubées 30 min dans le tampon de lyse (Tris-HCI: 50mM, pH 7.5, NaCl : 250mM, EDTA: 1mM, Triton X100: 0.1%, inhibiteurs de protéases) dans la glace. Après centrifugation à 13 000 rpm pendant 20 min à 4°C, le surnageant a été prélevé, dosé et conservé dans du Leammli. 40 µg de protéines ont été déposés sur gel gradient Tris Acétate 3-8% (Nupage Novex Invitrogen) ou sur gel 6% acrylamide. Après migration à 150V pendant 1h15, les protéines ont été transférées sur une membrane de PVDF dans du tampon froid contenant du CAPS (Sigma) 10mM à pH 11, de l'EtOH 10% et du SDS 0.01%, pendant 2h à 200mA sous agitation. Les sites non spécifiques de la membrane ont ensuite été saturés pendant 1h dans du tampon PBS-Tween (PBS-T) 0.1%, 5% lait écrémé. Puis la membrane a été incubée la nuit à 4°C dans du PBS-T 0.1% contenant l'anticorps anti-Pol Thêta 1B1 ou 10B2 (Surnageants d'hybridomes dilués au 10^{ème}) ou l'anticorps anti DNA-PKcs (mouse monoclonal, ab1832, Abcam, contrôle de charge) au 5000^{ème}. Après 3 lavages de 10 min au PBS-T 0.1%, les membranes ont été incubées avec l'anticorps secondaire anti-souris couplé HRP au 10 000ème (Jackson Immunoresearch), dans du PBS-T 0.1% pendant 1h à température ambiante. Après 3 nouveaux lavages de 10 min au PBS-T 0.1%, la révélation a été effectuée avec de l'ECL-plus (Pierce).

### Exemple 4 : Tests des anticorps commerciaux anti-Pol Thêta pour l'immunodétection par western blot de l'ADN Pol Thêta ectopique (dans les cellules Q14) et endogène (dans les cellules RKO)

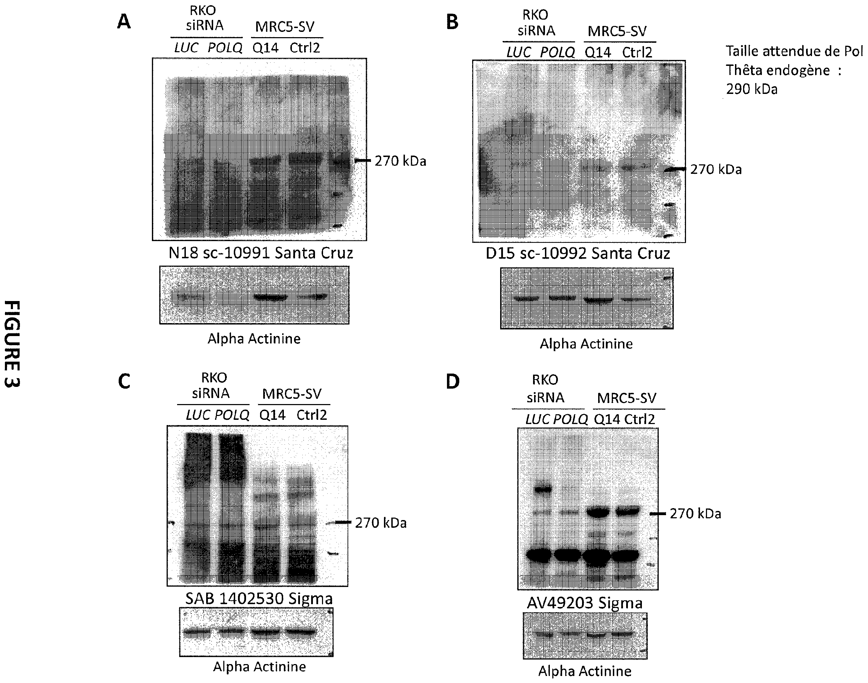

Figure 3 : Utilisation des anticorps anti-Pol Thêta vendus dans le commerce pour la détection par western blot de : (i) Pol Thêta ectopique dans des fibroblastes de poumon MRC5-SV surexprimant (Q14) ou non (Ctrl2) Pol Thêta stablement et (ii) Pol Thêta endogène dans des cellules RKO, 24h après transfection par un siRNA contrôle dirigé contre la luciférase (siRNA *LUC*) ou un siRNA dirigé spécifiquement contre *POLQ* (siRNA POLQ).

### Résultats

Les anticorps commerciaux en provenance de Sigma (SAB1402530 et AV49203) ou de Santa Cruz (N18 sc-10991 et D15 sc-10992) détectent une bande aspécifique, correspondant environ au poids moléculaire attendu pour Pol thêta dans les cellules Q14 et Ctrl2, qui ne présente pas de variation d'intensité entre les cellules Q14 qui surexpriment Pol Thêta par rapport à celles (Crtl2) qui ne la surexpriment pas (Figure 3).

Dans la lignée RKO, ces quatre anticorps détectent aussi des bandes aspécifiques aux alentours de 290 kDa qui ne diminuent pas d'intensité après extinction de la protéine par le siRNA *POLQ* (Figure 3).

L'anticorps Sigma AV49203 révèle aussi une bande de poids moléculaire supérieur à celui attendu pour Pol Thêta dans la lignée RKO (siRNA *LUC*) qui diminue après siRNA *POLQ* (Figure 3-D). Cependant la taille de cette bande ne correspond pas au poids moléculaire attendu pour Pol Thêta endogène et cet anticorps ne permet pas non plus la révélation, de manière spécifique, de la protéine Pol Thêta ectopique dans les cellules Q14 qui la surexpriment de façon stable par rapport aux cellules contrôles (Ctrl2) qui ne la surexpriment pas (Figure 3-D).

### Matériel & Méthodes

La lignée cellulaire RKO a été transfectée par des siRNA ciblant soit la luciférase (siRNA *LUC,* Sigma-Aldrich), soit l'ADN polymérase Thêta (siRNA *POLQ,* on Target plus smart pool Dharmacon L-015180-01) à 60nM avec la lipofectamine 2000 (Invitrogen) suivant les instructions du fournisseur. 24h après transfection, les cellules ont été récupérées par trypsinisation, lavées dans du PBS puis incubées 30 min dans le tampon de lyse comme précédemment décrit (Exemples 2 et 3). Les extraits cellulaires obtenus ont été traités de la même manière que décrit dans les exemples 2 et 3.

Les cellules MRC5-SV stablement transfectées soit par un vecteur vide (pcDNA3.1, Ctrl2) soit par un vecteur codant pour l'ADN polymérase Thêta (pcDNA3.1-*POLQ,* Q14) ont été récupérées par trypsinisation, lavées dans du PBS puis incubées dans la glace durant 30 min dans le tampon de lyse comme précédemment décrit (Exemples 2 et 3). Les extraits cellulaires obtenus ont été traités de la même manière que décrit dans les exemples 2 et 3.

Les membranes obtenues ont ensuite été incubées durant une nuit à 4°C dans du PBS-T 0.1% avec les différents anticorps anti-Pol Thêta vendus dans le commerce: DNA pol Thêta goat polyclonal antibodies N18 sc-10991 et D15 sc-10992 (Santa Cruz) au 200^{ème}, POLQ mouse monoclonal antibody SAB1402530 et POLQ rabbit polyclonal antibody AV49203 (Sigma) au 1000^{ème} (soit 1µg/ml) ou avec l'anticorps anti-Alpha Actinine au 1000^{ème} (MAB1682, Millipore, utilisé comme contrôle de charge). Après 3 lavages de 10 min au PBS-T 0.1%, les membranes ont été incubées avec les anticorps secondaires anti-souris, lapin ou chèvre couplés HRP au 10 000ème (Jackson Immunoresearch), dans du PBS-T 0.1% pendant 1h à température ambiante. Après 3 nouveaux lavages de 10 min au PBS-T 0.1%, la révélation est effectuée avec de l'ECL-plus (Pierce).

### Exemple 5 : Immunodétection par immunoflurorescence de l'ADN polymérase Thêta dans les fibroblastes pulmonaires MRC5-SV surexprimant stablement une forme ectopiaue de Pol Thêta (Q14).

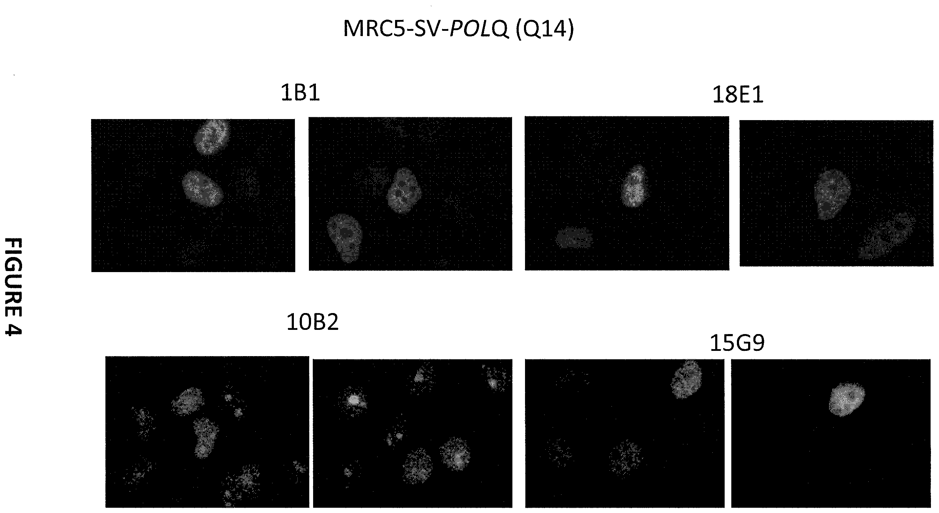

Figure 4 : Détection de Pol Thêta par immunofluorescence dans des cellules MRC5-SV surexprimant stablement la protéine Pol Thêta (MRC5-SV-*POLQ* : Q14) grâce à l'utilisation des anticorps anti-Pol Thêta 1B1, 18E1, 10B2 et 15G9.

### Résultats

On observe un marquage intense ponctiforme présent dans l'ensemble du noyau de certaines cellules (celles surexprimant fortement la protéine Pol Thêta), mis en évidence par les 4 anticorps. Le noyau des cellules est marqué en bleu et le marquage correspondant à Pol Thêta apparaît en vert (Figure 4). Un résultat identique est obtenu lorsque les anticorps anti-Pol Thêta purifiés sont utilisés en immunofluorescence à la dilution de 1µg/ml.

Il est à noter que l'anticorps anti-Pol Thêta 10B2 génère aussi un signal nucléolaire aspécifique.

### Matériel & Méthodes

Les cellules MRC5-SV-*POLQ* (Q14) surexprimant de façon stable la protéine Pol thêta ont été ensemencées sur des lamelles de verre. 24h après, les cellules ont été fixées par du PBS contenant du paraformaldéhyde 4% et du triton 0.1% durant 15 min à température ambiante. Après 3 lavages au PBS, les lamelles ont été saturées pendant une heure dans du PBS, BSA 1%, Triton 0.1%, puis lavées au PBS. Les lamelles ont ensuite été incubées en présence des anticorps anti-Pol Thêta 1B1, 18E1, 15G9 et 10B2 (surnageants des hybridomes dilués au 10^{ème}) dans du PBS-BSA 1% pendant 3h à température ambiante. 3 lavages au PBS ont été effectués puis les lamelles ont été incubées avec l'anticorps secondaire Alexa fluor anti-souris 488 (Alexa Fluor) pendant 1h. Après 3 lavages au PBS, les lamelles ont été montées sur lames à l'aide de milieu de montage Vectashield contenant du Dapi afin de marquer les noyaux. Les cellules ont ensuite été observées au microscope à fluorescence.

### Exemple 6 : Immunodétection par immunohistochimie de l'ADN polymérase Thêta dans les fibroblastes pulmonaires MRC5-SV surexprimant stablement une forme ectopique de Pol Thêta

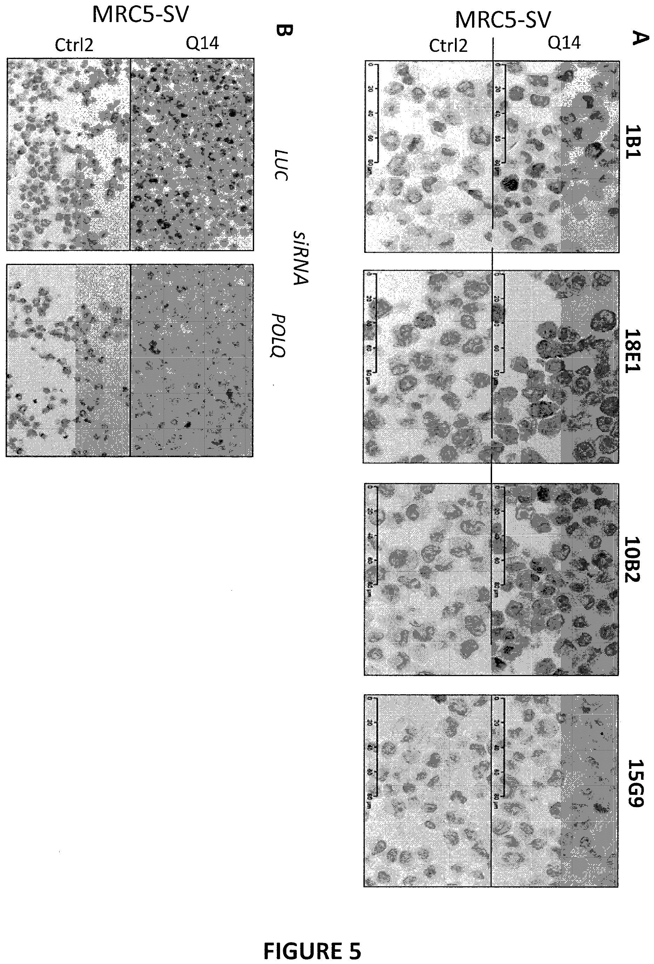

Figure 5 : (A) Détection de l'ADN Pol Thêta par Immunohistochimie avec les anticorps anti-Pol thêta 1B1 (10 µg/ml), 18E1 (2.2 µg/ml), 10B2 (1.8 µg/ml) et 15G9 (6 µg/ml) sur des coupes de fibroblastes de poumon fixés au formol puis inclus en paraffine MRC5-SV (Ctrl2) et MRC-SV-POLQ surexprimant de façon stable la protéine Pol Thêta (Q14). (B) La spécificité du signal généré par l'anticorps anti-Pol Thêta 1B1 a été confirmée dans les cellules Q14, 24h après transfection par un siRNA contrôle dirigé contre la luciférase (siRNA *LUC*) ou un siRNA dirigé spécifiquement contre *POLQ* (siRNA *POLQ*).

### Résultats

Le noyau des cellules est marqué en bleu et le marquage correspondant à la protéine Pol Thêta apparaît en brun (figure 5). La figure 5A montre un marquage majoritairement nucléaire (2+) ainsi qu'un marquage cytoplasmique moins intense dans 30% et 10 à 20% des cellules Q14 avec les anticorps 1B1 et 10B2 respectivement, alors que les cellules Ctrl2 ne sont pas marquées. En présence de l'anticorps 15G9, 5% de cellules Q14 présentent un marquage nucléaire (1+). En présence de l'anticorps 18E1, 80% des cellules Q14 présentent un marquage cytoplasmique granulaire (1+ à 2+) et 60% des cellules Ctrl2 présentent un marquage cytoplasmique (1+). La spécificité de l'anticorps 1B1 pour Pol Thêta a été confirmée dans les cellules Q14 grâce à l'utilisation de siRNA dirigés spécifiquement contre *POLQ,* aboutissant à une diminution significative du nombre de cellules surexprimant l'ADN Pol Thêta (figure 5B).

### Matériel & Méthodes

Après culture des cellules MRC5-SV Ctrl2 et Q14 à 37°C et centrifugation, elles sont fixées au formol et incluses en paraffine selon les procédures en vigueur au laboratoire d'anatomopathologie. Les blocs de tissus sont coupés au microtome à une épaisseur de 3 µm puis étalés sur lames de verre et mis à sécher sur la platine chauffante à 56°C pendant 10 min puis la nuit à l'étuve à 37°C. Les lames blanches sont stockées à 4°C. Les lames sont sorties 30 min à température ambiante. Le déparaffinage et le démasquage des sites antigéniques sont réalisés à pH9 dans l'automate PTLink (Dako) pendant 20 min. Les lames blanches sont ensuite techniquées avec le kit EnVision (Dako) à température ambiante. Entre chaque réactif mis en contact avec la coupe, celle-ci est rincée en présence d'un tampon de lavage à base de Tris pH7,6. Le blocage des peroxydases par le peroxyde d'hydrogène est réalisé pendant 5 min afin d'éliminer le bruit de fond dû à la présence de peroxydases endogènes. La coupe est incubée avec l'anticorps primaire anti- Pol Thêta pendant 20 min. L'anticorps secondaire couplé à l'enzyme HRP est incubé pendant 20 min. La lame blanche est incubée pendant 10 min en présence du substrat chromogène (DAB) puis 3 min en présence du contre colorant Hématoxyline (noyaux bleus). La lame blanche est enfin rincée à l'eau distillée avant déshydratation dans des bains d'alcool de degré croissant puis en bains de xylène. Le montage s'effectue sur un automate Tissue Tek Sakura. La lecture de la coloration et la détermination de l'intensité de cette coloration et du pourcentage de cellules tumorales positives est réalisée par un médecin anatomopathologiste. Les lames sont scannées et numérisées avec le scanner de lame Nanozoomer (Hamatsu). Les photos sont obtenues par sélection, grâce au logiciel ScreenHunter, de la partie d'intérêt de la lame numérisée à un grossissement 400x dans le logiciel NDP View.

Afin de s'assurer de la spécificité du signal généré par les anticorps anti-Pol Thêta 1B1, les cellules Ctrl2 et Q14 ont été transfectées par des siRNA ciblant soit la luciférase (siRNA *LUC,* Sigma) soit spécifiquement l'ADN polymérase Thêta (siRNA *POLQ*, on Target plus smart pool Dharmacon L-015180-01) à 60 nM avec la lipofectamine 2000 (Invitrogen) suivant les instructions du fournisseur. Les immunodétections de la figure 5B ont été réalisées dans les mêmes conditions que précédemment excepté l'utilisation supplémentaire du Mouse Linker (Dako) qui permet une amplification du signal des anticorps primaires. Le grossissement 200x a été retenu pour la visualisation de la quantité de cellules surexprimant Pol Thêta.

### Exemple 7 :Immunoprécipitation de l'ADN polymérase Pol Thêta par les anticorps 18E1, 10B2, 15G9 et 1B1.

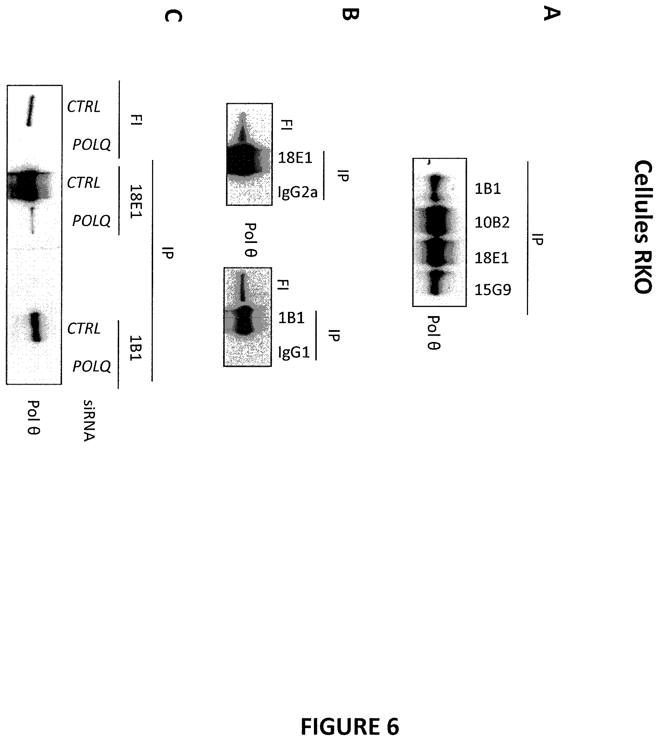

Figure 6 : (A) Détection de Pol Thêta après immunoprécipitation réalisée par les anticorps 18E1, 10B2, 1B1 ou 15G9. (B) Détection de Pol Thêta dans la fraction initiale (FI) et après immunoprécipitation (IP) réalisée par les anticorps 18E1 ou 1B1 ou par les immunoglobulines contrôles IgG1 et IgG2a. (C) Détection de Pol Thêta dans la fraction initiale (FI) et après immunoprécipitation (IP) réalisée par les anticorps 18E1 ou 1B1 dans les cellules RKO, 24h après transfection par un siRNA contrôle ou dirigé contre *POLQ.*

### Résultats

L'immunoprécipitation réalisée à l'aide des anticorps 18E1, 10B2, 1B1 et 15G9 dans les cellules humains RKO, permet de retrouver la présence de la protéine Pol Thêta pleine taille dans l'immunoprécipitat (IP) révélé par western blot (Figure 6A), et ce de façon enrichi par rapport à la fraction de départ (FI, avant immunoprécipitation) (Figure 6B). Par ailleurs, l'utilisation d'immunoglobulines contrôles de type IgG1 et IgG2a ne permet pas l'immunoprécipitation de Pol Thêta (absence de signal), démontrant la spécificité des anticorps 18E1 et 1B1 pour Pol Thêta (Figure 6B). La spécificité des anticorps 18E1 et 1B1 a encore été renforcée par une expérience d'immunoprécipitation réalisée après extinction de l'expression de la protéine Pol Thêta par stransfection des cellules RKO par un siRNA contrôle (*CTRL*) ou dirigé contre *POLQ.* En effet, l'intensité du signal correspondant à Pol Thêta dans la fraction initiale (FI) et dans l'immunoprécipitat (IP) est très significativement diminuée après extinction de la protéine par transfection avec le siRNA POLQ par rapport au siRNA *CTRL* (Figure 6C).

### Matériel & Méthodes

20X10⁶ de cellules humaines RKO ont été lysées dans 1 ml de tampon d'IP (Tris 50mM pH8, NaCl 150mM, EGTA 3mM, NP40 1%, inhibiteurs de proteases et phosphatases) et conservées dans la glace pendant 30 min. Après centrifugation à 13 000rpm pendant 10 min à 4°C, le surnageant a été incubé avec 40µl de protéine A et G dynabeads (Invitrogen) pendant 1 h à 4°C sur roue. Après enlèvement des billes, 5µg d'anticorps 18E1, 15G9, 10B2 ou 1B1 ont été incubés avec les surnageants à 4°C sur roue pendant 2h30. 40µl de protéines A et G dynabeads (Invitrogen) ont été ensuite rajoutés pendant 2 heures supplémentaires. Après 3 lavages avec le tampon d'IP, le matériel immunoprecipité a été resuspendu dans 60µl de Laemmli, bouilli pendant 3 min and analysé en western blot. La révélation de Pol Thêta en western blot a été effectuée grâce à l'utilisation de l'anticorps 1B1 (0.1µg/ml) comme décrit précédemment.

### Exemple 8 : Détection avec anticorps 15G9 en IF via une méthode PLA (Proximity Ligation Assav) d'amplification des signaux

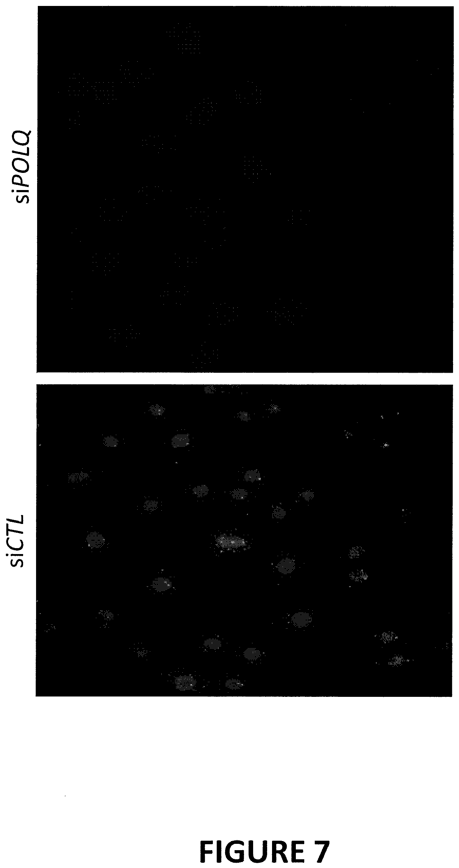

La Figure 7 illustre la détection en IF via une méthode PLA d'amplification des signaux dans le cas de cellules transfectées par un siRNA dirigé contre POLQ (si*POLQ*) ou un siRNA contrôle (si*CTL*),

### Résultats

Les cellules RKO ont été transfectées par un siRNA dirigé contre POLQ (si*POLQ*) ou un siRNA contrôle (si*CTL*), puis fixées 72h après à la PFA 4%. Un PLA (Proximity Ligation Assay) a été réalisé selon les instructions du fabricant (Duolink *in situ* PLA assay, Sigma Aldrich), avec des anticorps ciblant Pol Thêta (15G9) et ORC4 (ORC4-H300, sc-20634, Santa Cruz Biotechnology).

Dans la condition si*CTL*, le signal témoigne de la présence et de la proximité des 2 protéines, corroborant ainsi les résultats de co-immunoprécipitation publiés précédemment. Dans la condition si*POLQ*, le signal disparait, attestant de la spécificité du signal en condition contrôle.

### Matériel et Méthodes

Les cellules RKO ont été transfectées avec les siRNA à 50nM, en utilisant la lipofectamine 2000, selon les recommandations du fournisseur. 72h après transfection, les cellules ont été fixées au PBS, PFA4% pendant 15min, rincées 3 fois au PBS puis perméabilisées dans du PBS, triton 0.5% pendant 5min. Après 3 rinçages PBS, nous avons procédé au PLA selon le protocole fourni avec le kit. Les cellules ont été observées en microscopie à fluorescence à champs large.

### Exemple 9 : Co-immunoprécipiation Pol Theta avec Orc2 et Orc4.

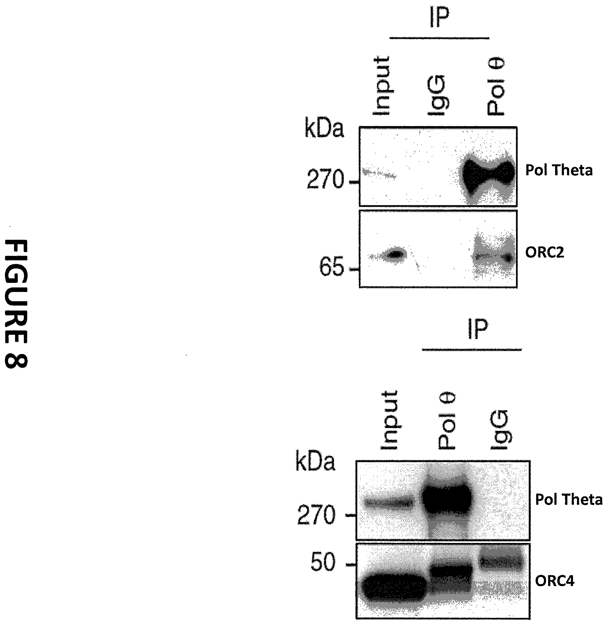

Figure 8 : illustre la co-immunoprécipitation de l'ADN Polymérase Pol Thêta (avec 18E1) avec Ocr2 et Ocr4.

### Résultats

Les extraits cellulaires obtenus sur des cellules RKO ont été immunoprécipités avec l'anticorps anti ADN Polymérase Pol Theta 18E1 ou une IgG contrôle, et analysés par Western Blot en utilisant les anticorps indiqués. La co-détection ADN Polymérase Thêta - ORC4 constitue un test pour valider l'évaluation des Anticorps en PLA. ORC4 a été choisi car il a déjà été montré que ces deux protéines co-immunoprécipitent. L'absence de signal dans les pistes IgG montre la spécificité de l'immunoprécipitation de Pol Theta par l'anticorps.

**Matériel et Méthodes** : similaires et correspondantes à ceux de l'Exemple 7.

## Revendications

1. Anticorps monoclonal **caractérisé en ce qu'**il est sélectionné dans le groupe comprenant les anticorps suivants :
i. anticorps capable de se lier à la séquence amino acide ayant la SEQ 1 du domaine central de l'ADN polymérase Pol Thêta,
ii. anticorps capable de se lier à la séquence amino acide ayant la SEQ 2 du domaine central de l'ADN polymérase Pol Thêta,
iii. anticorps capable de se lier à la séquence amino acide ayant la SEQ 4 du domaine central de l'ADN polymérase Pol Thêta.

2. Anticorps selon la revendication 1 **caractérisé en ce qu'**il est sélectionné dans le groupe comprenant les 4 anticorps suivants :
i. anticorps capable de se lier à la séquence amino acide ayant la SEQ 1 du domaine central l'ADN polymérase Pol Thêta **caractérisé en ce que** ledit anticorps comprend les 6 CDR comprenant les séquences amino acide respectives suivantes : SEQ ID 21, SEQ ID 22, SEQ ID 23, SEQ ID 24, SEQ ID 25, SEQ ID 26
ii. anticorps capable de se lier à la séquence amino acide ayant la SEQ 2 du domaine central l'ADN polymérase Pol Thêta **caractérisé en ce que** ledit anticorps comprend les 6 CDRs comprenant les séquences amino acide respectives suivantes : SEQ ID 5, SEQ ID 6, SEQ ID 7, SEQ ID 8, SEQ ID 9, SEQ ID 10.
iii. anticorps capable de se lier à la séquence amino acide ayant la SEQ 4 du domaine central l'ADN polymérase Pol Thêta **caractérisé en ce que** ledit anticorps comprend les 6 CDRs comprenant les séquences amino acide respectives suivantes : SEQ ID 13, SEQ ID 14, SEQ ID 15, SEQ ID 16, SEQ ID 17, SEQ ID 18
iv. anticorps capable de se lier à la séquence amino acide ayant la SEQ 4 du domaine central l'ADN polymérase Pol Thêta **caractérisé en ce que** ledit anticorps comprend les 6 CDR comprenant les séquences amino acide respectives suivantes : SEQ ID 29, SEQ ID 30, SEQ ID 31, SEQ ID 32, SEQ ID33, SEQ ID 34.

3. Anticorps selon la revendication 2, **caractérisé en ce que** :
i. L'anticorps capable de se lier à la séquence amino acide ayant la SEQ 1 du domaine central l'ADN polymérase Pol Thêta comprend :
i. Une chaîne lourde comprenant les 3 CDRs : CDR3H1, CDR3H2 et CDR3H3 ayant les séquences respectives suivantes SEQ ID 21, SEQ ID 22 et SEQ ID 23,
ii. Une chaîne légère comprenant les 3 CDRs : CDR3L1, CDR3L2 et CDR3L3 ayant les séquences respectives suivantes SEQ ID 24, SEQ ID 25 et SEQ ID 26.
ii. l'anticorps capable de se lier à la séquence amino acide ayant la SEQ 2 du domaine central l'ADN polymérase Pol Thêta comprend :
i. Une chaîne lourde comprenant les 3 CDRs : CDR1H1, CDR1H2 et CDR1H3 ayant les séquences respectives suivantes SEQ ID 5, SEQ ID 6 et SEQ ID 7,
ii. Une chaîne légère comprenant les 3 CDRs : CDR1L1, CDR1L2 et CDR1L3 ayant les séquences respectives suivantes SEQ ID 8., SEQ ID 9 et SEQ ID 10.
iii. l'anticorps capable de se lier à la séquence amino acide ayant la SEQ 4 du domaine central l'ADN polymérase Pol Thêta comprend :
i. Une chaîne lourde comprenant les 3 CDRs : CDR2H1, CDR2H2 et CDR2H3 ayant les séquences respectives suivantes SEQ ID 13, SEQ ID 14 et SEQ ID 15
ii. Une chaîne légère comprenant les 3 CDRs : CDR2L1, CDR2L2 et CDR2L3 ayant les séquences respectives suivantes SEQ ID 16, SEQ ID 17 et SEQ ID 18.
iv. l'anticorps capable de se lier à la séquence amino acide ayant la SEQ 4 du domaine central l'ADN polymérase Pol Thêta comprend :
i. une chaîne lourde comprenant les 3 CDRs : CDR4H1, CDR4H2 et CDR4H3 ayant les séquences respectives suivantes SEQ ID 29, SEQ ID 30 et SEQ ID 31,
ii. une chaîne légère comprenant les 3 CDRs : CDR4L1, CDR4L2 et CDR4L3 ayant les séquences respectives suivantes SEQ ID 32, SEQ ID 33 et SEQ ID 34.

4. Anticorps selon la revendication 2 **caractérisé en ce que** :
i. l'anticorps capable de se lier à la fraction peptidique ayant la SEQ 1 du domaine central de l'ADN polymérase Pol Thêta comprenant :
i. une chaîne lourde comprenant les 3 CDRs : CDR3H1, CDR3H2 et CDR3H3 ayant les séquences respectives suivantes SEQ ID 21, SEQ ID 22 et SEQ ID 23,
ii. un domaine variable de chaîne légère comprenant la SEQ ID 28
ii. l'anticorps capable de se lier à la fraction peptidique ayant la SEQ 2 du domaine central l'ADN polymérase Pol Thêta comprend :
i. une chaîne lourde comprenant les 3 CDRs : CDR1H1, CDR1H2 et CDR1H3 ayant les séquences respectives suivantes SEQ ID 5, SEQ ID 6 et SEQ ID 7,
ii. un domaine variable de chaîne légère comprenant la SEQ ID 12.
iii. l'anticorps capable de se lier à la fraction peptidique ayant la SEQ 4 du domaine central l'ADN polymérase Pol Thêta comprenant :
i. une chaîne lourde comprenant les 3 CDRs : CDR2H1, CDR2H2 et CDR2H3 ayant les séquences respectives suivantes SEQ ID 13, SEQ ID 14 et SEQ ID 15,
ii. un domaine variable de chaîne légère comprenant la SEQ ID 20.
iv. l'anticorps capable de se lier à la fraction peptidique ayant la SEQ 4 du domaine central l'ADN polymérase Pol Thêta comprenant :
i. une chaîne lourde comprenant les 3 CDRs : CDR4H1, CDR4H2 et CDR4H3 ayant les séquences respectives suivantes SEQ ID 29 SEQ ID 30 et SEQ ID 31,
ii. un domaine variable de chaîne légère comprenant la SEQ ID36.

5. Anticorps selon la revendication 2 **caractérisé en ce que**:
i. l'anticorps capable de se lier à la fraction peptidique ayant la SEQ 1 du domaine central de l'ADN polymérase Pol Thêta comprend :
i. une chaîne légère comprenant les 3 CDRs : CDR3L1, CDR3L2 et CDR3L3 ayant les séquences respectives suivantes SEQ ID 24, SEQ ID 25 et SEQ ID 26,
ii. un domaine variable de chaîne lourde comprenant la SEQ ID 27,
ii. l'anticorps capable de se lier à la fraction peptidique ayant la SEQ 2 du domaine central l'ADN polymérase Pol Thêta comprend :
i. une chaîne légère comprenant les 3 CDRs : CDR1L1, CDR1L2 et CDR1L3 ayant les séquences respectives suivantes SEQ ID 8, SEQ ID 9 et SEQ ID 10,
ii. Un domaine variable de chaîne lourde comprenant la SEQ ID 11,
iii. l'anticorps capable de se lier à la fraction peptidique ayant la SEQ 4 du domaine central l'ADN polymérase Pol Thêta comprend :
i. une chaîne légère comprenant les 3 CDRs : CDR2L1, CDR2L2 et CDR2L3 ayant les séquences respectives suivantes SEQ ID 16, SEQ ID 17 et SEQ ID 18
ii. un domaine variable de chaîne lourde comprenant la SEQ ID19,
iv. l'anticorps capable de se lier à la fraction peptidique ayant la SEQ 4 du domaine central l'ADN polymérase Pol Thêta comprend :
i. une chaîne légère comprenant les 3 CDRs : CDR4L1, CDR4L2 et CDR4L3 ayant les séquences respectives suivantes SEQ ID 32, SEQ ID 33 et SEQ ID 34,
ii. un domaine variable de chaîne lourde comprenant la SEQ ID35.

6. Anticorps selon la revendication 3 **caractérisé en ce que**:
i. l'anticorps capable de se lier à la fraction peptidique ayant la SEQ 1 du domaine central de l'ADN polymérase Pol Thêta comprend :
i. un domaine variable de la chaîne lourde comprenant la SEQ ID27,
ii. un domaine variable de chaîne légère comprenant la SEQ ID 28,
ii. l'anticorps capable de se lier à la fraction peptidique ayant la SEQ 2 du domaine central l'ADN polymérase Pol Thêta comprend :
i. un domaine variable de la chaîne lourde comprenant la SEQ ID11
ii. un domaine variable de la chaîne légère comprenant la SEQ ID12
iii. l'anticorps capable de se lier à la fraction peptidique ayant la SEQ 4 du domaine central l'ADN polymérase Pol Thêta comprend :
i. un domaine variable de la chaîne lourde comprenant la SEQ ID19
ii. un domaine variable de la chaîne légère comprenant la SEQ ID20
iv. L'anticorps capable de se lier à la fraction peptidique ayant la SEQ 4 du domaine central l'ADN polymérase thêta comprend :
i. un domaine variable de la chaîne lourde comprenant la SEQ ID35
ii. un domaine variable de la chaîne légère comprenant la SEQ ID36

7. Anticorps 1B1 selon la revendication 1 comprenant :
i. une chaîne lourde comprenant la séquence SEQ ID 39
ii. une chaîne légère comprenant la séquence SEQ ID 40

8. Anticorps 18E1 selon la revendication 1 comprenant :
i. une chaîne lourde comprenant SEQ ID 41
ii. une chaîne légère comprenant SEQ ID 42

9. Anticorps 10B2 selon la revendication 1 comprenant :
i. une chaîne lourde comprenant SEQ ID 37
ii. une chaîne légère comprenant SEQ ID 38

10. Anticorps 15G9 selon la revendication 1 comprenant :
i. une chaîne lourde comprenant SEQ ID 43
ii. une chaîne légère comprenant SEQ ID 44

11. Méthode pour déterminer *in vitro* ou *ex vivo* le niveau d'expression de l'ADN polymérase Pol Thêta chez un sujet comprenant les étapes suivantes :
i. mettre en contact un échantillon biologique obtenu du sujet avec un anticorps selon l'une des revendications 1 à 10
ii. quantifier le niveau d'expression de l'ADN polymérase Pol Thêta au sein de l'échantillon.

12. Kit comprenant au moins un anticorps selon l'une des revendications 1 à 10.

13. Kit selon la revendication 12 **caractérisé en ce qu'**il comprend en outre un réactif pour détecter la liaison de l'anticorps avec l'ADN polymérase Pol Thêta .

14. Kit selon l'une des revendications 12 ou 13 **caractérisé en ce qu'**il comprend un réactif pour quantifier le niveau de liaison de l'anticorps avec l'ADN polymérase Pol Thêta.

## Patentansprüche

1. Monoklonaler Antikörper, **dadurch gekennzeichnet, dass** er ausgewählt ist aus der Gruppe, umfassend die folgenden Antikörper:
i. Antikörper, der imstande ist, sich mit der Aminosäuresequenz mit der SEQ 1 der zentralen Domäne der DNA-Polymerase Pol-Theta zu binden,
ii. Antikörper, der imstande ist, sich mit der Aminosäuresequenz mit der SEQ 2 der zentralen Domäne der DNA-Polymerase Pol-Theta zu binden,
iii. Antikörper, der imstande ist, sich mit der Aminosäuresequenz mit der SEQ4 der zentralen Domäne der DNA-Polymerase Pol-Theta zu binden.

2. Antikörper nach Anspruch 1, **dadurch gekennzeichnet, dass** er ausgewählt ist aus der Gruppe, umfassend die folgenden 4 Antikörper:
i. Antikörper, der imstande ist, sich mit der Aminosäuresequenz mit der SEQ 1 der zentralen Domäne der DNA-Polymerase Pol-Theta zu binden, **dadurch gekennzeichnet, dass** der Antikörper die 6 CDRs umfasst, umfassend die folgenden jeweiligen Aminosäuresequenzen: SEQ ID 21, SEQ ID 22, SEQ ID 23, SEQ ID 24, SEQ ID 25, SEQ ID 26
ii. Antikörper, der imstande ist, sich mit der Aminosäuresequenz mit der SEQ 2 der zentralen Domäne der DNA-Polymerase Pol-Theta zu binden, **dadurch gekennzeichnet, dass** der Antikörper die 6 CDRs umfasst, umfassend die folgenden jeweiligen Aminosäuresequenzen: SEQ ID 5, SEQ ID 6, SEQ ID 7, SEQ ID 8, SEQ ID 9, SEQ ID 10.
iii. Antikörper, der imstande ist, sich mit der Aminosäuresequenz mit der SEQ4 der zentralen Domäne der DNA-Polymerase Pol-Theta zu binden, **dadurch gekennzeichnet, dass** der Antikörper die 6 CDRs umfasst, umfassend die folgenden jeweiligen Aminosäuresequenzen: SEQ ID 13, SEQ ID 14, SEQ ID 15, SEQ ID 16, SEQ ID 17, SEQ ID 18
iv. Antikörper, der imstande ist, sich mit der Aminosäuresequenz mit der SEQ4 der zentralen Domäne der DNA-Polymerase Pol-Theta zu binden, **dadurch gekennzeichnet, dass** der Antikörper die 6 CDRs umfasst, umfassend die folgenden jeweiligen Aminosäuresequenzen: SEQ ID 29, SEQ ID 30, SEQ ID 31, SEQ ID 32, SEQ ID 33, SEQ ID 34.

3. Antikörper nach Anspruch 2, **dadurch gekennzeichnet, dass**:
i. der Antikörper, der imstande ist, sich mit der Aminosäuresequenz mit der SEQ 1 der zentralen Domäne der DNA-Polymerase Pol-Theta zu binden, umfasst:
i. eine schwere Kette, umfassend die 3 CDRs: CDR3H1, CDR3H2 und CDR3H3 mit den folgenden jeweiligen Sequenzen SEQ ID 21, SEQ ID 22 und SEQ ID 23.
ii. eine leichte Kette, umfassend die 3 CDRs: CDR3L1, CDR3L2 und CDR3L3 mit den folgenden jeweiligen Sequenzen SEQ ID 24, SEQ ID 25 und SEQ ID 26.
ii. der Antikörper, der imstande ist, sich mit der Aminosäuresequenz mit der SEQ 2 der zentralen Domäne der DNA-Polymerase Pol-Theta zu binden, umfasst:
i. eine schwere Kette, umfassend die 3 CDRs: CDR1H1, CDR1H2 und CDR1H3 mit den folgenden jeweiligen Sequenzen SEQ ID 5, SEQ ID 6 und SEQ ID 7,
ii. eine leichte Kette, umfassend die 3 CDRs: CDR1L1, CDR1L2 und CDR1L3 mit den folgenden jeweiligen Sequenzen SEQ ID 8, SEQ ID 9 und SEQ ID 10.
iii. der Antikörper, der imstande ist, sich mit der Aminosäuresequenz mit der SEQ 4 der zentralen Domäne der DNA-Polymerase Pol-Theta zu binden, umfasst:
i. eine schwere Kette, umfassend die 3 CDRs: CDR2H1, CDR2H2 und CDR2H3 mit den folgenden jeweiligen Sequenzen SEQ ID 13, SEQ ID 14 und SEQ ID 15
ii. eine leichte Kette, umfassend die 3 CDRs: CDR2L1, CDR2L2 und CDR2L3 mit den folgenden jeweiligen Sequenzen SEQ ID 16, SEQ ID 17 und SEQ ID 18.
iv. der Antikörper, der imstande ist, sich mit der Aminosäuresequenz mit der SEQ 4 der zentralen Domäne der DNA-Polymerase Pol-Theta zu binden, umfasst:
i. eine schwere Kette, umfassend die 3 CDRs: CDR4H1, CDR4H2 und CDR4H3 mit den folgenden jeweiligen Sequenzen SEQ ID 29, SEQ ID 30 und SEQ ID 31,
ii. eine leichte Kette, umfassend die 3 CDRs: CDR4L1, CDR4L2 und CDR4L3 mit den folgenden jeweiligen Sequenzen SEQ ID 32, SEQ ID 33 und SEQ ID 34.

4. Antikörper nach Anspruch 2, **dadurch gekennzeichnet, dass**:
i. der Antikörper, der imstande ist, sich mit der Peptidfraktion mit der SEQ 1 der zentralen Domäne der DNA-Polymerase Pol-Theta zu binden, umfassend:
i. eine schwere Kette, umfassend die 3 CDRs: CDR3H1, CDR3H2 und CDR3H3 mit den folgenden jeweiligen Sequenzen SEQ ID 21, SEQ ID 22 und SEQ ID 23,
ii. eine variable Domäne einer leichten Kette, umfassend die SEQ ID 28
ii. der Antikörper, der imstande ist, sich mit der Peptidfraktion mit der SEQ 2 der zentralen Domäne der DNA-Polymerase Pol-Theta zu binden, umfasst:
i. eine schwere Kette, umfassend die 3 CDRs: CDR1H1, CDR1H2 und CDR1H3 mit den folgenden jeweiligen Sequenzen SEQ ID 5, SEQ ID 6 und SEQ ID 7,
ii. eine variable Domäne einer leichten Kette, umfassend die SEQ ID 12.
iii. der Antikörper, der imstande ist, sich mit der Peptidfraktion mit der SEQ 4 der zentralen Domäne der DNA-Polymerase Pol-Theta zu binden, umfassend:
i. eine schwere Kette, umfassend die 3 CDRs: CDR2H1, CDR2H2 und CDR2H3 mit den folgenden jeweiligen Sequenzen SEQ ID 13, SEQ ID 14 und SEQ ID 15,
ii. eine variable Domäne einer leichten Kette, umfassend die SEQ ID 20.
iv. der Antikörper, der imstande ist, sich mit der Peptidfraktion mit der SEQ 4 der zentralen Domäne der DNA-Polymerase Pol-Theta zu binden, umfassend:
i. eine schwere Kette, umfassend die 3 CDRs: CDR4H1, CDR4H2 und CDR4H3 mit den folgenden jeweiligen Sequenzen SEQ ID 29, SEQ ID 30 und SEQ ID 31,
ii. eine variable Domäne einer leichten Kette, umfassend die SEQ ID 36.

5. Antikörper nach Anspruch 2, **dadurch gekennzeichnet, dass**:
i. der Antikörper, der imstande ist, sich mit der Peptidfraktion mit der SEQ 1 der zentralen Domäne der DNA-Polymerase Pol-Theta zu binden, umfasst:
i. eine leichte Kette, umfassend die 3 CDRs: CDR3L1, CDR3L2 und CDR3L3 mit den folgenden jeweiligen Sequenzen SEQ ID 24, SEQ ID 25 und SEQ ID 26,
ii. eine variable Domäne einer schweren Kette, umfassend die SEQ ID 27,
ii. der Antikörper, der imstande ist, sich mit der Peptidfraktion mit der SEQ 2 der zentralen Domäne der DNA-Polymerase Pol-Theta zu binden, umfasst:
i. eine leichte Kette, umfassend die 3 CDRs: CDR1L1, CDR1L2 und CDR1L3 mit den folgenden jeweiligen Sequenzen SEQ ID 8, SEQ ID 9 und SEQ ID 10,
ii. eine variable Domäne einer schweren Kette, umfassend die SEQ ID 11,
iii. der Antikörper, der imstande ist, sich mit der Peptidfraktion mit der SEQ 4 der zentralen Domäne der DNA-Polymerase Pol-Theta zu binden, umfasst:
i. eine leichte Kette, umfassend die 3 CDRs: CDR2L1, CDR2L2 und CDR2L3 mit den folgenden jeweiligen Sequenzen SEQ ID 16, SEQ ID 17 und SEQ ID 18
ii. eine variable Domäne einer schweren Kette, umfassend die SEQ ID 19,
iv. der Antikörper, der imstande ist, sich mit der Peptidfraktion mit der SEQ 4 der zentralen Domäne der DNA-Polymerase Pol-Theta zu binden, umfasst:
i. eine leichte Kette, umfassend die 3 CDRs: CDR4L1, CDR4L2 und CDR4L3 mit den folgenden jeweiligen Sequenzen SEQ ID 32, SEQ ID 33 und SEQ ID 34,
ii. eine variable Domäne einer schweren Kette, umfassend die SEQ ID 35.

6. Antikörper nach Anspruch 3, **dadurch gekennzeichnet, dass**:
i. der Antikörper, der imstande ist, sich mit der Peptidfraktion mit der SEQ 1 der zentralen Domäne der DNA-Polymerase Pol-Theta zu binden, umfasst:
i. eine variable Domäne der schweren Kette, umfassend die SEQ ID 27,
ii. eine variable Domäne einer leichten Kette, umfassend die SEQ ID 28,
ii. der Antikörper, der imstande ist, sich mit der Peptidfraktion mit der SEQ 2 der zentralen Domäne der DNA-Polymerase Pol-Theta zu binden, umfasst:
i. eine variable Domäne der schwereren Kette, umfassend die SEQ ID 11,
ii. eine variable Domäne der leichten Kette, umfassend die SEQ ID 12,
iii. der Antikörper, der imstande ist, sich mit der Peptidfraktion mit der SEQ 4 der zentralen Domäne der DNA-Polymerase Pol-Theta zu binden, umfasst:
i. eine variable Domäne der schweren Kette, umfassend die SEQ ID 19
ii. eine variable Domäne der leichten Kette, umfassend die SEQ ID 20
iv. der Antikörper, der imstande ist, sich mit der Peptidfraktion mit der SEQ 4 der zentralen Domäne der DNA-Polymerase Pol-Theta zu binden, umfasst:
i. eine variable Domäne der schweren Ketten, umfassend die SEQ ID 35,
ii. eine variable Domäne der leichten Kette, umfassend die SEQ ID 36.

7. Antikörper 1B1 nach Anspruch 1, umfassend:
i. eine schwere Kette, umfassend die Sequenz SEQ ID 39
ii. eine leichte Kette, umfassend die Sequenz SEQ ID 40

8. Antikörper 18E1 nach Anspruch 1, umfassend:
i. eine schwere Kette, umfassend die Sequenz SEQ ID 41
ii. eine leichte Kette, umfassend die Sequenz SEQ ID 42

9. Antikörper 10B2 nach Anspruch 1, umfassend:
i. eine schwere Kette, umfassend die Sequenz SEQ ID 37
ii. eine leichte Kette, umfassend die Sequenz SEQ ID 38

10. Antikörper 15G9 nach Anspruch 1, umfassend:
i. eine schwere Kette, umfassend die Sequenz SEQ ID 43
ii. eine leichte Kette, umfassend die Sequenz SEQ ID 44

11. Verfahren zum *in-vitro-* oder *ex-vivo*-Bestimmen des Expressionsniveaus der DNA-Polymerase Pol-Theta bei einem Subjekt, umfassend die folgenden Schritte:
i. Inkontaktbringen einer vom Subjekt erhaltenen biologischen Probe mit einem Antikörper nach einem der Ansprüche 1 bis 10
ii. Quantifizieren des Expressionsniveaus der DNA-Polymerase Pol-Theta innerhalb der Probe.

12. Kit, umfassend mindestens einen Antikörper nach einem der Ansprüche 1 bis 10.

13. Kit nach Anspruch 12, **dadurch gekennzeichnet, dass** es weiter ein Reagens umfasst, um die Bindung des Antikörpers mit der DNA-Polymerase Pol-Theta zu erfassen.

14. Kit nach einem der Ansprüche 12 oder 13, **dadurch gekennzeichnet, dass** es ein Reagens umfasst, um das Bindungsniveau des Antikörpers mit der DNA-Polymerase Pol-Theta zu quantifizieren.

## Claims

1. Monoclonal antibody **characterised in that** it is selected from the group comprising the following antibodies:
i. antibody capable of binding with the amino acid sequence having SEQ 1 of the central domain of DNA polymerase Pol Theta
ii. antibody capable of binding with the amino acid sequence having SEQ 2 of the central domain of DNA polymerase Pol Theta
iii. antibody capable of binding with the amino acid sequence having SEQ 4 of the central domain of DNA polymerase Pol Theta

2. Antibody according to claim **characterised in that** is selected from the group comprising the 4 following antibodies:
i. antibody capable of binding with the amino acid sequence having SEQ 1 of the central domain of DNA polymerase Pol Theta **characterised in that** said antibody comprises the 6 CDRs comprising the following respective amino acid sequences: SEQ ID 21, SEQ ID 22, SEQ ID 23, SEQ ID 24, SEQ ID 25, SEQ ID 26
ii. antibody capable of binding with the amino acid sequence having SEQ 2 of the central domain of DNA polymerase Pol Theta **characterised in that** said antibody comprises the 6 CDRs comprising the following respective amino acid sequences: SEQ ID 5, SEQ ID 6, SEQ ID 7, SEQ I D 8, SEQ ID 9, SEQ ID 10.
iii. antibody capable of binding with the amino acid sequence having SEQ 4 of the central domain of DNA polymerase Pol Theta **characterised in that** said antibody comprises the 6 CDRs comprising the following respective amino acid sequences: SEQ ID 13, SEQ ID 14, SEQ ID 15, SEQ ID 16, SEQ ID 17, SEQ ID 18
iv. antibody capable of binding with the amino acid sequence having SEQ 4 of the central domain of DNA polymerase Pol Theta **characterised in that** said antibody comprises the 6 CDRs comprising the following respective amino acid sequences: SEQ ID 29, SEQ ID 30, SEQ ID 31, SEQ ID 32, SEQ ID33, SEQ ID 34.

3. Antibody according to claim 2, **characterised in that**:
i. The antibody capable of binding to amino acid sequence having SEQ 1 of the central domain of DNA polymerase Pol Theta comprises:
i. A heavy chain comprising the 3 CDRs: CDR3H1, CDR3H2 and CDR3H3 having the following respective sequences SEQ ID 21, SEQ ID 22 and SEQ ID 23,
ii. A light chain comprising the 3 CDRs: CDR3L1, CDR3L2 and CDR3L3 having the following respective sequences SEQ ID 24, SEQ ID 25 and SEQ ID 26,
ii. The antibody capable of binding to amino acid sequence having SEQ 2 of the central domain of DNA polymerase Pol Theta comprises:
i. A heavy chain comprising the 3 CDRs: CDR1H1, CDR1H2 and CDR1H3 having the following respective sequences SEQ ID 5, SEQ ID 6 and SEQ ID 7,
ii. A light chain comprising the 3 CDRs: CDR1L1, CDR1L2 and CDR1L3 having the following respective sequences SEQ ID 8, SEQ ID 9 and SEQ ID 10,
iii. The antibody capable of binding to amino acid sequence having SEQ 4 of the central domain of DNA polymerase Pol Theta comprises:
i. A heavy chain comprising the 3 CDRs: CDR2H1, CDR2H2 and CDR2H3 having the following respective sequences SEQ ID 13, SEQ ID 14 and SEQ ID 15,
ii. A light chain comprising the 3 CDRs: CDR2L1, CDR2L2 and CDR2L3 having the following respective sequences SEQ ID 16, SEQ ID 17 and SEQ ID 18,
iv. The antibody capable of binding to amino acid sequence having SEQ 4 of the central domain of DNA polymerase Pol Theta comprises:
i. A heavy chain comprising the 3 CDRs: CDR4H1, CDR4H2 and CDR4H3 having the following respective sequences SEQ ID 29, SEQ ID 30 and SEQ ID 31,
ii. A light chain comprising the 3 CDRs: CD42L1, CDR4L2 and CDR4L3 having the following respective sequences SEQ ID 32, SEQ ID 33 and SEQ ID 34,

4. Antibody according to claim 2, **characterised in that**:
i. The antibody capable of binding to the peptide fraction having SEQ 1 of the central domain of DNA polymerase Pol Theta comprises:
i. A heavy chain comprising the 3 CDRs: CDR3H1, CDR3H2 and CDR3H3 having the following respective sequences SEQ ID 21, SEQ ID 22 and SEQ ID 23,
ii. A variable light chain domain comprising SEQ ID 28.
ii. The antibody capable of binding to the peptide fraction having SEQ 2 of the central domain of DNA polymerase Pol Theta comprises:
i. A heavy chain comprising the 3 CDRs: CDR1H1, CDR1H2 and CDR1H3 having the following respective sequences SEQ ID 5, SEQ ID 6 and SEQ ID 7,
ii. A variable light chain domain comprising SEQ ID 12.
iii. The antibody capable of binding to the peptide fraction having SEQ 4 of the central domain of DNA polymerase Pol Theta comprises:
i. A heavy chain comprising the 3 CDRs: CDR2H1, CDR2H2 and CDR2H3 having the following respective sequences SEQ ID 13, SEQ ID 14 and SEQ ID 15,
ii. A variable light chain domain comprising SEQ ID 20.
iv. The antibody capable of binding to the peptide fraction having SEQ 4 of the central domain of DNA polymerase Pol Theta comprises:
i. A heavy chain comprising the 3 CDRs: CDR4H1, CDR4H2 and CDR4H3 having the following respective sequences SEQ ID 29, SEQ ID 30 and SEQ ID 31,
ii. A variable light chain domain comprising SEQ ID 36.

5. Antibody according to claim 2, **characterised in that**:
i. The antibody capable of binding to the peptide fraction having SEQ 1 of the central domain of DNA polymerase Pol Theta comprises:
i. A light chain comprising the 3 CDRs: CDR3L1, CDR3L2 and CDR3L3 having the following respective sequences SEQ ID 24, SEQ ID 25 and SEQ ID 26,
ii. A variable heavy chain domain comprising SEQ ID 27.
ii. The antibody capable of binding to the peptide fraction having SEQ 2 of the central domain of DNA polymerase Pol Theta comprises:
i. A light chain comprising the 3 CDRs: CDR1L1, CDR1L2 and CDR1L3 having the following respective sequences SEQ ID 8, SEQ ID 9 and SEQ ID 10,
ii. A variable heavy chain domain comprising SEQ ID 11.
iii. The antibody capable of binding to the peptide fraction having SEQ 4 of the central domain of DNA polymerase Pol Theta comprises:
i. A light chain comprising the 3 CDRs: CDR2L1, CDR2L2 and CDR2L3 having the following respective sequences SEQ ID 16, SEQ ID 17 and SEQ ID 18,
ii. A variable heavy chain domain comprising SEQ ID 19.
iv. The antibody capable of binding to the peptide fraction having SEQ 4 of the central domain of DNA polymerase Pol Theta comprises:
i. A light chain comprising the 3 CDRs: CDR4L1, CDR4L2 and CDR4L3 having the following respective sequences SEQ ID 32, SEQ ID 33 and SEQ ID 34,
ii. A variable heavy chain domain comprising SEQ ID 35.

6. Antibody according to claim 3, **characterised in that**:
i. The antibody capable of binding to the peptide fraction having SEQ 1 of the central domain of DNA polymerase Pol Theta comprises:
i. A variable heavy chain domain comprising SEQ ID 27,
ii. A variable light chain domain comprising SEQ ID 28.
ii. The antibody capable of binding to the peptide fraction having SEQ 2 of the central domain of DNA polymerase Pol Theta comprises:
i. A variable heavy chain domain comprising SEQ ID 11,
ii. A variable light chain domain comprising SEQ ID 12.
iii. The antibody capable of binding to the peptide fraction having SEQ 4 of the central domain of DNA polymerase Pol Theta comprises:
i. A variable heavy chain domain comprising SEQ ID 19,
ii. A variable light chain domain comprising SEQ ID 20.
iv. The antibody capable of binding to the peptide fraction having SEQ 4 of the central domain of DNA polymerase Pol Theta comprises:
i. A variable heavy chain domain comprising SEQ ID 35,
ii. A variable light chain domain comprising SEQ ID 36.

7. Antibody 1B1 according to claim 1, comprising:
i. a heavy chain comprising the sequence SEQ ID 39
ii. a light chain comprising the sequence SEQ ID 40

8. Antibody 18E1 according to claim 1, comprising:
i. a heavy chain comprising SEQ ID 41
ii. a light chain comprising SEQ ID 42

9. Antibody 10B2 according to claim 1, comprising:
i. a heavy chain comprising SEQ ID 37
ii. a light chain comprising SEQ ID 38

10. Antibody 15G9 according to claim 1, comprising:
i. a heavy chain comprising SEQ ID 43
ii. a light chain comprising SEQ ID 44

11. Method for determining *in vitro* or *ex vivo* the level of expression of the DNA polymerase Pol Theta in a subject comprising the following steps:
i. placing a biological sample obtained from the subject in contact with an antibody according to one of the claims 1 to 10
ii. quantifying the level of expression of the DNA polymerase Pol Theta in the sample

12. Kit comprising at least one antibody according to one of the claims 1 to 10.

13. Kit according to claim 12 **characterised in that** it further comprises a reagent to detect the bond of the antibody with the DNA polymerase Pol Theta.

14. Kit according to one of the claims 12 or 13 **characterised in that** it comprises a reagent to quantify the level of the bond of the antibody with the DNA polymerase Pol Theta.


### SEQUENCE LISTING

<110> CENTRE NATIONAL DE LA RECHERCHE SCIENTIFIQUE (CNRS) INSTITUT NATIONAL DE LA SANTE ET DE LA RECHERCHE MEDICALE (INSERM) UNIVERSITE PAUL SABATIER TOULOUSE III
<120> NOUVEAUX ANTICORPS UTILES POUR LE DIAGNOSTIC ET LE PRONOSTIC DU CANCER
<130> 367600D32932
<150> FR1453333
   <151> 2014-04-14
<160> 76
<170> PatentIn version 3.5
<210> 1
   <211> 20
   <212> PRT
   <213> artificial
<220>
   <223> Peptide 1 utilisé pour l'immunisation
<400> 1
   Cys Lys His Ser Pro Asn Ile Val Gln Asp Leu Asn Lys Ser Arg Glu
   1               5                   10                  15
   His Thr Ser Ser
               20
<210> 2
   <211> 20
   <212> PRT
   <213> artificial
<220>
   <223> Peptide 2 utilisé pour l'immunisation
<400> 2
   Cys Ser Ile Phe Arg Ala Arg Lys Arg Ala Ser Leu Asp Ile Asn Lys
   1               5                   10                  15
   Glu Lys Pro Gly
               20
<210> 3
   <211> 19
   <212> PRT
   <213> artificial
<220>
   <223> Peptide 3 utilisé pour l'immunisation
<400> 3
   Cys Leu Gln Glu Asp Leu Ile Lys Lys Ser Asn Val Asn Glu Asn Gln
   1               5                   10                  15
   Asp Thr His
<210> 4
   <211> 21
   <212> PRT
   <213> artificial
<220>
   <223> Peptide 4 utilisé pour l'immunisation
<400> 4
   His Asp Glu Thr Ser Ser Leu Leu Pro Arg Lys Glu Ser Asn Ile Val
   1               5                   10                  15
   Asp Asp Asn Gly Cys
               20
<210> 5
   <211> 8
   <212> PRT
   <213> artificial
<220>
   <223> Anticorps 1B1 - CDR1H1
<400> 5
   Gly Tyr Thr Phe Thr Asn Tyr Trp
   1               5
<210> 6
   <211> 8
   <212> PRT
   <213> artificial
<220>
   <223> Anticorps 1B1 - CDR1H2
<400> 6
   Ile Asp Pro Ser Asp Arg Tyr Thr
   1               5
<210> 7
   <211> 7
   <212> PRT
   <213> artificial
<220>
   <223> Anticorps 1B1 - CDR1H3
<400> 7
   Thr Met Tyr Ser Phe Ala Tyr
   1 5
<210> 8
   <211> 11
   <212> PRT
   <213> artificial
<220>
   <223> Anticorps 1B1 - CDR1L1
<400> 8
   Gln Ser Leu Leu Asp Ser Asp Gly Lys Thr Tyr
   1 5 10
<210> 9
   <211> 3
   <212> PRT
   <213> artificial
<220>
   <223> Anticorps 1B1 - CDR1L2
<400> 9
   Leu Val Ser
   1
<210> 10
   <211> 9
   <212> PRT
   <213> artificial
<220>
   <223> Anticorps 1B1 - CDR1L3
<400> 10
   Val Gln Gly Ala His Leu Pro Gln Thr
   1 5
<210> 11
   <211> 114
   <212> PRT
   <213> artificial
<220>
   <223> Anticorps 1B1 - Domaine variable chaine lourde
<400> 11
   Gln Val Gln Leu Gln Gln Pro Gly Ala Asp Leu Val Lys Pro Gly Ala
   1               5                   10                  15
   Ser Val Lys Leu Ser Cys Lys Thr Ser Gly Tyr Thr Phe Thr Asn Tyr
               20                  25                  30
   Trp Ile Gln Trp Ile Lys Gln Lys Pro Gly Gln Gly Leu Glu Trp Ile
           35                  40                  45
   Gly Glu Ile Asp Pro Ser Asp Arg Tyr Thr Asn Tyr Asn Gln Arg Phe
       50                  55                  60
   Lys Gly Lys Ala Thr Leu Thr Val Asp Thr Ser Ser Ser Thr Ala Tyr
   65                  70                  75                  80
   Met Gln Leu Asn Ser Leu Thr Ser Glu Asp Ser Ala Val Tyr Tyr Cys
                   85                  90                  95
   Thr Met Tyr Ser Phe Ala Tyr Trp Gly Gln Gly Thr Leu Val Thr Val
               100                 105                 110
   Ser Ala
<210> 12
   <211> 113
   <212> PRT
   <213> artificial
<220>
   <223> Anticorps 1B1 - Domaine variable chaine légère
<400> 12
   Asp Val Val Met Thr Gln Thr Pro Leu Thr Leu Ser Val Thr Ile Gly
   1               5                   10                  15
   Gln Pro Ala Ser Ile Ser Cys Lys Ser Ser Gln Ser Leu Leu Asp Ser
               20                  25                  30
   Asp Gly Lys Thr Tyr Leu Asn Trp Leu Leu Gln Ser Pro Gly Gln Ser
           35                  40                  45
   Pro Lys Leu Leu Ile Tyr Leu Val Ser Lys Leu Glu Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Val Gln Gly
                   85                  90                  95
   Ala His Leu Pro Gln Thr Phe Gly Ser Gly Thr Lys Leu Glu Ile Lys
                   100                 105                 110
   Arg
<210> 13
   <211> 8
   <212> PRT
   <213> artificial
<220>
   <223> Anticorps 18E1 - CDR2H1
<400> 13
   Gly Asp Thr Phe Thr Asp Tyr Pro
   1               5
<210> 14
   <211> 8
   <212> PRT
   <213> artificial
<220>
   <223> Anticorps 18E1 - CDR2H2
<400> 14
   Ile Asn Ala Glu Thr Ala Glu Pro
   1               5
<210> 15
   <211> 6
   <212> PRT
   <213> artificial
<220>
   <223> Anticorps 18E1 - CDR2H3
<400> 15
   Ala Ser Ser Tyr Gly Tyr
   1               5
<210> 16
   <211> 11
   <212> PRT
   <213> artificial
<220>
   <223> Anticorps 18E1 - CDR2L1
<400> 16
   Gln Ser Leu Leu Tyr Ser Asp Gly Lys Thr Tyr
   1               5                   10
<210> 17
   <211> 3
   <212> PRT
   <213> artificial
<220>
   <223> Anticorps 18E1 - CDR2L2
<400> 17
   Leu Val Ser
   1
<210> 18
   <211> 9
   <212> PRT
   <213> artificial
<220>
   <223> Anticorps 18E1 - CDR2L3
<400> 18
   Val Gln Gly Ser His Phe Pro His Thr
   1               5
<210> 19
   <211> 113
   <212> PRT
   <213> artificial
<220>
   <223> Anticorps 18E1 - Domaine variable chaine lourde
<400> 19
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Asp Thr Phe Thr Asp Tyr
               20                  25                  30
   Pro Met His Trp Val Lys Gln Ala Pro Gly Lys Gly Leu Lys Trp Met
           35                  40                  45
   Gly Trp Ile Asn Ala Glu Thr Ala Glu Pro Thr Tyr Val Asp Asp Phe
       50                  55                  60
   Lys Gly Arg Phe Ala Phe Ser Leu Glu Ser Ser Ala Ser Thr Ala Tyr
   65                  70                  75                  80
   Leu Gln Ile Asn Asn Leu Lys Asn Glu Asp Thr Ala Thr Tyr Phe Cys
                   85                  90                  95
   Ala Ser Ser Tyr Gly Tyr Trp Gly Gln Gly Thr Leu Val Thr Val Ser
                   100                 105                 110
   Ala
<210> 20
   <211> 113
   <212> PRT
   <213> artificial
<220>
   <223> Anticorps 18E1 - Domaine variable chaine légère
<400> 20
   Asp Ile Val Met Thr Gln Thr Pro Leu Thr Leu Ser Val Thr Ile Gly
   1               5                   10                  15
   Gln Pro Ala Ser Ile Ser Cys Lys Ser Ser Gln Ser Leu Leu Tyr Ser
               20                  25                  30
   Asp Gly Lys Thr Tyr Leu Asn Trp Leu Phe Gln Ser Pro Gly Gln Ser
           35                  40                  45
   Pro Lys Leu Leu Ile Tyr Leu Val Ser Lys Leu Glu Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Val Gln Gly
                   85                  90                  95
   Ser His Phe Pro His Thr Phe Gly Ser Gly Thr Lys Leu Glu Ile Asn
               100                 105                 110
   Arg
<210> 21
   <211> 8
   <212> PRT
   <213> artificial
<220>
   <223> Anticorps 10B2 - CDR3H1
<400> 21
   Gly Tyr Thr Phe Thr Asp Tyr Tyr
   1               5
<210> 22
   <211> 8
   <212> PRT
   <213> artificial
<220>
   <223> Anticorps 10B2 - CDR3H2
<400> 22
   Ile Asn Pro Asn Asn Gly Gly Ser
   1               5
<210> 23
   <211> 15
   <212> PRT
   <213> artificial
<220>
   <223> Anticorps 10B2 - CDR3H3
<400> 23
   Ala Arg Gly Asp Tyr Ser Gly Thr Ser Phe Val Met Phe Ala His
   1 5 10 15
<210> 24
   <211> 11
   <212> PRT
   <213> artificial
<220>
   <223> Anticorps 10B2 - CDR3L1
<400> 24
   Lys Ser Leu Leu His Ser Asn Gly Asn Thr Tyr
   1 5 10
<210> 25
   <211> 3
   <212> PRT
   <213> artificial
<220>
   <223> Anticorps 10B2 - CDR3L2
<400> 25
   Tyr Met Ser
   1
<210> 26
   <211> 9
   <212> PRT
   <213> artificial
<220>
   <223> Anticorps 10B2 - CDR3L3
<400> 26
   Met Gln Ser Leu Glu Tyr Pro Val Thr
   1 5
<210> 27
   <211> 122
   <212> PRT
   <213> artificial
<220>
   <223> Anticorps 10B2 - Domaine variable chaine lourde
<400> 27
   Glu Val Gln Leu Gln Gln Ser Gly Pro Glu Leu Val Lys Pro Gly Ala
   1 5 10 15
   Ser Val Lys Ile Ser Cys Lys Ala Ser Gly Tyr Thr Phe Thr Asp Tyr
   20 25 30
   Tyr Met Asn Trp Val Lys Gln Ser His Gly Lys Thr Leu Glu Trp Ile
   35 40 45
   Gly Asp Ile Asn Pro Asn Asn Gly Gly Ser His Tyr Asn Gln Lys Phe
   50 55 60
   Arg Gly Lys Ala Thr Leu Thr Ile Asp Lys Ser Ser Ser Thr Ala Tyr
   65 70 75 80
   Met Asp Leu Arg Ser Leu Thr Ser Glu Asp Ser Ala Val Tyr Tyr Cys
   85 90 95
   Ala Arg Gly Asp Tyr Ser Gly Thr Ser Phe Val Met Phe Ala His Trp
   100 105 110
   Gly Gln Gly Thr Leu Val Thr Val Ser Ala
   115 120
<210> 28
   <211> 113
   <212> PRT
   <213> artificial
<220>
   <223> Anticorps 10B2 - Domaine variable chaine légère
<400> 28
   Asp Ile Val Met Thr Gln Ala Ala Pro Ser Val Pro Val Thr Pro Gly
   1 5 10 15
   Glu Ser Val Ser Ile Ser Cys Arg Ser Thr Lys Ser Leu Leu His Ser
   20 25 30
   Asn Gly Asn Thr Tyr Leu Tyr Trp Phe Leu Gln Arg Pro Gly Gln Ser
   35 40 45
   Pro Gln Leu Leu Ile Tyr Tyr Met Ser Asn Pro Ala Ser Gly Val Pro
   50 55 60
   Asp Arg Phe Ser Gly Ser Gly Ser Gly Thr Asp Phe Thr Leu Arg Ile
   65 70 75 80
   Ser Arg Val Gly Ala Glu Asp Val Gly Ile Tyr Tyr Cys Met Gln Ser
   85 90 95
   Leu Glu Tyr Pro Val Thr Phe Gly Ala Gly Thr Lys Leu Glu Leu Lys
   100 105 110
   Arg
<210> 29
   <211> 8
   <212> PRT
   <213> artificial
<220>
   <223> Anticorps 15G9 - CDR4H1
<400> 29
   Gly Tyr Thr Phe Thr Asp Tyr Ala
   1 5
<210> 30
   <211> 8
   <212> PRT
   <213> artificial
<220>
   <223> Anticorps 15G9 - CDR4H2
<400> 30
   Ile Arg Thr Tyr Ser Gly Asp Ala
   1 5
<210> 31
   <211> 6
   <212> PRT
   <213> artificial
<220>
   <223> Anticorps 15G9 - CDR4H3
<400> 31
   Ala Thr Gly Phe Asn Tyr
   1 5
<210> 32
   <211> 11
   <212> PRT
   <213> artificial
<220>
   <223> Anticorps 15G9 - CDR4L1
<400> 32
   Gln Ser Leu Leu His Ser Asn Gly Lys Thr Tyr
   1 5 10
<210> 33
   <211> 3
   <212> PRT
   <213> artificial
<220>
   <223> Anticorps 15G9 - CDR4L2
<400> 33
   Leu Val Ser
   1
<210> 34
   <211> 9
   <212> PRT
   <213> artificial
<220>
   <223> Anticorps 15G9 - CDR4L3
<400> 34
   Leu Gln Ala Thr His Phe Pro His Thr
   1 5
<210> 35
   <211> 113
   <212> PRT
   <213> artificial
<220>
   <223> Anticorps 15G9 - Domaine variable chaine lourde
<400> 35
   Gln Val Gln Leu Gln Gln Ser Gly Ala Glu Leu Val Arg Pro Gly Val
   1 5 10 15
   Ser Val Lys Ile Ser Cys Lys Gly Ser Gly Tyr Thr Phe Thr Asp Tyr
               20                  25                  30
   Ala Met His Trp Val Lys Gln Ser His Ala Lys Ser Leu Glu Trp Ile
           35                  40                  45
   Gly Val Ile Arg Thr Tyr Ser Gly Asp Ala Thr Tyr Asn Gln Lys Phe
       50                  55                  60
   Lys Gly Lys Ala Thr Met Thr Val Asp Lys Ser Ser Ser Thr Ala Tyr
   65                  70                  75                  80
   Met Glu Leu Ala Arg Leu Thr Ser Glu Asp Ser Ala Ile Tyr Tyr Cys
                   85                  90                  95
   Ala Thr Gly Phe Asn Tyr Trp Gly Gln Gly Thr Thr Leu Thr Val Ser
               100                 105                 110
   Ser
<210> 36
   <211> 113
   <212> PRT
   <213> artificial
<220>
   <223> Anticorps 15G9 - Domaine variable chaine légère
<400> 36
   Asp Val Val Met Thr Gln Thr Pro Leu Thr Leu Ser Val Thr Ile Gly
   1               5                   10                  15
   Gln Pro Ala Ser Ile Ser Cys Lys Ser Ser Gln Ser Leu Leu His Ser
               20                  25                  30
   Asn Gly Lys Thr Tyr Leu Asn Trp Leu Leu Gln Arg Pro Gly Gln Ser
           35                  40                  45
   Pro Lys Leu Leu Ile Tyr Leu Val Ser Lys Leu Glu Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Leu Tyr Tyr Cys Leu Gln Ala
                   85                  90                  95
   Thr His Phe Pro His Thr Phe Gly Ser Gly Thr Lys Leu Glu Ile Lys
                   100                 105                 110
   Arg
<210> 37
   <211> 446
   <212> PRT
   <213> artificial
<220>
   <223> Anticorps 10B2 - Chaîne lourde
<400> 37
   Glu Val Gln Leu Gln Gln Ser Gly Pro Glu Leu Val Lys Pro Gly Ala
   1               5                   10                  15
   Ser Val Lys Ile Ser Cys Lys Ala Ser Gly Tyr Thr Phe Thr Asp Tyr
               20                  25                  30
   Tyr Met Asn Trp Val Lys Gln Ser His Gly Lys Thr Leu Glu Trp Ile
           35                  40                  45
   Gly Asp Ile Asn Pro Asn Asn Gly Gly Ser His Tyr Asn Gln Lys Phe
       50                  55                  60
   Arg Gly Lys Ala Thr Leu Thr Ile Asp Lys Ser Ser Ser Thr Ala Tyr
   65                  70                  75                  80
   Met Asp Leu Arg Ser Leu Thr Ser Glu Asp Ser Ala Val Tyr Tyr Cys
                   85                  90                  95
   Ala Arg Gly Asp Tyr Ser Gly Thr Ser Phe Val Met Phe Ala His Trp
               100                 105                 110
   Gly Gln Gly Thr Leu Val Thr Val Ser Ala Ala Lys Thr Thr Pro Pro
           115                 120                 125
   Ser Val Tyr Pro Leu Ala Pro Gly Ser Ala Ala Gln Thr Asn Ser Met
       130                 135                 140
   Val Thr Leu Gly Cys Leu Val Lys Gly Tyr Phe Pro Glu Pro Val Thr
   145                 150                 155                 160
   Val Thr Trp Asn Ser Gly Ser Leu Ser Ser Gly Val His Thr Phe Pro
                   165                 170                 175
   Ala Val Leu Gln Ser Asp Leu Tyr Thr Leu Ser Ser Ser Val Thr Val
               180                 185                 190
   Pro Ser Ser Thr Trp Pro Ser Gln Thr Val Thr Cys Asn Val Ala His
           195                 200                 205
   Pro Ala Ser Ser Thr Lys Val Asp Lys Lys Ile Val Pro Arg Asp Cys
       210                 215                 220
   Gly Cys Lys Pro Cys Ile Cys Thr Val Pro Glu Val Ser Ser Val Phe
   225                 230                 235                 240
   Ile Phe Pro Pro Lys Pro Lys Asp Val Leu Thr Ile Thr Leu Thr Pro
                   245                 250                 255
   Lys Val Thr Cys Val Val Val Asp Ile Ser Lys Asp Asp Pro Glu Val
               260                 265                 270
   Gln Phe Ser Trp Phe Val Asp Asp Val Glu Val His Thr Ala Gln Thr
           275                 280                 285
   Lys Pro Arg Glu Glu Gln Ile Asn Ser Thr Phe Arg Ser Val Ser Glu
       290                 295                 300
   Leu Pro Ile Met His Gln Asp Trp Leu Asn Gly Lys Glu Phe Lys Cys
   305                 310                 315                 320
   Arg Val Asn Ser Ala Ala Phe Pro Ala Pro Ile Glu Lys Thr Ile Ser
                   325                 330                 335
   Lys Thr Lys Gly Arg Pro Lys Ala Pro Gln Val Tyr Thr Ile Pro Pro
               340                 345                 350
   Pro Lys Glu Gln Met Ala Lys Asp Lys Val Ser Leu Thr Cys Met Ile
           355                 360                 365
   Thr Asn Phe Phe Pro Glu Asp Ile Thr Val Glu Trp Gln Trp Asn Gly
       370                 375                 380
   Gln Pro Ala Glu Asn Tyr Lys Asn Thr Gln Pro Ile Met Asp Thr Asp
   385                 390                 395                 400
   Gly Ser Tyr Phe Val Tyr Ser Lys Leu Asn Val Gln Lys Ser Asn Trp
                   405                 410                 415
   Glu Ala Gly Asn Thr Phe Thr Cys Ser Val Leu His Glu Gly Leu His
               420                 425                 430
   Asn His His Thr Glu Lys Ser Leu Ser His Ser Pro Gly Lys
   435 440 445
<210> 38
   <211> 219
   <212> PRT
   <213> artificial
<220>
   <223> Anticorps 10B2 - Chaîne légère
<400> 38
   Asp Ile Val Met Thr Gln Ala Ala Pro Ser Val Pro Val Thr Pro Gly
   1               5                   10                  15
   Glu Ser Val Ser Ile Ser Cys Arg Ser Thr Lys Ser Leu Leu His Ser
               20                  25                  30
   Asn Gly Asn Thr Tyr Leu Tyr Trp Phe Leu Gln Arg Pro Gly Gln Ser
           35                  40                  45
   Pro Gln Leu Leu Ile Tyr Tyr Met Ser Asn Pro Ala Ser Gly Val Pro
       50                  55                  60
   Asp Arg Phe Ser Gly Ser Gly Ser Gly Thr Asp Phe Thr Leu Arg Ile
   65                  70                  75                  80
   Ser Arg Val Gly Ala Glu Asp Val Gly Ile Tyr Tyr Cys Met Gln Ser
                   85                  90                  95
   Leu Glu Tyr Pro Val Thr Phe Gly Ala Gly Thr Lys Leu Glu Leu Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Arg Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
           195                 200                 205
   Pro Ile Val Lys Ser Phe Asn Arg Asn Glu Cys
       210                 215
<210> 39
   <211> 438
   <212> PRT
   <213> artificial
<220>
   <223> Anticorps 1B1 - Chaîne lourde
<400> 39
   Gln Val Gln Leu Gln Gln Pro Gly Ala Asp Leu Val Lys Pro Gly Ala
   1               5                   10                  15
   Ser Val Lys Leu Ser Cys Lys Thr Ser Gly Tyr Thr Phe Thr Asn Tyr
               20                  25                  30
   Trp Ile Gln Trp Ile Lys Gln Lys Pro Gly Gln Gly Leu Glu Trp Ile
           35                  40                  45
   Gly Glu Ile Asp Pro Ser Asp Arg Tyr Thr Asn Tyr Asn Gln Arg Phe
       50                  55                  60
   Lys Gly Lys Ala Thr Leu Thr Val Asp Thr Ser Ser Ser Thr Ala Tyr
   65                  70                  75                  80
   Met Gln Leu Asn Ser Leu Thr Ser Glu Asp Ser Ala Val Tyr Tyr Cys
                   85                  90                  95
   Thr Met Tyr Ser Phe Ala Tyr Trp Gly Gln Gly Thr Leu Val Thr Val
               100                 105                 110
   Ser Ala Ala Lys Thr Thr Pro Pro Ser Val Tyr Pro Leu Ala Pro Gly
           115                 120                 125
   Ser Ala Ala Gln Thr Asn Ser Met Val Thr Leu Gly Cys Leu Val Lys
       130                 135                 140
   Gly Tyr Phe Pro Glu Pro Val Thr Val Thr Trp Asn Ser Gly Ser Leu
   145                 150                 155                 160
   Ser Ser Gly Val His Thr Phe Pro Ala Val Leu Gln Ser Asp Leu Tyr
                   165                 170                 175
   Thr Leu Ser Ser Ser Val Thr Val Pro Ser Ser Thr Trp Pro Ser Gln
               180                 185                 190
   Thr Val Thr Cys Asn Val Ala His Pro Ala Ser Ser Thr Lys Val Asp
           195                 200                 205
   Lys Lys Ile Val Pro Arg Asp Cys Gly Cys Lys Pro Cys Ile Cys Thr
       210                 215                 220
   Val Pro Glu Val Ser Ser Val Phe Ile Phe Pro Pro Lys Pro Lys Asp
   225                 230                 235                 240
   Val Leu Thr Ile Thr Leu Thr Pro Lys Val Thr Cys Val Val Val Asp
                   245                 250                 255
   Ile Ser Lys Asp Asp Pro Glu Val Gln Phe Ser Trp Phe Val Asp Asp
               260                 265                 270
   Val Glu Val His Thr Ala Gln Thr Lys Pro Arg Glu Glu Gln Ile Asn
           275                 280                 285
   Ser Thr Phe Arg Ser Val Ser Glu Leu Pro Ile Met His Gln Asp Trp
       290                 295                 300
   Leu Asn Gly Lys Glu Phe Lys Cys Arg Val Asn Ser Ala Ala Phe Pro
   305                 310                 315                 320
   Ala Pro Ile Glu Lys Thr Ile Ser Lys Thr Lys Gly Arg Pro Lys Ala
                   325                 330                 335
   Pro Gln Val Tyr Thr Ile Pro Pro Pro Lys Glu Gln Met Ala Lys Asp
               340                 345                 350
   Lys Val Ser Leu Thr Cys Met Ile Thr Asn Phe Phe Pro Glu Asp Ile
           355                 360                 365
   Thr Val Glu Trp Gln Trp Asn Gly Gln Pro Ala Glu Asn Tyr Lys Asn
       370                 375                 380
   Thr Gln Pro Ile Met Asp Thr Asp Gly Ser Tyr Phe Val Tyr Ser Lys
   385                 390                 395                 400
   Leu Asn Val Gln Lys Ser Asn Trp Glu Ala Gly Asn Thr Phe Thr Cys
                   405                 410                 415
   Ser Val Leu His Glu Gly Leu His Asn His His Thr Glu Lys Ser Leu
               420                 425                 430
   Ser His Ser Pro Gly Lys
           435
<210> 40
   <211> 219
   <212> PRT
   <213> artificial
<220>
   <223> Anticorps 1B1 - Chaîne légère
<400> 40
   Asp Val Val Met Thr Gln Thr Pro Leu Thr Leu Ser Val Thr Ile Gly
   1               5                   10                  15
   Gln Pro Ala Ser Ile Ser Cys Lys Ser Ser Gln Ser Leu Leu Asp Ser
               20                  25                  30
   Asp Gly Lys Thr Tyr Leu Asn Trp Leu Leu Gln Ser Pro Gly Gln Ser
           35                  40                  45
   Pro Lys Leu Leu Ile Tyr Leu Val Ser Lys Leu Glu Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Val Gln Gly
                   85                  90                  95
   Ala His Leu Pro Gln Thr Phe Gly Ser Gly Thr Lys Leu Glu Ile Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Arg Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
           195                 200                 205
   Pro Ile Val Lys Ser Phe Asn Arg Asn Glu Cys
   210 215
<210> 41
   <211> 443
   <212> PRT
   <213> artificial
<220>
   <223> Anticorps 18E1 - Chaîne lourde
<400> 41
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Asp Thr Phe Thr Asp Tyr
               20                  25                  30
   Pro Met His Trp Val Lys Gln Ala Pro Gly Lys Gly Leu Lys Trp Met
           35                  40                  45
   Gly Trp Ile Asn Ala Glu Thr Ala Glu Pro Thr Tyr Val Asp Asp Phe
       50                  55                  60
   Lys Gly Arg Phe Ala Phe Ser Leu Glu Ser Ser Ala Ser Thr Ala Tyr
   65                  70                  75                  80
   Leu Gln Ile Asn Asn Leu Lys Asn Glu Asp Thr Ala Thr Tyr Phe Cys
                   85                  90                  95
   Ala Ser Ser Tyr Gly Tyr Trp Gly Gln Gly Thr Leu Val Thr Val Ser
               100                 105                 110
   Ala Ala Lys Thr Thr Ala Pro Ser Val Tyr Pro Leu Ala Pro Val Cys
           115                 120                 125
   Gly Asp Thr Thr Gly Ser Ser Val Thr Leu Gly Cys Leu Val Lys Gly
       130                 135                 140
   Tyr Phe Pro Glu Pro Val Thr Leu Thr Trp Asn Ser Gly Ser Leu Ser
   145                 150                 155                 160
   Ser Gly Val His Thr Phe Pro Ala Val Leu Gln Ser Asp Leu Tyr Thr
                   165                 170                 175
   Leu Ser Ser Ser Val Thr Val Thr Ser Ser Thr Trp Pro Ser Gln Ser
               180                 185                 190
   Ile Thr Cys Asn Val Ala His Pro Ala Ser Ser Thr Lys Val Asp Lys
           195                 200                 205
   Lys Ile Glu Pro Arg Gly Pro Thr Ile Lys Pro Cys Pro Pro Cys Lys
       210                 215                 220
   Cys Pro Ala Pro Asn Leu Leu Gly Gly Pro Ser Val Phe Ile Phe Pro
   225                 230                 235                 240
   Pro Lys Ile Lys Asp Val Leu Met Ile Ser Leu Ser Pro Ile Val Thr
                   245                 250                 255
   Cys Val Val Val Asp Val Ser Glu Asp Asp Pro Asp Val Gln Ile Ser
               260                 265                 270
   Trp Phe Val Asn Asn Val Glu Val His Thr Ala Gln Thr Gln Thr His
           275                 280                 285
   Arg Glu Asp Tyr Asn Ser Thr Leu Arg Val Val Ser Ala Leu Pro Ile
       290                 295                 300
   Gln His Gln Asp Trp Met Ser Gly Lys Glu Phe Lys Cys Lys Val Asn
   305                 310                 315                 320
   Asn Lys Asp Leu Pro Ala Pro Ile Glu Arg Thr Ile Ser Lys Pro Lys
                   325                 330                 335
   Gly Ser Val Arg Ala Pro Gln Val Tyr Val Leu Pro Pro Pro Glu Glu
               340                 345                 350
   Glu Met Thr Lys Lys Gln Val Thr Leu Thr Cys Met Val Thr Asp Phe
           355                 360                 365
   Met Pro Glu Asp Ile Tyr Val Glu Trp Thr Asn Asn Gly Lys Thr Glu
       370                 375                 380
   Leu Asn Tyr Lys Asn Thr Glu Pro Val Leu Asp Ser Asp Gly Ser Tyr
   385                 390                 395                 400
   Phe Met Tyr Ser Lys Leu Arg Val Glu Lys Lys Asn Trp Val Glu Arg
                   405                 410                 415
   Asn Ser Tyr Ser Cys Ser Val Val His Glu Gly Leu His Asn His His
               420                 425                 430
   Thr Thr Lys Ser Phe Ser Arg Thr Pro Gly Lys
           435                 440
<210> 42
   <211> 219
   <212> PRT
   <213> artificial
<220>
   <223> Anticorps 18E1 - Chaîne légère
<400> 42
   Asp Ile Val Met Thr Gln Thr Pro Leu Thr Leu Ser Val Thr Ile Gly
   1               5                   10                  15
   Gln Pro Ala Ser Ile Ser Cys Lys Ser Ser Gln Ser Leu Leu Tyr Ser
               20                  25                  30
   Asp Gly Lys Thr Tyr Leu Asn Trp Leu Phe Gln Ser Pro Gly Gln Ser
           35                  40                  45
   Pro Lys Leu Leu Ile Tyr Leu Val Ser Lys Leu Glu Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Val Gln Gly
                   85                  90                  95
   Ser His Phe Pro His Thr Phe Gly Ser Gly Thr Lys Leu Glu Ile Asn
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Arg Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
           195                 200                 205
   Pro Ile Val Lys Ser Phe Asn Arg Asn Glu Cys
   210 215
<210> 43
   <211> 449
   <212> PRT
   <213> artificial
<220>
   <223> Anticorps 15G9 - Chaîne lourde
<400> 43
   Gln Val Gln Leu Gln Gln Ser Gly Ala Glu Leu Val Arg Pro Gly Val
   1               5                   10                  15
   Ser Val Lys Ile Ser Cys Lys Gly Ser Gly Tyr Thr Phe Thr Asp Tyr
               20                  25                  30
   Ala Met His Trp Val Lys Gln Ser His Ala Lys Ser Leu Glu Trp Ile
           35                  40                  45
   Gly Val Ile Arg Thr Tyr Ser Gly Asp Ala Thr Tyr Asn Gln Lys Phe
       50                  55                  60
   Lys Gly Lys Ala Thr Met Thr Val Asp Lys Ser Ser Ser Thr Ala Tyr
   65                  70                  75                  80
   Met Glu Leu Ala Arg Leu Thr Ser Glu Asp Ser Ala Ile Tyr Tyr Cys
                   85                  90                  95
   Ala Thr Gly Phe Asn Tyr Trp Gly Gln Gly Thr Thr Leu Thr Val Ser
               100                 105                 110
   Ser Ala Lys Thr Thr Pro Pro Ser Val Tyr Pro Leu Ala Pro Gly Cys
           115                 120                 125
   Gly Asp Thr Thr Gly Ser Ser Val Thr Leu Gly Cys Leu Val Lys Gly
       130                 135                 140
   Tyr Phe Pro Glu Ser Val Thr Val Thr Trp Asn Ser Gly Ser Leu Ser
   145                 150                 155                 160
   Ser Ser Val His Thr Phe Pro Ala Leu Leu Gln Ser Gly Leu Tyr Thr
                   165                 170                 175
   Met Ser Ser Ser Val Thr Val Pro Ser Ser Thr Trp Pro Ser Gln Thr
               180                 185                 190
   Val Thr Cys Ser Val Ala His Pro Ala Ser Ser Thr Thr Val Asp Lys
           195                 200                 205
   Lys Leu Glu Pro Ser Gly Pro Ile Ser Thr Ile Asn Pro Cys Pro Pro
       210                 215                 220
   Cys Lys Glu Cys His Lys Cys Pro Ala Pro Asn Leu Glu Gly Gly Pro
   225                 230                 235                 240
   Ser Val Phe Ile Phe Pro Pro Asn Ile Lys Asp Val Leu Met Ile Ser
                   245                 250                 255
   Leu Thr Pro Lys Val Thr Cys Val Val Val Asp Val Ser Glu Asp Asp
               260                 265                 270
   Pro Asp Val Gln Ile Ser Trp Phe Val Asn Asn Val Glu Val His Thr
           275                 280                 285
   Ala Gln Thr Gln Thr His Arg Glu Asp Tyr Asn Ser Thr Ile Arg Val
       290                 295                 300
   Val Ser Thr Leu Pro Ile Gln His Gln Asp Trp Met Ser Gly Lys Glu
   305                 310                 315                 320
   Phe Lys Cys Lys Val Asn Asn Lys Asp Leu Pro Ser Pro Ile Glu Arg
                   325                 330                 335
   Thr Ile Ser Lys Ile Lys Gly Leu Val Arg Ala Pro Gln Val Tyr Ile
               340                 345                 350
   Leu Pro Pro Pro Ala Glu Gln Leu Ser Arg Lys Asp Val Ser Leu Thr
           355                 360                 365
   Cys Leu Val Val Gly Phe Asn Pro Gly Asp Ile Ser Val Glu Trp Thr
       370                 375                 380
   Ser Asn Gly His Thr Glu Glu Asn Tyr Lys Asp Thr Ala Pro Val Leu
   385                 390                 395                 400
   Asp Ser Asp Gly Ser Tyr Phe Ile Tyr Ser Lys Leu Asn Met Lys Thr
                   405                 410                 415
   Ser Lys Trp Glu Lys Thr Asp Ser Phe Ser Cys Asn Val Arg His Glu
               420                 425                 430
   Gly Leu Lys Asn Tyr Tyr Leu Lys Lys Thr Ile Ser Arg Ser Pro Gly
           435                 440                 445
   Lys
<210> 44
   <211> 219
   <212> PRT
   <213> artificial
<220>
   <223> Anticorps 15G9 - Chaîne légère
<400> 44
   Asp Val Val Met Thr Gln Thr Pro Leu Thr Leu Ser Val Thr Ile Gly
   1               5                   10                  15
   Gln Pro Ala Ser Ile Ser Cys Lys Ser Ser Gln Ser Leu Leu His Ser
               20                  25                  30
   Asn Gly Lys Thr Tyr Leu Asn Trp Leu Leu Gln Arg Pro Gly Gln Ser
           35                  40                  45
   Pro Lys Leu Leu Ile Tyr Leu Val Ser Lys Leu Glu Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Leu Tyr Tyr Cys Leu Gln Ala
                   85                  90                  95
   Thr His Phe Pro His Thr Phe Gly Ser Gly Thr Lys Leu Glu Ile Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Arg Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
                 195                 200                 205
   Pro Ile Val Lys Ser Phe Asn Arg Asn Glu Cys
       210                 215
<210> 45
   <211> 24
   <212> DNA
   <213> artificial
<220>
   <223> Anticorps 1B1 - CDR1H1
<400> 45
   ggctacacct tcaccaacta ctgg 24
<210> 46
   <211> 24
   <212> DNA
   <213> artificial
<220>
   <223> Anticorps 1B1 - CDR1H2
<400> 46
   attgatcctt ctgatagata tact 24
<210> 47
   <211> 21
   <212> DNA
   <213> artificial
<220>
   <223> Anticorps 1B1 - CDR1H3
<400> 47
   acaatgtatt cgtttgctta c 21
<210> 48
   <211> 33
   <212> DNA
   <213> artificial
<220>
   <223> Anticorps 1B1 - CDR1L1
<400> 48
   cagagcctct tagatagtga tggaaaaacc tat 33
<210> 49
   <211> 9
   <212> DNA
   <213> artificial
<220>
   <223> Anticorps 1B1 - CDR1L2
<400> 49
   ctggtgtct 9
<210> 50
   <211> 27
   <212> DNA
   <213> artificial
<220>
   <223> Anticorps 1B1 - CDR1L3
<400> 50
   gtgcaaggtg cacatctccc tcaaacg 27
<210> 51
   <211> 342
   <212> DNA
   <213> artificial
<220>
   <223> Anticorps 1B1 - Domaine variable chaine lourde
<400> 51
   caggtccaac tgcagcagcc tggggctgac cttgtgaagc ctggggcttc agtgaagctc 60
   tcctgcaaga cctctggcta caccttcacc aactactgga tacagtggat aaaacagaag 120
   cctggacagg gccttgagtg gatcggagag attgatcctt ctgatagata tactaattac 180
   aatcaaagat tcaagggcaa ggccacattg actgtagaca catcctccag cacagcctac 240
   atgcagctca acagcctaac atctgaggac tctgcggtct attactgtac aatgtattcg 300
   tttgcttact ggggccaagg gactctggtc actgtctctg ca 342
<210> 52
   <211> 339
   <212> DNA
   <213> artificial
<220>
   <223> Anticorps 1B1 - Domaine variable chaine légère
<400> 52
   gatgttgtga tgacccagac tccactcact ttgtctgtta ccattggaca gccagcttcc 60
   atttcttgca agtcaagtca gagcctctta gatagtgatg gaaaaaccta tttgaattgg 120
   ttattacaga gtccaggcca gtctccaaag ctcttaatct atctggtgtc taagctggaa 180
   tctggagtcc ctgacagatt cagtggcagt ggatcaggga cagattttac actgaaaatc 240
   agcagagtgg aggctgagga tttgggagtt tattactgcg tgcaaggtgc acatctccct 300
   caaacgttcg gatcggggac caaactggaa ataaaacgg 339
<210> 53
   <211> 24
   <212> DNA
   <213> artificial
<220>
   <223> Anticorps 18E1 - CDR1H1
<400> 53
   ggtgacacct tcacagacta tcca 24
<210> 54
   <211> 24
   <212> DNA
   <213> artificial
<220>
   <223> Anticorps 18E1 - CDR1H2
<400> 54
   ataaacgctg agactgctga gcca 24
<210> 55
   <211> 18
   <212> DNA
   <213> artificial
<220>
   <223> Anticorps 18E1 - CDR1H3
<400> 55
   gctagttcct atggttac 18
<210> 56
   <211> 33
   <212> DNA
   <213> artificial
<220>
   <223> Anticorps 18E1 - CDR1L1
<400> 56
   cagagcctct tatatagtga tggaaaaacc tat 33
<210> 57
   <211> 9
   <212> DNA
   <213> artificial
<220>
   <223> Anticorps 18E1 - CDR1L2
<400> 57
   ctggtgtct 9
<210> 58
   <211> 27
   <212> DNA
   <213> artificial
<220>
   <223> Anticorps 18E1 - CDR1L3
<400> 58
   gtgcaaggtt cacatttccc tcatacg 27
<210> 59
   <211> 339
   <212> DNA
   <213> artificial
<220>
   <223> Anticorps 18E1 - Domaine variable chaine lourde
<400> 59
   cagatccagt tggtgcagtc tggacctgag ctgaagaagc ctggagagac agtcaagatc        60
   tcctgcaagg cttctggtga caccttcaca gactatccaa tgcactgggt gaagcaggct       120
   ccaggaaagg gtttaaagtg gatgggctgg ataaacgctg agactgctga gccaacatat       180
   gtagacgact tcaagggacg gtttgccttc tctttggaat cctctgccag cactgcctat       240
   ttgcagatca acaacctcaa aaatgaggac acggctacat atttctgtgc tagttcctat       300
   ggttactggg gccaagggac tctggtcact gtctctgca                              339
<210> 60
   <211> 339
   <212> DNA
   <213> artificial
<220>
   <223> Anticorps 18E1 - Domaine variable chaine légère
<400> 60
   gatattgtga tgacccagac tccactcact ttgtctgtta ccattggaca gccagcttcc        60
   atttcttgca agtcaagtca gagcctctta tatagtgatg gaaaaaccta tttgaattgg       120
   ttattccaga gtccaggcca gtctccaaag ctcttaatct atctggtgtc taaactggaa       180
   tctggagtcc ctgacagatt cagtggcagt ggatcaggga cagattttac actgaaaatc       240
   agcagagtgg aggctgagga tttgggagtt tattactgcg tgcaaggttc acatttccct       300
   catacgttcg gatcggggac caagctggaa ataaaccgg                              339
<210> 61
   <211> 24
   <212> DNA
   <213> artificial
<220>
   <223> Anticorps 15G9 - CDR1H1
<400> 61
   ggctacacat tcactgatta tgct 24
<210> 62
   <211> 24
   <212> DNA
   <213> artificial
<220>
   <223> Anticorps 15G9 - CDR1H2
<400> 62
   attcgtactt actctggtga tgct 24
<210> 63
   <211> 18
   <212> DNA
   <213> artificial
<220>
   <223> Anticorps 15G9 - CDR1H3
<400> 63
   gcaaccgggt ttaactac 18
<210> 64
   <211> 33
   <212> DNA
   <213> artificial
<220>
   <223> Anticorps 15G9 - CDR1L1
<400> 64
   cagagcctct tacatagtaa tggaaagaca tat 33
<210> 65
   <211> 9
   <212> DNA
   <213> artificial
<220>
   <223> Anticorps 15G9 - CDR1L2
<400> 65
   ctggtgtct 9
<210> 66
   <211> 27
   <212> DNA
   <213> artificial
<220>
   <223> Anticorps 15G9 - CDR1L3
<400> 66
   ttgcaagcta cacattttcc tcatacg 27
<210> 67
   <211> 339
   <212> DNA
   <213> artificial
<220>
   <223> Anticorps 15G9 - Domaine variable chaine lourde
<400> 67
   caggtccagc tgcagcagtc tggggctgag ctggtgaggc ctggggtctc agtgaagatt 60
   tcctgcaagg gttctggcta cacattcact gattatgcta tgcactgggt gaagcagagt 120
   catgcaaaga gtctagagtg gattggagtt attcgtactt actctggtga tgctacctac 180
   aaccagaagt tcaagggcaa ggccacaatg actgtagaca aatcctccag cacagcctat 240
   atggaacttg ccagactgac atctgaggat tctgccatct attactgtgc aaccgggttt 300
   aactactggg gccaaggcac cactctcaca gtctcctca 339
<210> 68
   <211> 339
   <212> DNA
   <213> artificial
<220>
   <223> Anticorps 15G9 - Domaine variable chaine légère
<400> 68
   gatgttgtga tgactcagac cccactcact ttgtcggtta ccattggaca accagcctcc 60
   atctcttgca aatcaagtca gagcctctta catagtaatg gaaagacata tttgaattgg 120
   ttattacaga ggccaggcca gtctccaaag ctcttaatct atctggtgtc taaactggaa 180
   tctggagtcc ctgacaggtt cagtggcagt ggatcaggga cagatttcac actgaaaatc 240
   agcagagtgg aggctgagga tttgggactt tattactgct tgcaagctac acattttcct 300
   catacgttcg gatcggggac caagctggaa ataaaacgg 339
<210> 69
   <211> 24
   <212> DNA
   <213> artificial
<220>
   <223> Anticorps 10B2 - CDR1H1
<400> 69
   ggatacacgt tcactgatta ctac 24
<210> 70
   <211> 24
   <212> DNA
   <213> artificial
<220>
   <223> Anticorps 10B2 - CDR1H2
<400> 70
   attaatccta ataatggtgg tagt 24
<210> 71
   <211> 45
   <212> DNA
   <213> artificial
<220>
   <223> Anticorps 10B2 - CDR1H3
<400> 71
   gcaagaggcg attactccgg tactagtttc gttatgtttg ctcac 45
<210> 72
   <211> 33
   <212> DNA
   <213> artificial
<220>
   <223> Anticorps 10B2 - CDR1L1
<400> 72
   aagagtcttc tgcacagtaa tggcaacact tac 33
<210> 73
   <211> 9
   <212> DNA
   <213> artificial
<220>
   <223> Anticorps 10B2 - CDR1L2
<400> 73
   tatatgtcc 9
<210> 74
   <211> 27
   <212> DNA
   <213> artificial
<220>
   <223> Anticorps 10B2 - CDR1L3
<400> 74
   atgcaaagtc tagaatatcc tgtcacg 27
<210> 75
   <211> 366
   <212> DNA
   <213> artificial
<220>
   <223> Anticorps 10B2 - Domaine variable chaine lourde
<400> 75
   gaggtccagc tgcaacaatc tggacctgag ctggtgaagc ctggggcctc agttaagatt 60
   tcctgtaagg cttctggata cacgttcact gattactaca tgaactgggt gaagcagagc 120
   catggaaaga cccttgagtg gattggagat attaatccta ataatggtgg tagtcactac 180
   aaccagaagt tcaggggcaa ggccacattg actatagaca agtcctccag tacagcctac      240
   atggacctcc gcagcctgac atctgaagac tctgcagtct attactgtgc aagaggcgat      300
   tactccggta ctagtttcgt tatgtttgct cactggggcc aagggactct ggtcactgtc      360
   tctgca                                                                  366
<210> 76
   <211> 339
   <212> DNA
   <213> artificial
<220>
   <223> Anticorps 10B2 - Domaine variable chaine légère
<400> 76
   gatattgtga tgactcaggc tgcaccctct gtacctgtca ctcctggaga gtcagtatcc       60
   atctcctgca ggtctactaa gagtcttctg cacagtaatg gcaacactta cttgtattgg      120
   ttcctgcaga ggccaggcca gtctcctcaa ctcctgatat attatatgtc caaccctgcc      180
   tcaggagtcc cagacaggtt cagtggcagt gggtcaggaa ctgatttcac actgaggatc      240
   agtcgagtgg gggctgagga tgtaggtatt tattactgta tgcaaagtct agaatatcct      300
   gtcacgttcg gtgcggggac caagctggag ctgaaacgg                             339